# EUROPEAN PATENT APPLICATION

(11) **EP 2 119 719 A1**
(43) Date of publication of application: **18.11.2009**
(21) Application number: 07860009.5
(22) Date of filing: 25.12.2007
(51) Int. Cl.: C07D 513/04, A61K 31/554, A61P 3/00, A61P 3/04, A61P 3/06, A61P 3/10, A61P 5/46, A61P 9/12, A61P 43/00

(54) **THIAZEPINE DERIVATIVE**

(30) Priority: 26.12.2006 JP 2006348763
(71) Applicant: Daiichi Sankyo Company, Limited, Chuo-ku Tokyo 103-0023 (JP)
(72) Inventor: HASEGAWA, Toru, Tokyo 140-8710 (JP); SHINOZUKA, Tsuyoshi, Tokyo 140-8710 (JP)
(74) Representative: Smyth, Gyles Darren
(86) International application number: PCT/JP2007/074780
(87) International publication number: WO 2008/078725

(57) **Abstract**

The present invention relates to a thiazepine derivative or a pharmacologically acceptable salt thereof having an excellent effect of inhibiting 11β-hydroxysteroid dehydrogenase type 1. A thiazepine derivative or a pharmacologically acceptable salt thereof having general formula (I): 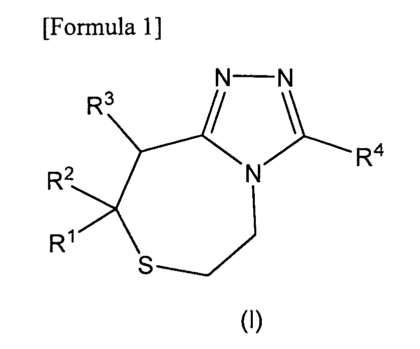 wherein
R¹ represents a hydrogen atom, a C₁-C₆ alkyl group or the like; R² represents a C₁-C₆ alkyl group, a C₁-C₆ halogenated alkyl group, a C₁-C₆ hydroxyalkyl group, or the like; R³ represents a hydrogen atom or a C₁-C₆ alkyl group; R⁴ represents a C₆-C₁₀ aryl group that may be substituted with 1 to 5 group(s) independently selected from Substituent Group a or a heterocyclic group that may be substituted with 1 to 3 group(s) independently selected from Substituent Group a; Substituent Group a consists of a halogen atom, a C₁-C₆ alkyl group, a C₆-C₁₀ aryl group that may be substituted with 1 to 5 group(s) independently selected from Substituent Group b, and so forth; Substituent Group b consists of a halogen atom, a C₁-C₆ alkyl group, a C₁-C₆ halogenated alkyl group, and so forth.

## Description

### Technical Field

The present invention relates to a thiazepine derivative or a pharmacologically acceptable salt thereof having a specific chemical structure that exhibits an inhibitory effect on 11 β-hydroxysteroid dehydrogenase type 1 (hereinafter, referred to as "11β-HSD 1 ") and a medicament containing the compound as an active ingredient (preferably, a therapeutic and/or prophylactic agent for diabetes, adiposity, hyperlipemia, hypertension, or metabolic syndrome).

### Background Art

Along with the changes in dietary habits, lifestyle, and living environment in recent years, patients with hyperglycemia, adiposity, hyperlipidemia, and hypertension as well as metabolic syndrome including two or more of these risk factors in combination are increasing in number worldwide, which has become a social problem (Non-Patent Document 1). In any treatment, dietary therapy and exercise therapy are used, and, if the effect is low, or the condition is severe, drug therapy is used in combination.

Diabetes is classified into insulin-dependent diabetes mellitus (type 1, IDDM) and non-insulin dependent diabetes mellitus (type 2, NIDDM), which affects 90% or more of diabetic patients. Insulin injections are used for treatment of IDDM, and sulfonylurea agents, which promote secretion of insulin, thiazolidine dione drugs, which improve insulin resistance, glycosidase inhibitors, which inhibit sugar digestion and absorption, and biguanide drugs, which inhibit gluconeogenesis in the liver, and the like are used for treatment of NIDDM (Non-Patent Document 2). However, none of these drugs necessarily has adequate effects, and an increasing number of patients has serious complications because efficacy attenuates after long term use.

In treatment of patients with severe adiposity conditions, agents for central suppression of food intake are used. However, adequate effects have not been achieved due to the limited duration of use and rebound.

Examples of therapeutic agents for hypertension include calcium antagonists, which produce vasodilatation, diuretics, which promote excretion of salts, β blockers, which suppress the sympathetic nerves to reduce the heart rate, α blockers, which suppress the sympathetic nerves to produce peripheral vasodilatation, angiotensin converting enzyme inhibitors and angiotensin receptor blockers, which inhibit vasoconstriction by angiotensin, and so forth. However, blood pressure control in early morning, the time of the day when cerebral apoplexy occurs most commonly, is difficult, and antihypertensive therapies are far from being adequate at present.

As therapeutic agents for hyperlipemia, HMG-CoA reducing enzyme inhibitors, which inhibit cholesterol synthesis in the liver, fibrate drugs, which inhibit triglyceride synthesis in the liver, anion exchange resins, which promote excretion of bile acid, and the like are used. The final aim of hyperlipemia treatment is prevention of artery sclerotic diseases including coronary artery diseases and cerebral infarction. However, since the condition of arteriosclerosis is caused by not only hyperlipemia but also coexisting risk factors such as hypertension, diabetes, adiposity, and aging, it is critical to pay attention to such other risk factors all the time, and multidimensional approaches are required (Non-Patent Document 3).

Glucocorticoids (cortisol in humans, corticosterone in rodents) are known to have various bioactivities for regulating blood sugar levels, blood pressure, and the like. For example, it is known that glucocorticoids have a bioactivity of promoting the release of amino acids from muscles and the release of fatty acids and glycerol from adipose tissues into blood via expression of various proteins to promote gluconeogenesis in the liver using these substrates, leading to the promotion of increase of blood sugar levels. Furthermore, it is also known that glucocorticoids have activities of maturing immature fat cells in adipose tissues, leading to adiposity, and acting on mineral corticoid receptors in the kidneys to elevate blood pressure. Mechanisms for regulating the glucocorticoid activity involve regulation of production and secretion of glucocorticoids in the hypothalamus-pituitary gland-adrenal cortex axis and recycling of glucocorticoids by 11β-HSD1 (conversion from an inactive form to an active form) in target organs such as the liver, adipose tissues, and the lungs (Non-Patent Document 4). 11β-HSD1 inhibitors are expected to prevent hyperglycemia, adiposity, hyperlipemia, and/or hypertension by inhibiting glucocorticoid actions in these tissues and to exhibit multidimensional effects on metabolic syndrome including these conditions in combination. These potentials are supported by the reports of adiposity with visceral fat, aggravation of glucose tolerance, insulin resistance, and elevated blood pressure in mice with highly expressed adipose tissue-specific 11β-HSD1 (Non-Patent Documents 5 and 6). The term "metabolic syndrome" means a combination of symptoms associated with abnormal metabolic functions for carbohydrates and lipids in the organism. The diagnosis criteria for this syndrome vary with a number of international organizations, but are consistent in having two or more symptoms among glucose tolerance impairment (or insulin resistance), adiposity, hypertension, hypertriglyceridemia, and low HDL cholesterol levels in blood. These symptoms are described in detail in Non-Patent Document 7. Aminothiazole sulfonamide derivatives (Non-Patent Document 8) and triazole derivatives (Patent Documents 1, 2, and 3) are known as 11β-HSD1 inhibitors, but have not been in practical use yet.
[Patent Document 1] WO03/065983
[Patent Document 2] WO04/089367
[Patent Document 3] WO04/089380
[Non-Patent Document 1] Nature, vol. 414, p.782 (2001)
[Non-Patent Document 2] The American Journal of Medicine, vol. 108, p.23 (2000)
[Non-Patent Document 3] The Journal of Clinical Endocrinology & Metabolism), vol. 89, p.2601 (2004)
[Non-Patent Document 4] Endocrinology, vol. 142, p.1371 (2001)
[Non-Patent Document 5] Science, vol. 294, p.2166 (2001)
[Non-Patent Document 6] The Journal of Clinical Investigation, vol. 112, p.83 (2003)
[Non-Patent Document 7] The Lancet, vol. 365, p.1415 (2005)
[Non-Patent Document 8] Journal of Medicinal Chemistry, vol. 45, p.3813 (2002)

### Disclosure of the Invention

### Problems to be Solved by the Invention

The inventors of the present invention conducted various researches about compounds having effects of inhibiting 11β-HSD1. As a result, they found that thiazepine derivatives having a specific chemical structure had excellent effects of inhibiting 11β-HSD1. Furthermore, they found that the present invention was useful as a medicament containing the thiazepine derivative or pharmacologically acceptable salt thereof as an active ingredient. Since the thiazepine derivative or pharmacologically acceptable salt thereof has the activity described above, the medicament is useful as an 11β-HSD1 inhibitor or a therapeutic or prophylactic agent for diabetes, adiposity, hyperlipemia, hypertension, and/or metabolic syndrome. Furthermore, they found that the present invention was useful as a method for inhibiting 11β-HSD1 in the organism by administration of this medicament or as a method for therapeutic or prophylactic treatment of diabetes, adiposity, hyperlipemia, hypertension, and/or metabolic syndrome. The present invention was accomplished based on the above-mentioned findings.

### Means for Solving the Problems

The present invention relates to (1) a thiazepine derivative having general formula (I): 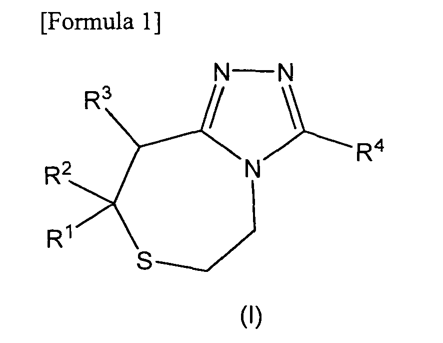 [wherein
R¹ represents a hydrogen atom, a C₁-C₆ alkyl group, a C₁-C₆ halogenated alkyl group, a C₁-C₆ hydroxyalkyl group, a (C₁-C₆ alkoxy)-(C₁-C₆ alkyl) group or a C₃-C₆ cycloalkyl group,
R² represents a C₁-C₆ alkyl group, a C₁-C₆ halogenated alkyl group, a C₁-C₆ hydroxyalkyl group, a (C₁-C₆ alkoxy)-(C₁-C₆ alkyl) group or a C₃-C₆ cycloalkyl group,
R³ represents a hydrogen atom or a C₁-C₆ alkyl group,
R⁴ represents a C₆-C₁₀ aryl group that may be substituted with 1 to 5 group(s) independently selected from Substituent Group a or a heterocyclic group that may be substituted with 1 to 3 group(s) independently selected from Substituent Group a,

Substituent Group a represents the group consisting of a halogen atom, a C₁-C₆ alkyl group, a C₁-C₆ halogenated alkyl group, a C₁-C₆ hydroxyalkyl group, a C₂-C₇ alkylcarbonyl group, a hydroxy group, a C₁-C₆ alkoxy group, a C₁-C₆ halogenated alkoxy group, a (C₁-C₆ alkoxy)-(C₁-C₆ alkyl) group, a C₃-C₆ cycloalkyl group, a C₁-C₆ alkyl group substituted with one C₃-C₆ cycloalkyl group, a carboxyl group, a C₂-C₇ alkoxycarbonyl group, a cyano group, a carbamoyl group, a C₂-C₇ alkylcarbonyloxy group, a C₁-C₆ alkylthio group, a C₁-C₆ alkylsulfonyl group, a nitro group, an amino group, a mono-C₁-C₆ alkylamino group, a di-(C₁-C₆ alkyl)amino group, a mono-(C₁-C₆ alkyl)aminocarbonyl group, a mono-C₂-C₇ alkylcarbonylamino group, a mono-C₁-C₆ alkylsulfonylamino group, a C₆-C₁₀ aryl group that may be substituted with 1 to 5 group(s) independently selected from Substituent Group b, a phenyloxy group that may be substituted with 1 to 5 group(s) independently selected from Substituent Group b, a phenylmethyloxy group that may be substituted with 1 to 5 group(s) independently selected from Substituent Group b, a phenylsulfonyl group that may be substituted with 1 to 5 group(s) independently selected from Substituent Group b and a heterocyclic group that may be substituted with 1 to 3 group(s) independently selected from Substituent Group b, and

Substituent Group b represents the group consisting of a halogen atom, a C₁-C₆ alkyl group, a C₁-C₆ halogenated alkyl group, a C₁-C₆ hydroxyalkyl group, a hydroxy group, a C₁-C₆ alkoxy group, a C₁-C₆ halogenated alkoxy group, a (C₁-C₆ alkoxy)-(C₁-C₆ alkyl) group, a carboxyl group, a C₂-C₇ alkoxycarbonyl group, a cyano group, a carbamoyl group, a C₂-C₇ alkylcarbonyloxy group, an oxo group, a C₁-C₆ alkylthio group, a C₁-C₆ alkylsulfonyl group, a nitro group, an amino group, a mono-C₁-C₆ alkylamino group, a di-(C₁-C₆ alkyl)amino group, a mono-(C₁-C₆ alkyl)aminocarbonyl group, a mono-C₂-C₇ alkylcarbonylamino group and a mono-C₁-C₆ alkylsulfonylamino group]
or a pharmacologically acceptable salt thereof.

The present invention preferably include
(2) the thiazepine derivative or pharmacologically acceptable salt thereof according to (1), wherein
   R¹ represents a C₁-C₆ alkyl group, R² represents a C₁-C₆ hydroxyalkyl group, and R³ represents a hydrogen atom;
(3) the thiazepine derivative or pharmacologically acceptable salt thereof according to (1), wherein
   R¹ represents a methyl group, R² represents a hydroxymethyl group, and R³ represents a hydrogen atom;
(4) the thiazepine derivative or pharmacologically acceptable salt thereof according to any one selected from (1) to (3), wherein
   R⁴ represents a phenyl group that may be substituted with 1 to 5 group(s) independently selected from Substituent Group a, an indolyl group that may be substituted with 1 to 3 group(s) independently selected from Substituent Group a or a benzothiophenyl group that may be substituted with 1 to 3 group(s) independently selected from Substituent Group a;
(5) the thiazepine derivative or pharmacologically acceptable salt thereof according to any one selected from (1) to (3), wherein,
   R⁴ represents a phenyl group that may be substituted with 1 to 5 group(s) independently selected from [a halogen atom, a C₁-C₆ halogenated alkyl group, a C₁-C₆ alkoxy group, a phenyl group that may be substituted with 1 to 5 group(s) independently selected from (a halogen atom, a C₂-C₇ alkoxycarbonyl group and a cyano group), a pyridin-4-yl group, a pyridin-3-yl group, a morpholin-4-yl group, a piperidin-1-yl group, a pyrrolidin-1-yl group and a 1H-pyrrol-1-yl group], an indolyl group that may be substituted with 1 to 3 group(s) independently selected from (a C₁-C₆ alkyl group and a C₁-C₆ alkyl group substituted with one C₃-C₆ cycloalkyl group) or a benzothiophenyl group that may be substituted with 1 to 3 group(s) independently selected from (a halogen atom and a C₁-C₆ alkyl group);
(6) the thiazepine derivative or pharmacologically acceptable salt thereof according to any one selected from (1) to (3), wherein
   R⁴ represents a phenyl group substituted with 2 groups independently selected from [a chlorine atom, a methoxy group and a phenyl group that may be substituted with 1 or 2 group(s) independently selected from (a fluorine atom, a chlorine atom and a cyano group)];
(7) the thiazepine derivative or pharmacologically acceptable salt thereof according to any one selected from (1) to (3), wherein
   R⁴ represents a 3-chlorobiphenyl-4-yl group, a 3-methoxybiphenyl-4-yl group, a 3-chloro-3'-fluorobiphenyl group, a 3,3'-dichlorobiphenyl-4-yl group, a 3-chloro-4'-fluorobiphenyl-4-yl group, a 3,4'-dichlorobiphenyl-4-yl group, a 3'-fluoro-3-methoxybiphenyl-4-yl group, a 3'-chloro-3-methoxybiphenyl-4-yl group, a 3'-cyano-3-methoxybiphenyl-4-yl group, a 4'-fluoro-3-methoxybiphenyl-4-yl group, a 3',4'-difluoro-3-methoxybiphenyl-4-yl group or a 3'-chloro-4'-fluoro-3-methoxybiphenyl-4-yl group;
(8) the thiazepine derivative or pharmacologically acceptable salt thereof according to (1), wherein
   R¹ represents a C₁-C₆ alkyl group, R² represents a C₁-C₆ hydroxyalkyl group, R³ represents a hydrogen atom, and R⁴ represents a phenyl group that may be substituted with 1 to 5 group(s) independently selected from Substituent Group a, an indolyl group that may be substituted with 1 to 3 group(s) independently selected from Substituent Group a or a benzothiophenyl group that may be substituted with 1 to 3 group(s) independently selected from Substituent Group a;
(9) the thiazepine derivative or pharmacologically acceptable salt thereof according to (1), wherein
   R¹ represents a C₁-C₆ alkyl group, R² represents a C₁-C₆ hydroxyalkyl group, R³ represents a hydrogen atom, and R⁴ represents a phenyl group that may be substituted with 1 to 5 group(s) independently selected from [a halogen atom, a C₁-C₆ halogenated alkyl group, a C₁-C₆ alkoxy group, a phenyl group that may be substituted with 1 to 5 group(s) independently selected from (a halogen atom, a C₂-C₇ alkoxycarbonyl group and a cyano group), a pyridin-4-yl group, a pyridin-3-yl group, a morpholin-4-yl group, a piperidin-1-yl group, a pyrrolidin-1-yl group and a 1H-pyrrol-1-yl group], an indolyl group that may be substituted with 1 to 3 group(s) independently selected from (a C₁-C₆ alkyl group and a C₁-C₆ alkyl group substituted with one C₃-C₆ cycloalkyl group) or a benzothiophenyl group that may be substituted with 1 to 3 group(s) independently selected from (a halogen atom and a C₁-C₆ alkyl group);
(10) the thiazepine derivative or pharmacologically acceptable salt thereof according to (1), wherein
   R¹ represents a methyl group, R² represents a hydroxymethyl group, R³ represents a hydrogen atom, and R⁴ represents a phenyl group substituted with 2 groups independently selected from [a chlorine atom, a methoxy group and a phenyl group that may be substituted with 1 or 2 group(s) independently selected from (a fluorine atom, a chlorine atom and a cyano group)];
(11) the thiazepine derivative or pharmacologically acceptable salt thereof according to (1), wherein
   R¹ represents a methyl group, R² represents a hydroxymethyl group, R³ represents a hydrogen atom, and R⁴ represents a 3-chlorobiphenyl-4-yl group, a 3-methoxybiphenyl-4-yl group, a 3-chloro-3'-fluorobiphenyl group, a 3,3'-dichlorobiphenyl-4-yl group, a 3-chloro-4'-fluorobiphenyl-4-yl group, a 3,4'-dichlorobiphenyl-4-yl group, a 3'-fluoro-3-methoxybiphenyl-4-yl group, a 3'-chloro-3-methoxybiphenyl-4-yl group, a 3'-cyano-3-methoxybiphenyl-4-yl group, a 4'-fluoro-3-methoxybiphenyl-4-yl group, a 3',4'-difluoro-3-methoxybiphenyl-4-yl group or a 3'-chloro-4'-fluoro-3-methoxybiphenyl-4-yl group;
(12) the thiazepine derivative or pharmacologically acceptable salt thereof according to (1), wherein the compound having the general formula (I) is
   {8-methyl-3-[2-methoxy-4-(trifluoromethyl)phenyl]-5,6,8,9-tetrahydro[1,2,4]triazolo[4,3-d][1,4]thiazepin-8-yl}methanol,
   [8-methyl-3-(1-methyl-1H-indol-4-yl)-5,6,8,9-tetrahydro[1,2,4]triazolo[4,3-d] [1,4]thiazepin-8-yl]methanol,
   {3-[1-(cyclopropylmethyl)-1H-indol-4-yl]-8-methyl-5,6,8,9-tetrahydro[1,2,4]triazolo[4,3-d][1,4]thiazepin-8-yl}methanol,
   [3-(5-chloro-3-methyl-1-benzothiophen-2-yl)-8-methyl-5,6,8,9-tetrahydro[1,2,4]triazolo[4,3-d][1,4]thiazepin-8-yl]methanol,
   [3-(3-chlorobiphenyl-4-yl)-8-methyl-5,6,8,9-tetrahydro[1,2,4]triazolo[4,3-d][1,4]thiazepin-8-yl]methanol,
   [3-(3-methoxybiphenyl-4-yl)-8-methyl-5,6,8,9-tetrahydro[1,2,4]triazolo[4,3-d][1,4]thiazepin-8-yl]methanol,
   [3-(3-chloro-3'-fluorobiphenyl-4-yl)-8-methyl-5,6,8,9-tetrahydro[1,2,4]triazolo[4,3-d][1,4]thiazepin-8-yl]methanol,
   [3-(3,3'-dichlorobiphenyl-4-yl)-8-methyl-5,6,8,9-tetrahydro[1,2,4]triazolo[4,3-d][1,4]thiazepin-8-yl]methanol,
   [3-(3-chloro-4'-fluorobiphenyl-4-yl)-8-methyl-5,6,8,9-tetrahydro[1,2,4]triazolo[4,3-d] [ 1,4]thiazepin-8-yl]methanol,
   [3-(3,4'-dichlorobiphenyl-4-yl)-8-methyl-5,6,8,9-tetrahydro[1,2,4]triazolo[4,3-d] [1,4]thiazepin-8-yl]methanol,
   [3-(3-chloro-4'-methoxycarbonylbiphenyl-4-yl)-8-methyl-5,6,8,9-tetrahydro[1,2,4]triazolo[4,3-d][1,4]thiazepin-8-yl]methanol,
   {3-[4'-fluoro-3-(trifluoromethyl)biphenyl-4-yl)-8-methyl-5,6,8,9-tetrahydro[1,2,4]triazolo[4,3-d][1,4]thiazepin-8-yl]methanol,
   [3-(3'-fluoro-3-methoxybiphenyl-4-yl)-8-methyl-5,6,8,9-tetrahydro[1,2,4]triazolo[4,3-d][1,4]thiazepin-8-yl]methanol,
   [3-(3'-chloro-3-methoxybiphenyl-4-yl)-8-methyl-5,6,8,9-tetrahydro[1,2,4]triazolo[4,3-d][1,4]thiazepin-8-yl]methanol,
   4'-[8-(hydroxymethyl)-8-methyl-5,6,8,9-tetrahydro[1,2,4]triazolo[4,3-d] [1,4]thiazepin-3-yl]-3'-methoxybiphenyl-3-carbonitrile,
   [3-(4'-fluoro-3-methoxybiphenyl-4-yl)-8-methyl-5,6,8,9-tetrahydro[1,2,4]triazolo[4,3-d][1,4]thiazepin-8-yl]methanol,
   [3-(3',4'-difluoro-3-methoxybiphenyl-4-yl)-8-methyl-5,6,8,9-tetrahydro[1,2,4]triazolo[4,3-d][1,4]thiazepin-8-yl]methanol,
   [3-(3'-chloro-4'-fluoro-3-methoxybiphenyl-4-yl)-8-methyl-5,6,8,9-tetrahydro[1,2,4]triazolo[4,3-d][1,4]thiazepin-8-yl]methanol,
   {3-[2-chloro-4-(pyridin-3-yl)phenyl]-8-methyl-5,6,8,9-tetrahydro[1,2,4]triazolo[4,3-d][1,4]thiazepin-8-yl}methanol,
   {3-[2-chloro-4-(pyridin-4-yl)phenyl]-8-methyl-5,6,8,9-tetrahydro[1,2,4]triazolo[4,3-d][1,4]thiazepin-8-yl}methanol,
   {3-[2-chloro-4-(morpholin-4-yl)phenyl]-8-methyl-5,6,8,9-tetrahydro[11,2,4]triazolo[4,3-d][1,4]thiazepin-8-yl}methanol,
   {3-[2-chloro-4-(piperidin-1-yl)phenyl]-8-methyl-5,6,8,9-tetrahydro[1,2,4]triazolo[4,3-d][1,4]thiazepin-8-yl}methanol,
   {3-[2-chloro-4-(pyrrolidin-1-yl)phenyl]-8-methyl-5,6,8,9-tetrahydro[1,2,4]triazolo[4,3-d][1,4]thiazepin-8-yl}methanol or
   {3-[2-chloro-4-(1H-pyrrol-1-yl)phenyl]-8-methyl-5,6,8,9-tetrahydro[1,2,4]triazolo[4,3-d][1,4]thiazepin-8-yl}methanol.
(13) the thiazepine derivative or pharmacologically acceptable salt thereof according to (1), wherein the compound having the general formula (I) is:
   [3-(3-chlorobiphenyl-4-yl)-8-methyl-5,6,8,9-tetrahydro[1,2,4]triazolo[4,3-d][1,4]thiazepin-8-yl]methanol,
   [3-(3-methoxybiphenyl-4-yl)-8-methyl-5,6,8,9-tetrahydro[1,2,4]triazolo[4,3-d][1,4]thiazepin-8-yl]methanol,
   [3-(3-chloro-3'-fluorobiphenyl-4-yl)-8-methyl-5,6,8,9-tetrahydro[1,2,4]triazolo[4,3-d][1,4]thiazepin-8-yl]methanol,
   [3-(3,3'-dichlorobiphenyl-4-yl)-8-methyl-5,6,8,9-tetrahydro[1,2,4]triazolo[4,3-d][1,4]thiazepin-8-yl]methanol,
   [3-(3-chloro-4'-fluorobiphenyl-4-yl)-8-methyl-5,6,8,9-tetrahydro[1,2,4]triazolo[4,3-d] [1,4]thiazepin-8-yl]methanol,
   [3-(3,4'-dichlorobiphenyl-4-yl)-8-methyl-5,6,8,9-tetrahydro[1,2,4]triazolo[4,3-d][1,4]thiazepin-8-yl]methanol,
   [3-(3'-fluoro-3-methoxybiphenyl-4-yl)-8-methyl-5,6,8,9-tetrahydro[1,2,4]triazolo[4,3-d][1,4]thiazepin-8-yl]methanol,
   [3-(3'-chloro-3-methoxybiphenyl-4-yl)-8-methyl-5,6,8,9-tetrahydro[1,2,4]triazolo[4,3-d][1,4]thiazepin-8-yl]methanol,
   4'-[8-(hydroxymethyl)-8-methyl-5,6,8,9-tetrahydro[1,2,4]triazolo[4,3-d][1,4]thiazepin-3-yl]-3'-methoxybiphenyl-3-carbonitrile,
   [3-(4'-fluoro-3-methoxybiphenyl-4-yl)-8-methyl-5,6,8,9-tetrahydro[1,2,4]triazolo[4,3-d][1,4]thiazepin-8-yl]methanol,
   [3-(3',4'-difluoro-3-methoxybiphenyl-4-yl)-8-methyl-5,6,8,9-tetrahydro[1,2,4]triazolo[4,3-d][1,4]thiazepin-8-yl]methanol or
   [3-(3'-chloro-4'-fluoro-3-methoxybiphenyl-4-yl)-8-methyl-5,6,8,9-tetrahydro[1,2,4]triazolo[4,3-d][1,4]thiazepin-8-yl]methanol;
(14) the thiazepine derivative or pharmacologically acceptable salt thereof according to any one of (1) to (11), wherein
   the general formula (I) is general formula (Ia) or (Ib): 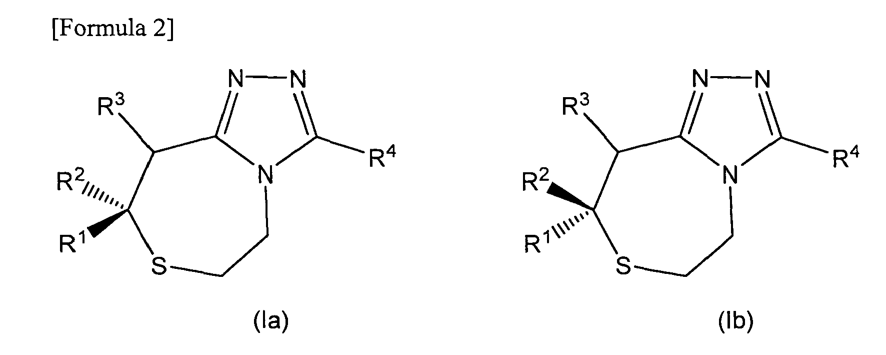
(15) the thiazepine derivative or pharmacologically acceptable salt thereof according to (14), wherein the compound having the general formula (Ia) or (Ib) is
   (-)-[3-(3-chloro-4'-fluorobiphenyl-4-yl)-8-methyl-5,6,8,9-tetrahydro[1,2,4]triazolo[4,3-d][1,4]thiazepin-8-yl]methanol or
   (+)-[3-(3-chloro-4'-fluorobiphenyl-4-yl)-8-methyl-5,6,8,9-tetrahydro[1,2,4]triazolo[4,3-d][1,4]thiazepin-8-yl]methanol;
(16) an 11β-hydroxysteroid dehydrogenase type 1 inhibitor containing the thiazepine derivative or pharmacologically acceptable salt thereof according to any one of (1) to (15) as an active ingredient;
(17) a pharmaceutical composition containing the thiazepine derivative or pharmacologically acceptable salt thereof according to any one of (1) to (15) as an active ingredient;
(18) the pharmaceutical composition according to (17), which has an effect of inhibiting 11β-hydroxysteroid dehydrogenase type 1;
(19) the pharmaceutical composition according to (17), which is used for therapeutic and/or prophylactic treatment of diabetes, adiposity, hyperlipemia, hypertension, or metabolic syndrome;
(20) the pharmaceutical composition according to (17), which is used for therapeutic and/or prophylactic treatment of diabetes;
(21) use of the thiazepine derivative or pharmacologically acceptable salt thereof according to any one of (1) to (15) for the manufacture of a pharmaceutical composition;
(22) the use according to (21), wherein the pharmaceutical composition is a composition for inhibiting 11β-hydroxysteroid dehydrogenase type 1;
(23) the use according to (21), wherein the pharmaceutical composition is a composition for therapeutic and/or prophylactic treatment of diabetes, adiposity, hyperlipemia, hypertension, or metabolic syndrome;
(24) the use according to (21), wherein the pharmaceutical composition is a composition for therapeutic and/or prophylactic treatment of diabetes;
(25) a method for inhibiting 11β-hydroxysteroid dehydrogenase type 1 comprising administering a pharmacologically effective amount thiazepine derivative or pharmacologically acceptable salt thereof according to any one of (1) to (15) to a warm-blooded animal;
(26) a method for therapeutic and/or prophylactic treatment of a disease, comprising administering a pharmacologically effective amount thiazepine derivative or pharmacologically acceptable salt thereof according to any one of (1) to (15) to a warm-blooded animal;
(27) the method according to (26), wherein the disease is diabetes, adiposity, hyperlipemia, hypertension, or metabolic syndrome;
(28) the method according to (26), wherein the disease is diabetes;
(29) the method according to any one of (25) to (28), wherein the warm-blooded animal is a human.

In the present invention, "halogen atom" means a fluorine atom, a chlorine atom, a bromine atom, or an iodine atom, preferably a fluorine atom, a chlorine atom, or a bromine atom, more preferably a fluorine atom or a chlorine atom.

In the present invention, "C₁-C₆ alkyl group" means a straight or branched alkyl group having 1 to 6 carbon atom(s). Examples thereof include a methyl group, an ethyl group, a propyl group, an isopropyl group, a butyl group, an isobutyl group, an s-butyl group, a t-butyl group, a pentyl group, an isopentyl group, a 2-methylbutyl group, a neopentyl group, a 1-ethylpropyl group, a hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, and a 1,2-dimethylbutyl group. A straight or branched alkyl group having 1 to 4 carbon atom(s) (C₁-C₄ alkyl group) is preferred, a methyl group or an ethyl group (C₁-C₂ alkyl group) is more preferred, and a methyl group is yet more preferred.

In the present invention, "C₁-C₆ halogenated alkyl group" means a group in which 1 to 5 of the above-mentioned "halogen atom" that are identical to or different from each other are bonded to the above-mentioned "C₁-C₆ alkyl group". Examples thereof include a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group, and a 2-fluoroethyl group. A group in which 1 to 5 of the above-mentioned "halogen atom" that are identical to or different from each other are bonded to the above-mentioned "C₁-C₄ alkyl group" (C₁-C₄ halogenated alkyl group) is preferred, a group in which 1 to 5 of the above-mentioned "halogen atom" that are identical to or different from each other are bonded to the above-mentioned "C₁-C₂ alkyl group" (C₁-C₂ halogenated alkyl group) is more preferred, and a trifluoromethyl group is yet more preferred.

In the present invention, "C₁-C₆ hydroxyalkyl group" is a group in which a hydroxy group is bonded to the above-mentioned "C₁-C₆ alkyl group". Examples thereof include a hydroxymethyl group, a hydroxyethyl group, and a hydroxypropyl group. A group in which a hydroxy group is bonded to a straight or branched alkyl group having 1 to 4 carbon atom(s) (C₁-C₄ alkyl group substituted with a hydroxy group) is preferred, a hydroxymethyl group or a 2-hydroxyethyl group is more preferred, and a hydroxymethyl group is yet more preferred.

In the present invention, "C₂-C₇ alkylcarbonyl group" means a group in which the above-mentioned "C₁-C₆ alkyl group" is bonded to a carbonyl group. Examples thereof include an acetyl group, an ethylcarbonyl group, a propylcarbonyl group, an isopropylcarbonyl group, a butylcarbonyl group, an isobutylcarbonyl group, an s-butylcarbonyl group, a t-butylcarbonyl group, a pentylcarbonyl group, an isopentylcarbonyl group, a 2-methylbutylcarbonyl group, and a neopentylcarbonyl group. A group in which the above-mentioned "C₁-C₄ alkyl group" is bonded to a carbonyl group (C₂-C₅ alkylcarbonyl group) is preferred, an acetyl group or an ethylcarbonyl group (C₂-C₃ alkylcarbonyl group) is more preferred, and an acetyl group is yet more preferred.

In the present invention, "C₁-C₆ alkoxy group" means a group in which the above-mentioned "C₁-C₆ alkyl group" is bonded to an oxygen atom, specifically a straight or branched alkoxy group having 1 to 6 carbon atom(s). Examples thereof include a methoxy group, an ethoxy group, a propoxy group, an isopropoxy group, a butoxy group, an isobutoxy group, an s-butoxy group, a t-butoxy group, a pentoxy group, an isopentoxy group, a 2-methylbutoxy group, a 1-ethylpropoxy group, a 2-ethylpropoxy group, and a neopentoxy group. A straight or branched alkoxy group having 1 to 4 carbon atom(s) (C₁-C₄ alkoxy group) is preferred, a methoxy group or an ethoxy group (C₁-C₂ alkoxy group) is more preferred, and a methoxy group is yet more preferred.

In the present invention, "C₁-C₆ halogenated alkoxy group" means a group in which 1 to 5 of the above-mentioned "halogen atom" that are identical to or different from each other are bonded to the above-mentioned "C₁-C₆ alkoxy group". Examples thereof include a trifluoromethoxy group, a trichloromethoxy group, a difluoromethoxy group, a dichloromethoxy group, a dibromomethoxy group, a fluoromethoxy group, a 2,2,2-trifluoroethoxy group, a 2,2,2-trichloroethoxy group, a 2-bromoethoxy group, a 2-chloroethoxy group, and a 2-fluoroethoxy group. A group in which 1 to 5 of the above-mentioned "halogen atom" that are identical to or different from each other are bonded to the above-mentioned "C₁-C₄ alkoxy group" (C₁-C₄ halogenated alkoxy group) is preferred, a group in which 1 to 5 of the above-mentioned "halogen atom" that are identical to or different from each other are bonded to the above-mentioned "C₁-C₂ alkoxy group" (C₁-C₂ halogenated alkoxy group) is more preferred, and a trifluoromethoxy group is yet more preferred.

In the present invention, "(C₁-C₆ alkoxy)-(C₁-C₆ alkyl) group" means a group in which one of the above-mentioned "C₁-C₆ alkoxy group" is bonded to the above-mentioned "C₁-C₆ alkyl group". Examples thereof include a methoxymethyl group, an ethoxymethyl group, a propoxymethyl group, an isopropoxymethyl group, a butoxymethyl group, an isobutoxymethyl group, an s-butoxymethyl group, a t-butoxymethyl group, a 2-methoxyethyl group, a 2-ethoxyethyl group, a 2-isopropoxyethyl group, and a 2-(t-butoxy)ethyl group. A group in which 1 above-mentioned "C₁-C₄ alkoxy group" is bonded to the above-mentioned "C₁-C₄ alkyl group" ((C₁-C₄ alkoxy)-(C₁-C₄ alkyl) group) is preferred, a group in which one of the above-mentioned "C₁-C₂ alkoxy group" is bonded to the above-mentioned "C₁-C₂ alkyl group" ((C₁-C₂ alkoxy)-(C₁-C₂ alkyl) group) is more preferred, and a methoxymethyl group is yet more preferred.

In the present invention, "C₃-C₆ cycloalkyl group" means a cyclopropyl group, a cyclobutyl group, a cyclopentyl group, or a cyclohexyl group. A cyclopropyl group is preferred.

In the present invention, "C₁-C₆ alkyl group substituted with one C₃-C₆ cycloalkyl group" means a group in which one of the above-mentioned "C₃-C₆ cycloalkyl group" is bonded to the above-mentioned "C₁-C₆ alkyl group". Examples thereof include a cyclopropylmethyl group, a cyclobutylmethyl group, a cyclopentylmethyl group, a cyclohexylmethyl group, a cyclopropylethyl group, and a cyclobutylethyl group. A cyclopropylmethyl group is preferred.

In the present invention, "C₂-C₇ alkoxycarbonyl group" means a group in which one of the above-mentioned "C₁-C₆ alkoxy group" is bonded to a carbonyl group. Examples thereof include a methoxycarbonyl group, an ethoxycarbonyl group, a propoxycarbonyl group, an isopropoxycarbonyl group, a butoxycarbonyl group, an isobutoxycarbonyl group, an s-butoxycarbonyl group, and a t-butoxycarbonyl group. A group in which one of the above-mentioned "C₁-C₄ alkoxy group" is bonded to a carbonyl group (C₂-C₅ alkoxycarbonyl group) is preferred, a methoxycarbonyl group or an ethoxycarbonyl group (C₂-C₃ alkoxycarbonyl group) is more preferred, and a methoxycarbonyl group is yet more preferred.

In the present invention, "C₂-C₇ alkylcarbonyloxy group" means a group in which a carbonyl group bound with one of the above-mentioned "C₁-C₆ alkyl group" is bonded to an oxygen atom. Examples thereof include an acetoxy group, a propionyloxy group, a butyryloxy group, an isobutyryloxy group, a pentanoyloxy group, a pivaloyloxy group, a valeryloxy group, and an isovaleryloxy group. A group in which a carbonyl group bound with one of the above-mentioned "C₁-C₄ alkyl group" is bonded to an oxygen atom (C₂-C₅ alkylcarbonyloxy group) is preferred, an acetoxy group or a propionyloxy group (C₂-C₃ alkylcarbonyloxy group) is more preferred, and an acetoxy group is yet more preferred.

In the present invention, "C₁-C₆ alkylthio group" means a group in which one of the above-mentioned "C₁-C₆ alkyl group" is bonded to a sulfur atom, specifically a straight or branched alkylthio group having 1 to 6 carbon atom(s). Examples thereof include a methylthio group, an ethylthio group, a propylthio group, an isopropylthio group, a butylthio group, an isobutylthio group, an s-butylthio group, a t-butylthio group, and a pentylthio group. A straight or branched alkylthio group having 1 to 4 carbon atom(s) (C₁-C₄ alkylthio group) is preferred, a methylthio group or an ethylthio group (C₁-C₂ alkylthio group) is more preferred, and a methylthio group is yet more preferred.

In the present invention, "C₁-C₆ alkylsulfonyl group" means a group in which one of the above-mentioned "C₁-C₆ alkyl group" is bonded to a sulfonyl group, specifically a straight or branched alkylsulfonyl group having 1 to 6 carbon atom(s). Examples thereof include a methylsulfonyl group, an ethylsulfonyl group, a propylsulfonyl group, an isopropylsulfonyl group, a butylsulfonyl group, an isobutylsulfonyl group, an s-butylsulfonyl group, and a t-butylsulfonyl group. A straight or branched alkylsulfonyl group having 1 to 4 carbon atom(s) (C₁-C₄ alkylsulfonyl group) is preferred, a methylsulfonyl group or an ethylsulfonyl group (C₁-C₂ alkylsulfonyl group) is more preferred, and a methylsulfonyl group is yet more preferred.

In the present invention, "mono-C₁-C₆ alkylamino group" means a group in which one of the above-mentioned "C₁-C₆ alkyl group" is bonded to an amino group. Examples thereof include a methylamino group, an ethylamino group, a propylamino group, an isopropylamino group, a butylamino group, an isobutylamino group, an s-butylamino group, a t-butylamino group, a pentylamino group, an isopentylamino group, a 2-methylbutylamino group, a neopentylamino group, a 1-ethylpropylamino group, a hexylamino group, an isohexylamino group, and a 4-methylpentylamino group. A group in which one -of the above-mentioned "C₁-C₄ alkyl group" is bonded to an amino group (mono-C₁-C₄ alkylamino group) is preferred, a methylamino group or an ethylamino group (mono-C₁-C₂ alkylamino group) is more preferred, and a methylamino group is yet more preferred.

In the present invention, "di-(C₁-C₆ alkyl)amino group" means a group in which two of the above-mentioned "C₁-C₆ alkyl group" that are identical to or different from each other are bonded to an amino group. Examples thereof include a dimethylamino group, a diethylamino group, a dipropylamino group, a diisopropylamino group, a dibutylamino group, a diisobutylamino group, a dipentylamino group, a diisopentylamino group, a dineopentylamino group, a dihexylamino group, an N-ethyl-N-methylamino group, an N-methyl-N-propylamino group, and an N-isopropyl-N-methylamino group. A group in which two of the above-mentioned "C₁-C₄ alkyl group" that are identical to or different from each other are bonded to an amino group (di-(C₁-C₄ alkyl)amino group) is preferred, a dimethylamino group, a diethylamino group, or an N-ethyl-N-methylamino group (di-(C₁-C₂ alkyl)amino group) is more preferred, and a dimethylamino group is yet more preferred.

In the present invention, "mono-(C₁-C₆ alkyl)aminocarbonyl group" means a group in which an amino group bound with one of the above-mentioned "C₁-C₆ alkyl group" is bonded to a carbonyl group. Examples thereof include a methylaminocarbonyl group, an ethylaminocarbonyl group, a propylaminocarbonyl group, an isopropylaminocarbonyl group, a butylaminocarbonyl group, an isobutylaminocarbonyl group, an s-butylaminocarbonyl group, a t-butylaminocarbonyl group, a pentylaminocarbonyl group, an isopentylaminocarbonyl group, and a 2-methylbutylaminocarbonyl group. A group in which an amino group bound with one of the above-mentioned "C₁-C₄ alkyl group" is bonded to a carbonyl group (mono-(C₁-C₄ alkyl)aminocarbonyl group) is preferred, a methylaminocarbonyl group or an ethylaminocarbonyl group (mono-(C₁-C₂ alkyl)aminocarbonyl group) is more preferred, and a methylaminocarbonyl group is yet more preferred.

In the present invention, "mono-C₂-C₇ alkylcarbonylamino group" means a group in which a carbonyl group bound with one of the above-mentioned "C₁-C₆ alkyl group" is bonded to an amino group. Examples thereof include an acetamide group, an ethylcarbonylamino group, a propylcarbonylamino group, an isopropylcarbonylamino group, a butylcarbonylamino group, an isobutylcarbonylamino group, an s-butylcarbonylamino group, and a t-butylcarbonylamino group. A group in which a carbonyl group bound with one of the above-mentioned "C₁-C₄ alkyl group" is bonded to an amino group (mono-C₂-C₅ alkylcarbonylamino group) is preferred, an acetamide group or an ethylcarbonylamino group (mono-C₂-C₃ alkylcarbonylamino group) is more preferred, and an acetamide group is yet more preferred.

In the present invention, "mono-C₁-C₆ alkylsulfonylamino group" means a group in which one of the above-mentioned "C₁-C₆ alkylsulfonyl group" is bonded to an amino group, specifically a straight or branched alkylsulfonylamino group having 1 to 6 carbon atom(s). Examples thereof include a methylsulfonylamino group, an ethylsulfonylamino group, a propylsulfonylamino group, and an isopropylsulfonylamino group. A straight or branched alkylsulfonylamino group having 1 to 4 carbon atom(s) (C₁-C₄ alkylsulfonylamino group) is preferred, a methylsulfonylamino group or an ethylsulfonylamino group (C₁-C₂ alkylsulfonylamino group) is more preferred, and a methylsulfonylamino group is yet more preferred.

In the present invention, "C₆-C₁₀ aryl group" means an aromatic hydrocarbon group having 6 to 10 carbon atoms. A phenyl group or a naphthyl group is preferred, and a phenyl group is more preferred.

In the present invention, "heterocyclic group" means a 4 to 7-membered heterocyclic group that includes 1 to 3 sulfur atom(s), oxygen atom(s) and/or nitrogen atom(s) and may further include 1 or 2 nitrogen atom(s), in which the sulfur atom may be bonded to 2 oxygen atoms. Examples thereof include "aromatic heterocyclic group" such as a furyl group, a thienyl group, a pyrrolyl group, an azepinyl group, a pyrazolyl group, an imidazolyl group, an oxazolyl group, an isoxazolyl group, a thiazolyl group, an isothiazolyl group, a 1,2,3-oxadiazolyl group, a triazolyl group, a tetrazolyl group, a thiadiazolyl group, a pyranyl group, a pyridyl group, a pyridazinyl group, a pyrimidinyl group, or a pyrazinyl group, and "partially or completely reduced saturated heterocyclic group" such as a tetrahydropyranyl group, a tetrahydrothienyl group, a morpholinyl group, thiomorpholinyl group, a pyrrolidinyl group, a pyrrolinyl group, an imidazolidinyl group, a pyrazolidinyl group, a piperidinyl group, a piperazinyl group, an oxazolidinyl group, an isoxazolidinyl group, a thiazolidinyl group, a pyrazolidinyl group, a dioxolanyl group, or a dioxanyl group. The above-mentioned heterocyclic group may be condensed with another cyclic group such as a benzene ring ("condensed bicyclic heterocyclic group"), and examples thereof include a benzothienyl group, a benzothiazolyl group, a benzooxazolyl group, an isobenzofuranyl group, a 1,3-dihydroisobenzofuranyl group, a quinolyl group, a 1,3-benzodioxolanyl group, a 1,4-benzodioxanyl group, an indolyl group, an isoindolyl group, and an indolinyl group. In R⁴, a condensed bicyclic heterocyclic group is preferred, an indolyl group or a benzothiophenyl group is more preferred, and a 4-indolyl group or a 2-benzothiophenyl group is yet more preferred. In the Substituent Group a, a 5 or 6-membered heterocyclic group that includes 1 nitrogen atom and may further include 1 oxygen atom is preferred, a pyridyl group, a morpholinyl group, a piperidinyl group, a pyrrolidinyl group, or a pyrrolyl group is more preferred, and a pyridin-4-yl group, a pyridin-3-yl group, a morpholin-4-yl group, a piperidin-1-yl group, a pyrrolidin-1-yl group, or a 1H-pyrrol-1-yl group is yet more preferred.

In the present invention, "C₆-C₁₀ aryl group that may be substituted with 1 to 5 group(s) independently selected from Substituent Group b" means the above-mentioned "C₆-C₁₀ aryl group" substituted with 1 to 5 group(s) independently selected from Substituent Group b. Phenyl group that may be substituted with 1 to 5 group(s) independently selected from a halogen atom, a C₂-C₇ alkoxycarbonyl group, and a cyano group is preferred, phenyl group that may be substituted with 1 or 2 group(s) independently selected from a fluorine atom, a chlorine atom, and a cyano group is more preferred, and phenyl group that may be substituted with 1 or 2 group(s) independently selected from a fluorine atom, a chlorine atom, and a cyano group is yet more preferred, and a 3-fluorophenyl group, a 3-chlorophenyl group, a 4-fluorophenyl group, a 4-chlorophenyl group, a 3-cyanophenyl group, a 3,4-difluorophenyl group, or a 3-chloro-4-fluorophenyl group is particularly preferred.

In the present invention, "phenyloxy group that may be substituted with 1 to 5 group(s) independently selected from Substituent Group b" means a phenyloxy group that may be substituted with 1 to 5 group(s) independently selected from Substituent Group b. Phenyloxy group that may be substituted with 1 to 3 halogen atom(s) is preferred, and a 4-fluorophenyloxy group is more preferred.

In the present invention, "phenylmethyloxy group that may be substituted with 1 to 5 group(s) independently selected from Substituent Group b" means a phenylmethyloxy group that may be substituted with 1 to 5 group(s) independently selected from Substituent Group b. Phenylmethyloxy group that may be substituted with 1 to 3 halogen atom(s) is preferred, and a 4-fluorophenylmethyloxy group is more preferred.

In the present invention, "phenylsulfonyl group that may be substituted with 1 to 5 group(s) independently selected from Substituent Group b" means a phenylsulfonyl group that may be substituted with 1 to 5 group(s) independently selected from Substituent Group b. Phenylsulfonyl group that may be substituted with 1 to 3 halogen atom(s) is preferred, and a phenylsulfonyl group or a 4-fluorophenylsulfonyl group is more preferred.

In the present invention, "heterocyclic group that may be substituted with 1 to 3 group(s) independently selected from Substituent Group b" means the above-mentioned "heterocyclic group" that may be substituted with 1 to 3 group(s) independently selected from Substituent Group b. A morpholin-4-yl group, a piperidin-1-yl group, a pyrrolidin-1-yl group, or a 1H-pyrrol-1-yl group is preferred.

In the present invention, "C₆-C₁₀ aryl group that may be substituted with 1 to 5 group(s) independently selected from Substituent Group a" means the above-mentioned "C₆-C₁₀ aryl group" that may be substituted with 1 to 5 group(s) independently selected from Substituent Group a. Phenyl group that may be substituted with 1 to 5 group(s) independently selected from [a halogen atom, a C₁-C₆ halogenated alkyl group, a C₁-C₆ alkoxy group, a phenyl group that may be substituted with 1 to 5 group(s) independently selected from (a halogen atom, a C₂-C₇ alkoxycarbonyl group, and a cyano group), a pyridin-4-yl group, a pyridin-3-yl group, a morpholin-4-yl group, a piperidin-1-yl group, a pyrrolidin-1-yl group, and a 1H-pyrrol-1-yl group] is preferred, phenyl group that is substituted with 2 groups independently selected from [a chlorine atom, a methoxy group, and a phenyl group that may be substituted with 1 or 2 group(s) independently selected from (a fluorine atom, a chlorine atom, and a cyano group)] is more preferred, and a 3-chlorobiphenyl-4-yl group, a 3-methoxybiphenyl-4-yl group, a 3-chloro-3'-fluorobiphenyl group, a 3,3'-dichlorobiphenyl-4-yl group, a 3-chloro-4'-fluorobiphenyl-4-yl group, a 3,4'-dichlorobiphenyl-4-yl group, a 3'-fluoro-3-methoxybiphenyl-4-yl group, a 3'-chloro-3-methoxybiphenyl-4-yl group, a 3'-cyano-3-methoxybiphenyl-4-yl group, a 4'-fluoro-3-methoxybiphenyl-4-yl group, a 3',4'-difluoro-3-methoxybiphenyl-4-yl group, or a 3'-chloro-4'-fluoro-3-methoxybiphenyl-4-yl group is yet more preferred.

In the present invention, "heterocyclic group that may be substituted with 1 to 3 group(s) independently selected from Substituent Group a" means the above-mentioned "heterocyclic group" that may be substituted with 1 to 3 group(s) independently selected from Substituent Group a. An indolyl group that may be substituted with 1 to 3 group(s) independently selected from Substituent Group a or a benzothiophenyl group that may be substituted with 1 to 3 group(s) independently selected from Substituent Group a is preferred, and an indolyl group that may be substituted with 1 to 3 group(s) independently selected from (a C₁-C₆ alkyl group and a C₁-C₆ alkyl group substituted with one C₃-C₆ cycloalkyl group) or a benzothiophenyl group that may be substituted with 1 to 3 group(s) independently selected from (a halogen atom and a C₁-C₆ alkyl group) is more preferred.

In the present invention, R¹ preferably represents a C₁-C₆ alkyl group, more preferably a methyl group.

In the present invention, R² preferably represents a C₁-C₆ hydroxyalkyl group, more preferably a hydroxymethyl group.

In the present invention, R³ preferably represents a hydrogen atom.

In the present invention, R⁴ preferably represents a phenyl group that may be substituted with 1 to 5 group(s) independently selected from Substituent Group a, an indolyl group that may be substituted with 1 to 3 group(s) independently selected from Substituent Group a, or a benzothiophenyl group that may be substituted with 1 to 3 group(s) independently selected from Substituent Group a, more preferably a phenyl group that may be substituted with 1 to 5 group(s) independently selected from [a halogen atom, a C₁-C₆ halogenated alkyl group, a C₁-C₆ alkoxy group, a phenyl group that may be substituted with 1 to 5 group(s) independently selected from (a halogen atom, a C₂-C₇ alkoxycarbonyl group, and a cyano group), a pyridin-4-yl group, a pyridin-3-yl group, a morpholin-4-yl group, a piperidin-1-yl group, a pyrrolidin-1-yl group, and a 1H-pyrrol-1-yl group], an indolyl group that may be substituted with 1 to 3 group(s) independently selected from (a C₁-C₆ alkyl group and a C₁-C₆ alkyl group substituted with one C₃-C₆ cycloalkyl group), or a benzothiophenyl group that may be substituted with 1 to 3 group(s) independently selected from (a halogen atom and a C₁-C₆ alkyl group), yet more preferably a phenyl group that is substituted with 2 groups independently selected from [a chlorine atom, a methoxy group, and a phenyl group that may be substituted with 1 or 2 group(s) independently selected from (a fluorine atom, a chlorine atom, and a cyano group)], particularly preferably a 3-chlorobiphenyl-4-yl group, a 3-methoxybiphenyl-4-yl group, a 3-chloro-3'-fluorobiphenyl group, a 3,3'-dichlorobiphenyl-4-yl group, a 3-chloro-4'-fluorobiphenyl-4-yl group, a 3,4'-dichlorobiphenyl-4-yl group, a 3'-fluoro-3-methoxybiphenyl-4-yl group, a 3'-chloro-3-methoxybiphenyl-4-yl group, a 3'-cyano-3-methoxybiphenyl-4-yl group, a 4'-fluoro-3-methoxybiphenyl-4-yl group, a 3',4'-difluoro-3-methoxybiphenyl-4-yl group, or a 3'-chloro-4'-fluoro-3-methoxybiphenyl-4-yl group.

The thiazepine derivative or pharmacologically acceptable salt thereof having the general formula (I) of the present invention includes all isomers (keto-enol isomer, diastereoisomer, optical isomer, rotational isomer, etc.).

The thiazepine derivative or pharmacologically acceptable salt thereof having the general formula (I) of the present invention has various isomers because asymmetric carbon atom(s) exist in the molecule. In the compounds of the present invention, these isomers and mixtures of these isomers are represented by a single formula, that is, the general formula (I). Therefore, the present invention also includes all these isomers and mixtures of these isomers in arbitrary ratios.

The above-mentioned stereoisomers can be obtained by using stereospecific starting compounds, or by synthesizing the compounds of the present invention using asymmetric synthesis or asymmetric induction techniques, or by isolating the synthesized compounds of the present invention by usual optical resolution or isolation methods as desired.

The "pharmacologically acceptable salt thereof' means a salt of the thiazepine derivative having the general formula (I) of the present invention because it forms a salt by reacting with an acid when it has a basic group such as an amino group or by reacting with a base when it has an acidic group such as a carboxyl group.

Examples of salts of a basic group include inorganic acid salts including hydrohalides such as hydrofluorides, hydrochlorides, hydrobromides, and hydroiodides, nitrates, perchlorates, sulfates, and phosphates; organic acid salts including C₁-C₆ alkylsulfonic acid salts such as methanesulfonates, trifluoromethanesulfonates, and ethanesulfonates, arylsulfonic acid salts such as benzenesulfonates and p-toluenesulfonates, acetates, malates, fumarates, succinates, citrates, ascorbates, tartarates, oxalates, and maleates; and amino acid salts such as glycine salts, lysine salts, arginine salts, ornithine salts, glutamic acid salts, and aspartic acid salts.

Meanwhile, examples of salts of an acidic group include metal salts including alkali metal salts such as sodium salts, potassium salts, and lithium salts, alkaline earth metal salts such as calcium salts and magnesium salts, aluminium salts, and iron salts; and amine salts including inorganic salts such as ammonium salts and organic salts such as t-octylamine salts, dibenzylamine salts, morpholine salts, glucosamine salts, phenyl glycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanol amine salts, N-benzylphenethylamine salts, piperazine salts, tetramethyl ammonium salts, and tris(hydroxymethyl)aminomethane salts; and amino acid salts such as glycine salts, lysine salts, arginine salts, ornithine salts, glutamic acid salts, and aspartic acid salts.

When the thiazepine derivative or pharmacologically acceptable salt thereof having the general formula (I) of the present invention is left in the atmosphere or recrystallized, a hydrate may be formed due to absorption of moisture or attachment of adsorbed water. Such hydrates also fall within the salts of the present invention.

The thiazepine derivative or pharmacologically acceptable salt thereof having the general formula (I) of the present invention may absorb other specific solvents to form solvates, and such solvates also fall within the salts of the present invention.

Specific examples of compounds having the general formula (I) of the present invention include the compounds shown in the following Tables 1 and 2, but the present invention is not limited these groups.

Abbreviations in the following Tables 1 and 2 have the following meanings. Specifically,
Me represents a methyl group,
Et represents an ethyl group,
cPr represents a cyclopropyl group,
Ph represents a phenyl group,
1-Naph represents a 1-naphthyl group,
4-OMe-C₆H₄ represents a 4-methoxyphenyl group,
2-Cl-4-(3,4-di-F-C₆H₃)-C₆H₃ represents a 2-chloro-4-(3,4-difluorophenyl)phenyl group,
2-Cl-4-OCH₂(4-F-C₆H₄)-C₆H₃ represents a 2-chloro-4-(4-fluorophenyl)methyloxyphenyl group,
Ph(A) represents a 2-chloro-4-(morpholin-4-yl)phenyl group,
Ph(B) represents a 2-chloro-4-(piperidin-1-yl)phenyl group,
Ph(C) represents a 2-chloro-4-(pyrrolidin-1-yl)phenyl group,
Ph(D) represents a 2-chloro-4-(1H-pyrrol-1-yl)phenyl group,
Ph(E) represents a 2-chloro-4-(4-methylsulfonylpiperazin-1-yl)phenyl group,
Ph(F) represents a 2-chloro-4-(2-oxopyrrolidin-1-yl)phenyl group,
Ph(G) represents a 2-methoxy-4-(morpholin-4-yl)phenyl group,
Ph(H) represents a 2-methoxy-4-(piperidin-1-yl)phenyl group,
Ph(I) represents a 2-methoxy-4-(pyrrolidin-1-yl)phenyl group,
Ph(J) represents a 2-methoxy-4-(1H-pyrrol-1-yl)phenyl group,
Ph(K) represents a 2-methoxy-4-(4-methylsulfonylpiperazin-1-yl)phenyl group,
Ph(L) represents a 2-methoxy-4-(2-oxopyrrolidin-1-yl)phenyl group,
Het(A) represents a 1-methyl-4-indolyl group,
Het(B) represents a 1-cyclopropylmethyl-4-indolyl group,
Het(C) represents a 5-chloro-3-methyl-2-benzothiophenyl group,
Het(D) represents a 1-phenyl-4-indolyl group,
Het(E) represents a 1-(4-fluorophenyl)-4-indolyl group,
Het(F) represents a 1-phenylsulfonyl-4-indolyl group, and
Het(G) represents a 1-(4-fluorophenylsulfonyl)-4-indolyl group.

**(Table 1)**

| | |
|---|---|
| 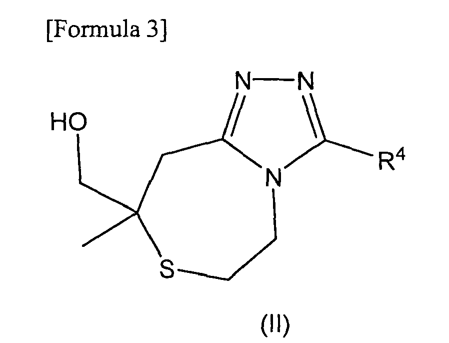 | |

| Compound No. | R⁴ |
|---|---|
| 1-1 | C₆H₅ |
| 1-2 | 4-F-C₆H₄ |
| 1-3 | 3-F-C₆H₄ |
| 1-4 | 2-F-C₆H₄ |
| 1-5 | 4-Cl-C₆H₄ |
| 1-6 | 3-Cl-C₆H₄ |
| 1-7 | 2-Cl-C₆H₄ |
| 1-8 | 4-Me-C₆H₄ |
| 1-9 | 3-Me-C₆H₄ |
| 1-10 | 2-Me-C₆H₄ |
| 1-11 | 4-Et-C₆H₄ |
| 1-12 | 3-Et-C₆H₄ |
| 1-13 | 2-Et-C₆H₄ |
| 1-14 | 4-OMe-C₆H₄ |
| 1-15 | 3-OMe-C₆H₄ |
| 1-16 | 2-OMe-C₆H₄ |
| 1-17 | 4-OEt-C₆H₄ |
| 1-18 | 3-OEt-C₆H₄ |
| 1-19 | 2-OEt-C₆H₄ |
| 1-20 | 4-CF₃-C₆H₄ |
| 1-21 | 3-CF₃-C₆H₄ |
| 1-22 | 2-CF₃-C₆H₄ |
| 1-23 | 4-OCF₃-C₆H₄ |
| 1-24 | 3-OCF₃-C₆H₄ |
| 1-25 | 2-OCF₃-C₆H₄ |
| 1-26 | 2-Cl-3-F-C₆H₃ |
| 1-27 | 2-Cl-4-F-C₆H₃ |
| 1-28 | 2-Cl-5-F-C₆H₃ |
| 1-29 | 3-Cl-2-Me-C₆H₃ |
| 1-30 | 3-Cl-4-Me-C₆H₃ |
| 1-31 | 3-Cl-5-Me-C₆H₃ |
| 1-32 | 2-OMe-3-CF₃-C₆H₃ |
| 1-33 | 2-OMe-4-CF₃-C₆H₃ |
| 1-34 | 2-OMe-5-CF₃-C₆H₃ |
| 1-35 | 1-Naph |
| 1-36 | 2-Naph |
| 1-37 | Het(A) |
| 1-38 | Het(B) |
| 1-39 | Het(C) |
| 1-40 | 2-Cl-4-Ph-C₆H₃ |
| 1-41 | 2-OMe-4-Ph-C₆H₃ |
| 1-42 | 2-F-4-(2-F-C₆H₄)-C₆H₃ |
| 1-43 | 4-(2-Cl-C₆H₄)-2-F-C₆H₃ |
| 1-44 | 2-F-4-(3-F-C₆H₄)-C₆H₃ |
| 1-45 | 4-(3-Cl-C₆H₄)-2-F-C₆H₃ |
| 1-46 | 2-F-4-(3-Me-C₆H₄)-C₆H₃ |
| 1-47 | 2-F-4-(3-CF₃-C₆H₄)-C₆H₃ |
| 1-48 | 2-F-4-(3-OMe-C₆H₄)-C₆H₃ |
| 1-49 | 4-(3-CN-C₆H₄)-2-F-C₆H₃ |
| 1-50 | 2-F-4-(3-NHSO₂Me-C₆H₄)-C₆H₃ |
| 1-51 | 2-F-4-(4-F-C₆H₄)-C₆H₃ |
| 1-52 | 4-(4-Cl-C₆H₄)-2-F-C₆H₃ |
| 1-53 | 2-F-4-(4-Me-C₆H₄)-C₆H₃ |
| 1-54 | 2-F-4-(4-CF₃-C₆H₄)-C₆H₃ |
| 1-55 | 2-F-4-(4-OMe-C₆H₄)-C₆H₃ |
| 1-56 | 2-F-4-(4-OCF₃-C₆H₄)-C₆H₃ |
| 1-57 | 4-(4-CN-C₆H₄)-2-F-C₆H₃ |
| 1-58 | 4-(4-CO₂H-C₆H₄)-2-F-C₆H₃ |
| 1-59 | 2-F-4-(4-CO₂Me-C₆H₄)-C₆H₃ |
| 1-60 | 2-Cl-4-(2-F-C₆H₄)-C₆H₃ |
| 1-61 | 2-Cl-4-(2-Cl-C₆H₄)-C₆H₃ |
| 1-62 | 2-Cl-4-(3-F-C₆H₄)-C₆H₃ |
| 1-63 | 2-Cl-4-(3-Cl-C₆H₄)-C₆H₃ |
| 1-64 | 2-Cl-4-(3-Me-C₆H₄)-C₆H₃ |
| 1-65 | 2-Cl-4-(3-CF₃-C₆H₄)-C₆H₃ |
| 1-66 | 2-Cl-4-(3-OMe-C₆H₄)-C₆H₃ |
| 1-67 | 2-Cl-4-(3-CN-C₆H₄)-C₆H₃ |
| 1-68 | 2-Cl-4-(3-NHSO₂Me-C₆H₄)-C₆H₃ |
| 1-69 | 2-Cl-4-(4-F-C₆H₄)-C₆H₃ |
| 1-70 | 2-Cl-4-(4-Cl-C₆H₄)-C₆H₃ |
| 1-71 | 2-Cl-4-(4-Me-C₆H₄)-C₆H₃ |
| 1-72 | 2-Cl-4-(4-CF₃-C₆H₄)-C₆H₃ |
| 1-73 | 2-Cl-4-(4-OMe-C₆H₄)-C₆H₃ |
| 1-74 | 2-Cl-4-(4-OCF₃-C₆H₄)-C₆H₃ |
| 1-75 | 2-Cl-4-(4-CN-C₆H₄)-C₆H₃ |
| 1-76 | 4-(4-CO₂H-C₆H₄)-2-Cl-C₆H₃ |
| 1-77 | 2-Cl-4-(4-CO₂Me-C₆H₄)-C₆H₃ |
| 1-78 | 4-(2-F-C₆H₄)-2-CF₃-C₆H₃ |
| 1-79 | 4-(2-Cl-C₆H₄)-2-CF₃-C₆H₃ |
| 1-80 | 4-(3-F-C₆H₄)-2-CF₃-C₆H₃ |
| 1-81 | 4-(3-Cl-C₆H₄)-2-CF₃-C₆H₃ |
| 1-82 | 4-(3-Me-C₆H₄)-2-CF₃-C₆H₃ |
| 1-83 | 2-CF₃-4-(3-CF₃-C₆H₄)-C₆H₃ |
| 1-84 | 4-(3-OMe-C₆H₄)-2-CF₃-C₆H₃ |
| 1-85 | 4-(3-CN-C₆H₄)-2-CF₃-C₆H₃ |
| 1-86 | 4-(3-NHSO₂Me-C₆H₄)-2-CF₃-C₆H₃ |
| 1-87 | 4-(4-F-C₆H₄)-2-CF₃-C₆H₃ |
| 1-88 | 4-(4-Cl-C₆H₄)-2-CF₃-C₆H₃ |
| 1-89 | 4-(4-Me-C₆H₄)-2-CF₃-C₆H₃ |
| 1-90 | 2-CF₃-4-(4-CF₃-C₆H₄)-C₆H₃ |
| 1-91 | 4-(4-OMe-C₆H₄)-2-CF₃-C₆H₃ |
| 1-92 | 4-(4-OCF₃-C₆H₄)-2-CF₃-C₆H₃ |
| 1-93 | 4-(4-CN-C₆H₄)-2-CF₃-C₆H₃ |
| 1-94 | 4-(4-CO₂H-C₆H₄)-2-CF₃-C₆H₃ |
| 1-95 | 4-(4-CO₂Me-C₆H₄)-2-CF₃-C₆H₃ |
| 1-96 | 4-(2-F-C₆H₄)-2-OMe-C₆H₃ |
| 1-97 | 4-(2-Cl-C₆H₄)-2-OMe-C₆H₃ |
| 1-98 | 4-(3-F-C₆H₄)-2-OMe-C₆H₃ |
| 1-99 | 4-(3-Cl-C₆H₄)-2-OMe-C₆H₃ |
| 1-100 | 2-OMe-4-(3-Me-C₆H₄)-C₆H₃ |
| 1-101 | 2-OMe-4-(3-CF₃-C₆H₄)-C₆H₃ |
| 1-102 | 2-OMe-4-(3-OMe-C₆H₄)-C₆H₃ |
| 1-103 | 4-(3-CN-C₆H₄)-2-OMe-C₆H₃ |
| 1-104 | 2-OMe-4-(3-NHSO₂Me-C₆H₄)-C₆H₃ |
| 1-105 | 4-(4-F-C₆H₄)-2-OMe-C₆H₃ |
| 1-106 | 4-(4-Cl-C₆H₄)-2-OMe-C₆H₃ |
| 1-107 | 2-OMe-4-(4-Me-C₆H₄)-C₆H₃ |
| 1-108 | 2-OMe-4-(4-CF₃-C₆H₄)-C₆H₃ |
| 1-109 | 2-OMe-4-(4-OMe-C₆H₄)-C₆H₃ |
| 1-110 | 2-OMe-4-(4-OCF₃-C₆H₄)-C₆H₃ |
| 1-111 | 4-(4-CN-C₆H₄)-2-OMe-C₆H₃ |
| 1-112 | 4-(4-CO₂H-C₆H₄)-2-OMe-C₆H₃ |
| 1-113 | 2-OMe-4-(4-CO₂Me-C₆H₄)-C₆H₃ |
| 1-114 | 2-Cl-4-(3,4-di-F-C₆H₃)-C₆H₃ |
| 1-115 | 2-Cl-4-(3,5-di-F-C₆H₃)-C₆H₃ |
| 1-116 | 2-Cl-4-(3-Cl-2-F-C₆H₃)-C₆H₃ |
| 1-117 | 2-Cl-4-(5-Cl-2-F-C₆H₃)-C₆H₃ |
| 1-118 | 2-Cl-4-(3-Cl-4-F-C₆H₃)-C₆H₃ |
| 1-119 | 4-(3,4-di-F-C₆H₃)-2-OMe-C₆H₃ |
| 1-120 | 4-(3,5-di-F-C₆H₃)-2-OMe-C₆H₃ |
| 1-121 | 4-(3-Cl-2-F-C₆H₃)-2-OMe-C₆H₃ |
| 1-122 | 4-(5-Cl-2-F-C₆H₃)-2-OMe-C₆H₃ |
| 1-123 | 4-(3-Cl-4-F-C₆H₃)-2-OMe-C₆H₃ |
| 1-124 | 2-Cl-3-(3,4-di-Cl-C₆H₃)-C₆H₃ |
| 1-125 | 2-Cl-3-(4-F-C₆H₄)-C₆H₃ |
| 1-126 | 6-Cl-3-(3,4-di-Cl-C₆H₃)-C₆H₃ |
| 1-127 | 6-Cl-3-(4-F-C₆H₄)-C₆H₃ |
| 1-128 | 2-Cl-4-(2-Py)-C₆H₃ |
| 1-129 | 2-Cl-4-(3-Py)-C₆H₃ |
| 1-130 | 2-Cl-4-(4-Py)-C₆H₃ |
| 1-131 | 3-(3,4-di-Cl-C₆H₃)-2-OMe-C₆H₃ |
| 1-132 | 3-(4-F-C₆H₄)-2-OMe-C₆H₃ |
| 1-133 | 3-(3,4-di-Cl-C₆H₃)-6-OMe-C₆H₃ |
| 1-134 | 3-(4-F-C₆H₄)-6-OMe-C₆H₃ |
| 1-135 | 2-OMe-4-(2-Py)-C₆H₃ |
| 1-136 | 2-OMe-4-(3-Py)-C₆H₃ |
| 1-137 | 2-OMe-4-(4-Py)-C₆H₃ |
| 1-138 | Ph(A) |
| 1-139 | Ph(B) |
| 1-140 | Ph(C) |
| 1-141 | Ph(D) |
| 1-142 | Ph(E) |
| 1-143 | Ph(F) |
| 1-144 | Ph(G) |
| 1-145 | Ph(H) |
| 1-146 | Ph(I) |
| 1-147 | Ph(J) |
| 1-148 | Ph(K) |
| 1-149 | Ph(L) |
| 1-150 | 2-OCH₂Ph-C₆H₄ |
| 1-151 | 2-OPh-C₆H₄ |
| 1-152 | 2-Cl-4-OCH₂Ph-C₆H₃ |
| 1-153 | 2-Cl-4-OPh-C₆H₃ |
| 1-154 | 2-OMe-4-OCH₂Ph-C₆H₃ |
| 1-155 | 2-OMe-4-OPh-C₆H₃ |
| 1-156 | 2-OCH₂(4-F-C₆H₄)-C₆H₄ |
| 1-157 | 2-O(4-F-C₆H₄)-C₆H₄ |
| 1-158 | 2-Cl-4-OCH₂(4-F-C₆H₄)-C₆H₃ |
| 1-159 | 2-Cl-4-O(4-F-C₆H4)-C₆H₃ |
| 1-160 | 4-OCH₂(4-F-C₆H₄)-2-OMe-C₆H₃ |
| 1-161 | 4-O(4-F-C₆H₄)-2-OMe-C₆H₃ |
| 1-162 | Het(D) |
| 1-163 | Het(E) |
| 1-164 | Het(F) |
| 1-165 | Het(G) |

**(Table 2)**

| | | | |
|---|---|---|---|
| 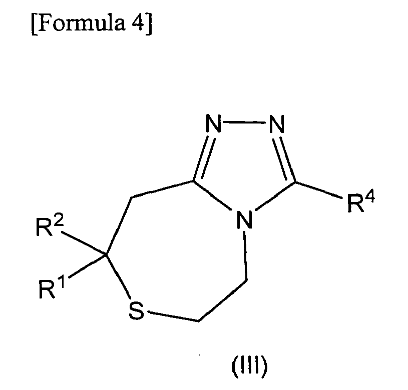 | | | |

| Compound No. | R¹ | R² | R⁴ |
|---|---|---|---|
| 2-1 | H | Me | 2-Cl-C₆H₄ |
| 2-2 | H | Me | 2-Me-C₆H₄ |
| 2-3 | H | Me | 2-OMe-C₆H₄ |
| 2-4 | H | Me | 2-Et-C₆H₄ |
| 2-5 | H | Me | 2-OEt-C₆H₄ |
| 2-6 | H | Me | 2-Cl-4-F-C₆H₃ |
| 2-7 | H | Me | 2-Cl-4-(2-Cl-C₆H₄)-C₆H₃ |
| 2-8 | H | Me | 2-Cl-4-(3-F-C₆H₄)-C₆H₃ |
| 2-9 | H | Me | 2-Cl-4-(3-Cl-C₆H₄)-C₆H₃ |
| 2-10 | H | Me | 4-(3-Cl-C₆H₄)-2-OMe-C₆H₃ |
| 2-11 | H | Me | 4-(4-F-C₆H₄)-2-OMe-C₆H₃ |
| 2-12 | H | Me | 2-Cl-4-(3-Cl-2-F-C₆H₃)-C₆H₃ |
| 2-13 | H | Me | 4-(3-Cl-4-F-C₆H₃)-2-OMe-C₆H₃ |
| 2-14 | H | Et | 2-Cl-C₆H₄ |
| 2-15 | H | Et | 2-Me-C₆H₄ |
| 2-16 | H | Et | 2-OMe-C₆H₄ |
| 2-17 | H | Et | 2-Et-C₆H₄ |
| 2-18 | H | Et | 2-OEt-C₆H₄ |
| 2-19 | H | Et | 2-Cl-4-F-C₆H₃ |
| 2-20 | H | Et | 2-Cl-4-(2-Cl-C₆H₄)-C₆H₃ |
| 2-21 | H | Et | 2-Cl-4-(3-F-C₆H₄)-C₆H₃ |
| 2-22 | H | Et | 2-Cl-4-(3-Cl-C₆H₄)-C₆H₃ |
| 2-23 | H | Et | 4-(3-Cl-C₆H₄)-2-OMe-C₆H₃ |
| 2-24 | H | Et | 4-(4-F-C₆H₄)-2-OMe-C₆H₃ |
| 2-25 | H | Et | 2-Cl-4-(3-Cl-2-F-C₆H₃)-C₆H₃ |
| 2-26 | H | Et | 4-(3-Cl-4-F-C₆H₃)-2-OMe-C₆H₃ |
| 2-27 | H | CH₂OH | 2-Cl-C₆H₄ |
| 2-28 | H | CH₂OH | 2-Me-C₆H₄ |
| 2-29 | H | CH₂OH | 2-OMe-C₆H₄ |
| 2-30 | H | CH₂OH | 2-Et-C₆H₄ |
| 2-31 | H | CH₂OH | 2-OEt-C₆H₄ |
| 2-32 | H | CH₂OH | 2-Cl-4-F-C₆H₃ |
| 2-33 | H | CH₂OH | 2-Cl-4-(2-Cl-C₆H₄)-C₆H₃ |
| 2-34 | H | CH₂OH | 2-Cl-4-(3-F-C₆H₄)-C₆H₃ |
| 2-35 | H | CH₂OH | 2-Cl-4-(3-Cl-C₆H₄)-C₆H₃ |
| 2-36 | H | CH₂OH | 4-(3-Cl-C₆H₄)-2-OMe-C₆H₃ |
| 2-37 | H | CH₂OH | 4-(4-F-C₆H₄)-2-OMe-C₆H₃ |
| 2-38 | H | CH₂OH | 2-Cl-4-(3-Cl-2-F-C₆H₃)-C₆H₃ |
| 2-39 | H | CH₂OH | 4-(3-Cl-4-F-C₆H₃)-2-OMe-C₆H₃ |
| 2-40 | Me | Me | 2-Cl-C₆H₄ |
| 2-41 | Me | Me | 2-Me-C₆H₄ |
| 2-42 | Me | Me | 2-OMe-C₆H₄ |
| 2-43 | Me | Me | 2-Et-C₆H₄ |
| 2-44 | Me | Me | 2-OEt-C₆H₄ |
| 2-45 | Me | Me | 2-Cl-4-F-C₆H₃ |
| 2-46 | Me | Me | 2-Cl-4-(2-Cl-C₆H₄)-C₆H₃ |
| 2-47 | Me | Me | 2-Cl-4-(3-F-C₆H₄)-C₆H₃ |
| 2-48 | Me | Me | 2-Cl-4-(3-Cl-C₆H₄)-C₆H₃ |
| 2-49 | Me | Me | 4-(3-Cl-C₆H₄)-2-OMe-C₆H₃ |
| 2-50 | Me | Me | 4-(4-F-C₆H₄)-2-OMe-C₆H₃ |
| 2-51 | Me | Me | 2-Cl-4-(3-Cl-2-F-C₆H₃)-C₆H₃ |
| 2-52 | Me | Me | 4-(3-Cl-4-F-C₆H₃)-2-OMe-C₆H₃ |
| 2-53 | Me | Et | 2-Cl-C₆H₄ |
| 2-54 | Me | Et | 2-Me-C₆H₄ |
| 2-55 | Me | Et | 2-OMe-C₆H₄ |
| 2-56 | Me | Et | 2-Et-C₆H₄ |
| 2-57 | Me | Et | 2-OEt-C₆H₄ |
| 2-58 | Me | Et | 2-Cl-4-F-C₆H₃ |
| 2-59 | Me | Et | 2-Cl-4-(2-Cl-C₆H₄)-C₆H₃ |
| 2-60 | Me | Et | 2-Cl-4-(3-F-C₆H₄)-C₆H₃ |
| 2-61 | Me | Et | 2-Cl-4-(3-Cl-C₆H₄)-C₆H₃ |
| 2-62 | Me | Et | 4-(3-Cl-C₆H₄)-2-OMe-C₆H₃ |
| 2-63 | Me | Et | 4-(4-F-C₆H₄)-2-OMe-C₆H₃ |
| 2-64 | Me | Et | 2-Cl-4-(3-Cl-2-F-C₆H₃)-C₆H₃ |
| 2-65 | Me | Et | 4-(3-Cl-4-F-C₆H₃)-2-OMe-C₆H₃ |
| 2-66 | Me | cPr | 2-Cl-C₆H₄ |
| 2-67 | Me | cPr | 2-Me-C₆H₄ |
| 2-68 | Me | cPr | 2-OMe-C₆H₄ |
| 2-69 | Me | cPr | 2-Et-C₆H₄ |
| 2-70 | Me | cPr | 2-OEt-C₆H₄ |
| 2-71 | Me | cPr | 2-Cl-4-F-C₆H₃ |
| 2-72 | Me | cPr | 2-Cl-4-(2-Cl-C₆H₄)-C₆H₃ |
| 2-73 | Me | cPr | 2-Cl-4-(3-F-C₆H₄)-C₆H₃ |
| 2-74 | Me | cPr | 2-Cl-4-(3-Cl-C₆H₄)-C₆H₃ |
| 2-75 | Me | cPr | 4-(3-Cl-C₆H₄)-2-OMe-C₆H₃ |
| 2-76 | Me | cPr | 4-(4-F-C₆H₄)-2-OMe-C₆H₃ |
| 2-77 | Me | cPr | 2-Cl-4-(3-Cl-2-F-C₆H₃)-C₆H₃ |
| 2-78 | Me | cPr | 4-(3-Cl-4-F-C₆H₃)-2-OMe-C₆H₃ |
| 2-79 | Me | CH₂F | 2-Cl-C₆H₄ |
| 2-80 | Me | CH₂F | 2-Me-C₆H₄ |
| 2-81 | Me | CH₂F | 2-OMe-C₆H₄ |
| 2-82 | Me | CH₂F | 2-Et-C₆H₄ |
| 2-83 | Me | CH₂F | 2-OEt-C₆H₄ |
| 2-84 | Me | CH₂F | 2-Cl-4-F-C₆H₃ |
| 2-85 | Me | CH₂F | 2-Cl-4-(2-Cl-C₆H₄)-C₆H₃ |
| 2-86 | Me | CH₂F | 2-Cl-4-(3-F-C₆H₄)-C₆H₃ |
| 2-87 | Me | CH₂F | 2-Cl-4-(3-Cl-C₆H₄)-C₆H₃ |
| 2-88 | Me | CH₂F | 4-(3-Cl-C₆H₄)-2-OMe-C₆H₃ |
| 2-89 | Me | CH₂F | 4-(4-F-C₆H₄)-2-OMe-C₆H₃ |
| 2-90 | Me | CH₂F | 2-Cl-4-(3-Cl-2-F-C₆H₃)-C₆H₃ |
| 2-91 | Me | CH₂F | 4-(3-Cl-4-F-C₆H₃)-2-OMe-C₆H₃ |
| 2-92 | Me | CH₂CH₂OH | 2-Cl-C₆H₄ |
| 2-93 | Me | CH₂CH₂OH | 2-Me-C₆H₄ |
| 2-94 | Me | CH₂CH₂OH | 2-OMe-C₆H₄ |
| 2-95 | Me | CH₂CH₂OH | 2-Et-C₆H₄ |
| 2-96 | Me | CH₂CH₂OH | 2-OEt-C₆H₄ |
| 2-97 | Me | CH₂CH₂OH | 2-Cl-4-F-C₆H₃ |
| 2-98 | Me | CH₂CH₂OH | 2-Cl-4-(2-Cl-C₆H₄)-C₆H₃ |
| 2-99 | Me | CH₂CH₂OH | 2-Cl-4-(3-F-C₆H₄)-C₆H₃ |
| 2-100 | Me | CH₂CH₂OH | 2-Cl-4-(3-Cl-C₆H₄)-C₆H₃ |
| 2-101 | Me | CH₂CH₂OH | 4-(3-Cl-C₆H₄)-2-OMe-C₆H₃ |
| 2-102 | Me | CH₂CH₂OH | 4-(4-F-C₆H₄)-2-OMe-C₆H₃ |
| 2-103 | Me | CH₂CH₂OH | 2-Cl-4-(3-Cl-2-F-C₆H₃)-C₆H₃ |
| 2-104 | Me | CH₂CH₂OH | 4-(3-Cl-4-F-C₆H₃)-2-OMe-C₆H₃ |

In Tables 1 and 2, preferred compounds include Compound Nos. 1-4, 1-7, 1-16, 1-22, 1-25, 1-30, 1-33, 1-35, 1-37, 1-38, 1-39, 1-40, 1-41, 1-62, 1-63, 1-69, 1-70, 1-77, 1-87, 1-98, 1-99, 1-103, 1-105, 1-119, 1-123, 1-125, 1-127, 1-129, 1-130, 1-138, 1-139, 1-140, 1-141, 1-156, 1-159, 1-163, 2-22, 2-33, 2-40, 2-41, 2-42, 2-50, 2-53, 2-58, 2-66, 2-77, 2-81, 2-89, 2-92 or 2-102.

More preferred compounds include Compound Nos. 1-7, 1-22, 1-25, 1-33, 1-35, 1-37, 1-38, 1-39, 1-40, 1-41, 1-62, 1-63, 1-69, 1-70, 1-77, 1-87, 1-98, 1-99, 1-103, 1-105, 1-119, 1-123, 1-125, 1-127, 1-129, 1-130, 1-138, 1-139, 1-140, 1-141, 1-156, 1-159, 1-163, 2-22, 2-50, 2-77, 2-89 or 2-102.

Yet more preferred compounds include
{8-methyl-3-[2-methoxy-4-(trifluoromethyl)phenyl]-5,6,8,9-tetrahydro[1,2,4]triazolo[4,3-d][1,4]thiazepin-8-yl}methanol (Compound No. 1-33),
[8-methyl-3-(1-methyl-1H-indol-4-yl)-5,6,8,9-tetrahydro[1,2,4]triazolo[4,3-d][1,4]thiazepin-8-yl]methanol (Compound No. 1-37),
{3-[1-(cyclopropylinethyl)-1H-indol-4-yl]-8-methyl-5,6,8,9-tetrahydro[1,2,4]triazolo[4,3-d][1,4]thiazepin-8-yl}methanol (Compound No. 1-38),
[3-(5-chloro-3-methyl-1-benzothiophen-2-yl)-8-methyl-5,6,8,9-tetrahydro[1,2,4]triazolo[4,3-d][1,4]thiazepin-8-yl]methanol (Compound No. 1-39),
[3-(3-chlorobiphenyl-4-yl)-8-methyl-5,6,8,9-tetrahydro[1,2,4]triazolo[4,3-d][1,4]thiazepin-8-yl]methanol (Compound No. 1-40),
[3-(3-methoxybiphenyl-4-yl)-8-methyl-5,6,8,9-tetrahydro[1,2,4]triazolo[4,3-d][1,4]thiazepin-8-yl]methanol (Compound No. 1-41),
[3-(3-chloro-3'-fluorobiphenyl-4-yl)-8-methyl-5,6,8,9-tetrahydro[1,2,4]triazolo[4,3-d][1,4]thiazepin-8-yl]methanol (Compound No. 1-62),
[3-(3,3'-dichlorobiphenyl-4-yl)-8-methyl-5,6,8,9-tetrahydro[1,2,4]triazolo[4,3-d][1,4]thiazepin-8-yl]methanol (Compound No. 1-63),
[3-(3-chloro-4'-fluorobiphenyl-4-yl)-8-methyl-5,6,8,9-tetrahydro[1,2,4]triazolo[4,3-d][1,4]thiazepin-8-yl]methanol (Compound No. 1-69),
[3-(3,4'-dichlorobiphenyl-4-yl)-8-methyl-5,6,8,9-tetrahydro[1,2,4]triazolo[4,3-d][1,4]thiazepin-8-yl]methanol (Compound No. 1-70),
[3-(3-chloro-4'-methoxycarbonylbiphenyl-4-yl)-8-methyl-5,6,8,9-tetrahydro[1,2,4]triazolo[4,3-d][1,4]thiazepin-8-yl]methanol (Compound No. 1-77),
{3-[4'-fluoro-3-(trifluoromethyl)biphenyl-4-yl)-8-methyl-5,6,8,9-tetrahydro[1,2,4]triazolo[4,3-d][1,4]thiazepin-8-yl]methanol (Compound No. 1-87),
[3-(3'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-98),
[3-(3'-chloro-3-methoxybiphenyl-4-yl)-8-methyl-5,6,8,9-tetrahydro[1,2,4]triazolo[4,3-d][1,4]thiazepin-8-yl]methanol (Compound No. 1-99),
4'-[8-(hydroxymethyl)-8-methyl-5,6,8,9-tetrahydro[1,2,4]triazolo[4,3-d][1,4]thiazepin-3-yl]-3'-methoxybiphenyl-3-carbonitrile(Compound No. 1-103),
[3-(4'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-105),
[3-(3',4'-difluoro-3-methoxybiphenyl-4-yl)-8-methyl-5,6,8,9-tetrahydro[1,2,4]triazolo[4,3-d][1,4]thiazepin-8-yl]methanol (Compound No. 1-119),
[3-(3'-chloro-4'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-123),
{3-[2-chloro-4-(pyridin-3-yl)phenyl]-8-methyl-5,6,8,9-tetrahydro[1,2,4]triazolo [4,3-d][1,4]thiazepin-8-yl}methanol (Compound No. 1-129),
{3-[2-chloro-4-(pyridin-4-yl)phenyl]-8-methyl-5,6,8,9-tetrahydro[1,2,4]triazolo[4,3-d][1,4]thiazepin-8-yl}methanol (Compound No. 1-130),
{3-[2-chloro-4-(morpholin-4-yl)phenyl]-8-methyl-5,6,8,9-tetrahydro[1,2,4]triazolo[4,3-d][1,4]thiazepin-8-yl)methanol (Compound No. 1-138),
{3-[2-chloro-4-(piperidin-1-yl)phenyl]-8-methyl-5,6,8,9-tetrahydro[1,2,4]triazolo[4,3-d][1,4]thiazepin-8-yl}methanol (Compound No. 1-139),
{3-[2-chloro-4-(pyrrolidin-1-yl)phenyl]-8-methyl-5,6,8,9-tetrahydro[1,2,4]triazolo[4,3-d][1,4]thiazepin-8-yl}methanol (Compound No. 1-140), or
{3-[2-chloro-4-(1H-pyrrol-1-yl)phenyl]-8-methyl-5,6,8,9-tetrahydro[1,2,4]triazolo[4,3-d][1,4]thiazepin-8-yl}methanol (Compound No. 1-141).

Particularly preferred compounds include
[3-(3-chlorobiphenyl-4-yl)-8-methyl-5,6,8,9-tetrahydro[1,2,4]triazolo[4,3-d][1,4]thiazepin-8-yl]methanol (Compound No. 1-40),
[3-(3-methoxybiphenyl-4-yl)-8-methyl-5,6,8,9-tetrahydro[1,2,4]triazolo[4,3-d][1,4]thiazepin-8-yl]methanol (Compound No. 1-41),
[3-(3-chloro-3'-fluorobiphenyl-4-yl)-8-methyl-5,6,8,9-tetrahydro[1,2,4]triazolo[4,3-d][1,4]thiazepin-8-yl]methanol (Compound No. 1-62),
[3-(3,3'-dichlorobiphenyl-4-yl)-8-methyl-5,6,8,9-tetrahydro[1,2,4]triazolo[4,3-d][1,4]thiazepin-8-yl]methanol (Compound No. 1-63),
[3-(3-chloro-4'-fluorobiphenyl-4-yl)-8-methyl-5,6,8,9-tetrahydro[1,2,4]triazolo[4,3-d][1,4]thiazepin-8-yl]methanol (Compound No. 1-69),
[3-(3,4'-dichlorobiphenyl-4-yl)-8-methyl-5,6,8,9-tetrahydro[1,2,4]triazolo[4,3-d][1,4]thiazepin-8-yl]methanol (Compound No. 1-70),
[3-(3'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-98),
[3-(3'-chloro-3-methoxybiphenyl-4-yl)-8-methyl-5,6,8,9-tetrahydro[1,2,4]triazolo[4,3-d][1,4]thiazepin-8-yl]methanol (Compound No. 1-99),
4'-[8-(hydroxymethyl)-8-methyl-5,6,8,9-tetrahydro[1,2,4]triazolo[4,3-d][1,4]thiazepin-3-yl]-3'-methoxybiphenyl-3-carbonitrile(Compound No. 1-103),
[3-(4'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-105),
[3-(3',4'-difluoro-3-methoxybiphenyl-4-yl)-8-methyl-5,6,8,9-tetrahydro[1,2,4]triazolo[4,3-d][1,4]thiazepin-8-yl]methanol (Compound No. 1-119), or
[3-(3'-chloro-4'-fluoro-3-methoxybiphenyl-4-yl)-8-methyl-5,6,8,9-tetrahydro[1,2,4]triazolo[4,3-d][1,4]thiazepin-8-yl]methanol (Compound No. 1-123).

### Effect of the Invention

The novel thiazepine derivative or pharmacologically acceptable salt thereof represented by the general formula (I) of the present invention has excellent effects of inhibiting 11β-HSD1 and is useful as a medicament for prophylactic and/or therapeutic treatment of diseases in warm-blooded animals (preferably a mammal animal including a human) selected from the group consisting of the following diseases: diabetes, adiposity, hyperlipemia, hypertension, and metabolic syndrome including these symptoms in combination.

### Best Mode for Carrying Out the Invention

The compounds having the general formula (I) of the present invention can be produced by Methods A to D described below.

The solvents used for the reactions at each step of the following Methods A to D are not particularly limited so long as they do not inhibit the reaction and dissolve the starting materials to some extent and are, for example, selected from the following solvent groups. The solvent groups include hydrocarbons such as pentane, hexane, octane, petroleum ether, ligroin, and cyclohexane; amides such as formamide, N,N-dimethylformamide, N,N-dimethylacetamide, N-methyl-2-pyrrolidone, N-methyl-2-pyrrolidinone, and hexamethylphosphoric acid triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, 2-butanol, 2-methyl-1-propanol, t-butanol, isoamyl alcohol, diethylene glycol, glycerine, octanol, cyclohexanol, and methyl cellosolve; sulfoxides such as dimethyl sulfoxide; sulfones such as sulfolane; nitriles such as acetonitrile, propionitrile, butyronitrile, and isobutyronitrile; esters such as ethyl formate, ethyl acetate, propyl acetate, butyl acetate, and diethyl carbonate; ketones such as acetone, methyl ethyl ketone, 4-methyl-2-pentanone, methyl isobutyl ketone, isophorone, and cyclohexanone; nitro compounds such as nitroethane and nitrobenzene; halogenated hydrocarbons such as dichloromethane, 1,2-dichloroethane, chlorobenzene, dichlorobenzene, chloroform, and carbon tetrachloride; aromatic hydrocarbons such as benzene, toluene, and xylene; carboxylic acids such as acetic acid, formic acid, propionic acid, butyric acid, and trifluoroacetic acid; amines such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), 1,8-dieabicyclo[5.4.0]undec-7-ene (DBU), and piperidine; water; and mixed solvents thereof.

Examples of bases used for the reactions at each step of the following Methods A to D include inorganic bases including alkali metal carbonates such as sodium carbonate, potassium carbonate, lithium carbonate, and cesium carbonate; alkali metal hydrogen carbonates such as sodium hydrogencarbonate, potassium hydrogencarbonate, and lithium hydrogencarbonate; alkali metal hydrides such as lithium hydride, sodium hydride, and potassium hydride; alkali metal hydroxides such as sodium hydroxide, potassium hydroxide, barium hydroxide, and lithium hydroxide; alkali metal fluorides such as sodium fluoride and potassium fluoride; alkali metal alkoxides such as sodium methoxide, sodium ethoxide, sodium t-butoxide, potassium methoxide, potassium ethoxide, potassium t-butoxide, and lithium methoxide; alkali metal trialkylsilanolates such as sodium trimethylsilanolate, potassium trimethylsilanolate, and lithium trimethylsilanolate; mercaptan alkali metals such as methyl mercaptan sodium and ethylmercaptan sodium; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), and 1,8-diazabicyclo[5.4.0]undec-7-ene (DBU); and organic metal bases such as butyllithium, lithium diisopropyl amide, and lithium bis(trimethylsilyl)amide.

In the reactions at each step of the following Methods A to D, the reaction temperature varies depending on the solvents, starting materials, reagents, and the like, and the reaction time varies depending on the solvents, starting materials, reagents, reaction temperature, and the like.

In the reactions at each step of the following Methods A to D, after completion of the reaction, each target compound is collected from the reaction mixture according to usual methods. For example, the target compound is obtained by suitably neutralizing the reaction mixture or removing insoluble matter, if any exists, by filtration, then adding water and an immiscible organic solvent, such as ethyl acetate, to isolate an organic layer containing the target compound, washing the organic layer with water or the like, drying with anhydrous magnesium sulfate, anhydrous sodium sulfate, anhydrous sodium hydrogencarbonate, or the like, filtering, and then evaporating the solvent. The resulting target compound may be isolated and purified if necessary by appropriately combining usual methods, for example, methods suitably used for isolation and purification of organic compounds such as recrystallization and reprecipitation and eluting with an appropriate eluent by application of chromatography. A target compound that is insoluble in the solvent can be purified by washing the crude product of the resulting solid with the solvent. The target compound at each step can be used as it is in the subsequent reaction without being purified.

Hereafter, the reactions at each step of Methods A to D will be explained.

Method A is a method for producing a compound having the general formula (I).

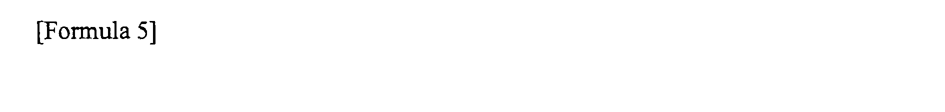

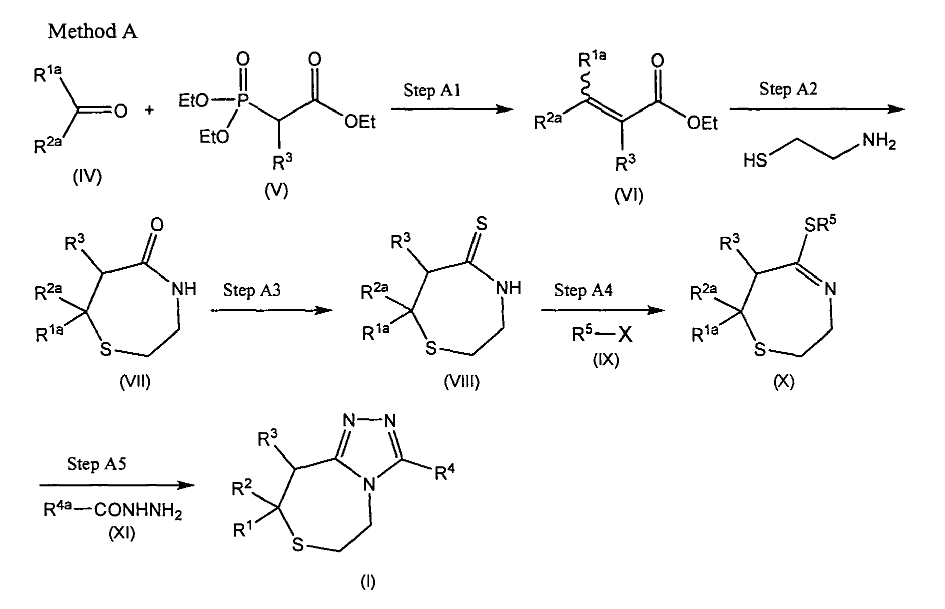

In the present invention, R¹, R², R³, and R⁴ have the same meanings as defined above, R^{1a} represents the same groups as those defined for R¹, except that a hydroxy group included in the R¹ group as a substituent is a hydroxy group that may be protected, R^{2a} represents the same groups as those defined for R², except that a hydroxy group included in the R² group as a substituent is a hydroxy group that may be protected, R^{4a} represents the same groups as those defined for R⁴, except that an amino group, a hydroxy group, and/or a carboxyl group included in the R⁴ group as a substituent are an amino group, a hydroxy group, and/or a carboxyl group that may be protected, R⁵ represents a C₁-C₆ alkyl group (preferably a methyl group), X represents a halogen atom (preferably a chlorine atom, a bromine atom, or an iodine atom, more preferably a bromine atom or an iodine atom).

### Step A1

This step is a step for producing a compound having the general formula (VI).

This step is carried out by reacting a compound having the general formula (IV) which is a known compound or is easily obtained from a known compound as a starting material by a method similar to a known method with a compound having the general formula (V) which is a known compound or is easily obtained from a known compound as a starting material by a method similar to a known method, in a solvent in the presence of a base.

The solvent used in this step is preferably an ether or an amide, more preferably tetrahydrofuran.

The base used in this step is preferably an organic metal base, more preferably n-butyllithium.

The reaction temperature in this step is usually -100°C to 100°C, preferably - 78°C to 0°C.

The reaction time in this step is usually 0.5 h to 100 h, preferably 1 h to 3 h.

### Step A2

This step is a step for producing a compound having the general formula (VII). This step is carried out by reacting the compound having the general formula (VI) with 2-aminoethanethiol in a solvent.

The solvent used in this step is preferably an alcohol or an amine, more preferably piperidine.

The reaction temperature in this step is usually 30°C to 200°C, preferably 100°C to 150°C.

The reaction time in this step is usually 1 h to 100 h, preferably 3 h to 24 h.

### Step A3

This step is a step for producing a compound having the general formula (VIII).

This step is carried out by reacting the compound having the general formula (VII) with a thiating agent in a solvent.

The solvent used in this step is preferably a hydrocarbon, more preferably toluene.

The thiating agent used in this step is, for example, Lawesson's reagent or diphosphorus pentasulfide, preferably Lawesson's reagent.

The reaction temperature in this step is usually 30°C to 200°C, preferably 80°C to 120°C.

The reaction time in this step is usually 0.5 h to 24 h, preferably 1 h to 3 h.

### Step A4

This step is a step for producing a compound having the general formula (X).

This step is carried out by reacting the compound having the general formula (VIII) with a compound having the general formula (IX) which is a known compound or is easily obtained from a known compound as a starting material by a method similar to a known method, in a solvent in the presence of a base.

The solvent used in this step is preferably an ether, more preferably a mixed solvent of tetrahydrofuran and water.

The base used in this step is preferably an alkali metal hydroxide, more preferably potassium hydroxide.

The reaction temperature in this step is usually -20°C to 100°C, preferably 0°C to 30°C.

The reaction time in this step is usually 0.5 h to for 24 h, preferably 1 h to 12 h.

### Step A5

This step is a step for producing a compound having the general formula (I).

This step is carried out by reacting the compound having the general formula (X) with a compound having the general formula (XI) in a solvent and subsequent removal of a protecting group of an amino group, a hydroxy group, and/or a carboxyl group in R^{1a}, R^{2a}, and/or R^{4a} as desired.

The solvent used in this step is preferably an alcohol, more preferably n-butanol.

The reaction temperature in this step is usually 80°C to 200°C, preferably 100°C to 140°C.

The reaction time in this step is usually 0.5 h to 24 h, preferably 1 h to 4 h.

Method B is a method for producing a compound having the general formula (XI) used in the above-mentioned Method A Step A5.

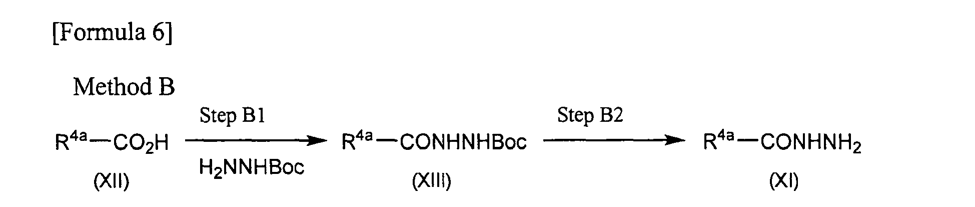

In the present invention, R^{4a} has the same meaning as defined above.

### Step B1

This step is a step for producing a compound having the general formula (XIII) and consists of steps (i) and (ii).
(i) This step is carried out by reacting a compound having the general formula (XII) which is a known compound or is easily obtained from a known compound as a starting material by a method similar to a known method with a chlorinating agent in the presence or absence of (preferably in the presence of) N,N-dimethylformamide in a solvent.

The solvent used in this step is preferably a halogenated hydrocarbon, more preferably methylene chloride.

Examples of the chlorinating agent used in this step include inorganic acids such as hydrochloric acid; halogen molecules such as chlorine; phosphorus reagents such as phosphorus trichloride, phosphorus pentachloride, and phosphorus oxychloride; chlorides such as oxalyl chloride; sulfinic acid reagents such as thionyl chloride and toluenesulfonic acid chloride, and sulfonic acid reagents such as sulfonyl chloride. Chlorides are preferred, and oxalyl chloride is more preferred.

The reaction temperature in this step is usually 0°C to 80°C, preferably 15°C to 30°C.

The reaction time in this step is usually 0.5 h to 24 h, preferably 1 h to 3 h.
(ii) This step is carried out by reacting the compound obtained in the above (i) with t-butyl carbazate in the presence or absence of (preferably in the presence of) a base in a solvent.

The solvent used in this step is preferably a halogenated hydrocarbon, more preferably methylene chloride.

The base used in this step is preferably an organic base, more preferably triethylamine.

The reaction temperature in this step is usually 0°C to 40°C, preferably 10°C to 25°C.

The reaction time in this step is usually 0.5 h to 12 h, preferably 1 h to 2 h.

### Step B2

This step is a step for producing a compound having the general formula (XI).

This step is carried out by reacting the compound having the general formula (XIII) with an acid in a solvent.

The solvent used in this step is preferably an ester, more preferably ethyl acetate.

Examples of the acid used in this step include hydrogen halides such as hydrogen chloride gas and hydrogen bromide gas; mineral acids such as sulfuric acid, methyl sulfate, hydrobromic acid, and hydrochloric acid; organic sulfonic acids such as methanesulfonic acid, p-toluenesulfonic acid, camphor sulfonic acid, and trifluoromethanesulfonic acid; carboxylic acids such as acetic acid, formic acid, and trifluoroacetic acid; Lewis acids such as aluminium chloride, zinc chloride, tin tetrachloride, titanium trichloride, boron trifluoride, and boron tribromide; and acidic ion exchange resins. Hydrogen halides are preferred, and hydrogen chloride is more preferred.

The reaction temperature in this step is usually 0°C to 100°C, preferably 10°C to 30°C.

The reaction time in this step is usually 0.5 h to 12 h, preferably 1 h to 2 h.

Method C is a method for producing a compound having the general formula (XI) used in the above-mentioned Method A Step A5.

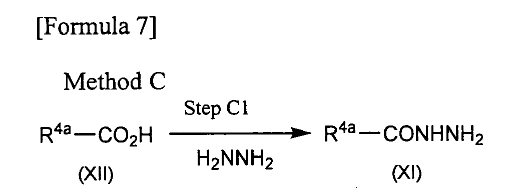

In the present invention, R^{4a} has the same meaning as defined above.

### Step C1

This step is carried out by reacting a compound having the general formula (XII) which is a known compound or is easily obtained from a known compound as a starting material by a method similar to a known method with hydrazine in the presence of a condensing agent and a base in a solvent.

The solvent used in this step is preferably an amide, more preferably N,N-dimethylformamide.

Examples of the condensing agent used in this step include 0-(7-azabenzotriazol-1-yl)-N,N,N',N'-tetramethyluronium hexafluorophosphate (HATU), 1-propanephosphonic acid cyclic anhydride (T3P), dicyclohexylcarbodiimide (DCCD), 1-ethyl-3-(3-dimethylaminopropyl)carbodiimide hydrochloride (EDCI), 1-ethyl-3-(3-dimethylaminopropyl)carbodiimide (EDAC), isobutyl chloroformate (IBCF), 1,1'-carbonyl bis-1H-imidazole (CDI), diethyl phosphoryl cyanide (DEPC), diphenyl phosphoryl azide (DPPA), N-hydroxysuccinimide, N-hydroxy-5-norbornene-2,3-dicarboxyimide and dipyridyl disulfide. If necessary, 1-hydroxybenzotriazole or 1-hydroxybenzotriazole hydrate (HOBt) can also be present. 1-Ethyl-3-(3-dimethylaminopropyl)carbodiimide hydrochloride (EDCI) is preferred.

The base used in this step is preferably an organic base, more preferably triethylamine.

The reaction temperature in this step is usually -20°C to 100°C, preferably 0°C to 30°C.

The reaction time in this step is usually 0.5 h to 24 h, preferably 1 h to 2 h.

Method D is a method for producing a compound having the general formula (XI) used in the above-mentioned Method A Step A5.

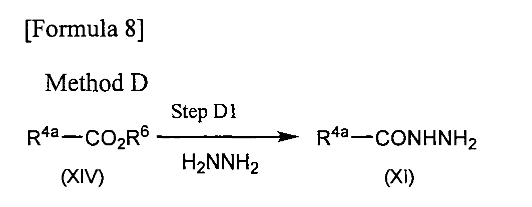

In the present invention, R^{4a} has the same meaning as defined above, and R⁶ represents a C₁-C₆ alkyl group (preferably a methyl group).

### Step D1

This step is carried out by reacting a compound having the general formula (XIV) which is a known compound or is easily obtained from a known compound as starting material by a method similar to a known method with hydrazine in a solvent.

The solvent used in this step is preferably an alcohol, more preferably ethanol.

The reaction temperature in this step is usually 50°C to 150°C, preferably 80°C to 100°C.

The reaction time in this step is usually 1 h to 24 h, preferably 2 h to 4 h.

The starting compounds having the general formulas (IV), (V), (XII) and (XIV) are known compounds or are easily produced using known compounds as starting materials by known methods or similar methods.

In the above description, protecting groups of the "amino group that may be protected," the "hydroxy group that may be protected," and the "carboxyl group that may be protected" in the definitions of R^{1a}, R^{2a} , and R^{4a} are protecting groups that can be cleaved by chemical methods such as hydrogenolysis, hydrolysis, electrolysis, and photodegradation, and represent protecting groups commonly used in synthetic organic chemistry (for example, refer to T. W. Greene et al., Protective Groups in Organic Synthesis, 3rd Edition, John Wiley & Sons, Inc. [1999]).

In the above description, "protecting groups" of the "hydroxy group that may be protected" in the definitions of R^{1a}, R^{2a}, and R^{4a} are not particularly limited so long as they are protecting groups of a hydroxy group used in the field of synthetic organic chemistry, and examples thereof include "general protecting groups for an ester of a hydroxy group." Preferred examples include "alkylcarbonyl groups" including a formyl group, the above-mentioned "C₂-C₇ alkylcarbonyl group", halogenated alkylcarbonyl groups such as a chloroacetyl group, a dichloroacetyl group, a trichloroacetyl group, and a trifluoroacetyl group, alkoxyalkylcarbonyl groups such as a methoxyacetyl group, unsaturated alkylcarbonyl groups such as an acryloyl group, a propioloyl group, a methacryloyl group, a crotonoyl group, an isocrotonoyl group, and an (E)-2-methyl-2-butenoyl group; "arylcarbonyl groups" including arylcarbonyl groups such as a benzoyl group, an α-naphthoyl group and a β-naphthoyl group, halogenated arylcarbonyl groups such as a 2-bromobenzoyl group and a 4-chlorobenzoyl group, C₁-C₆ alkylated arylcarbonyl groups such as a 2,4,6-trimethylbenzoyl group and a 4-toluoyl group, C₁-C₆ alkoxylated arylcarbonyl groups such as a 4-anisoyl group, nitrated arylcarbonyl groups such as a 4-nitrobenzoyl group and a 2-nitrobenzoyl group, C₂-C₇ alkoxycarbonylated arylcarbonyl groups such as a 2-(methoxycarbonyl)benzoyl group, and arylated arylcarbonyl groups such as a 4-phenylbenzoyl group; "alkoxycarbonyl groups" including the above-mentioned "C₂-C₇ alkoxycarbonyl group", and C₂-C₇ alkoxycarbonyl groups substituted with a halogen or a tri-(C₁-C₆ alkyl)silyl group such as a 2,2,2-trichloroethoxycarbonyl group and a 2-trimethylsilylethoxycarbonyl group; "tetrahydropyranyl or tetrahydrothiopyranyl groups" such as a tetrahydropyran-2-yl group, a 3-bromotetrahydropyran-2-yl group, a 4-methoxytetrahydropyran-4-yl group, a tetrahydrothiopyran-2-yl group, and a 4-methoxytetrahydrothiopyran-4-yl group; "tetrahydrofuranyl or tetrahydrothiofuranyl groups" such as a tetrahydrofuran-2-yl group and a tetrahydrothiofuran-2-yl group; "silyl groups" including tri-(C₁-C₆ alkyl)silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group, and (C₁-C₆ alkyl)diarylsilyl groups or di-(C₁-C₆ alkyl)arylsilyl groups such as a diphenylmethylsilyl group, a diphenylbutylsilyl group, a diphenylisopropylsilyl group, and a phenyldiisopropylsilyl group; "alkoxymethyl groups" including (C₁-C₆ alkoxy)methyl groups such as a methoxymethyl group, a 1,1-dimethyl-1-methoxymethyl group, an ethoxymethyl group, a propoxymethyl group, an isopropoxymethyl group, a butoxymethyl group, and a t-butoxymethyl group, (C₁-C₆ alkoxy)-(C₁-C₆ alkoxy)methyl groups such as a 2-methoxyethoxymethyl group, (C₁-C₆ halogenated alkoxy)methyl groups such as a 2,2,2-trichloroethoxymethyl group and a bis(2-chloroethoxy)methyl group; "substituted ethyl groups" including (C₁-C₆ alkoxy)ethyl groups such as a 1-ethoxyethyl group and a 1-(isopropoxy)ethyl group and halogenated ethyl groups such as a 2,2,2-trichloroethyl group; "aralkyl groups" including C₁-C₆ alkyl groups substituted with 1 to 3 aryl group(s) such as a benzyl group, an α-naphthylmethyl group, a β-naphthylmethyl group, a diphenylmethyl group, a triphenylmethyl group, an α-naphthyldiphenylmethyl group, and a 9-anthrylmethyl group and C₁-C₆ alkyl groups substituted with 1 to 3 aryl group(s) having an aryl ring substituted with C₁-C₆ alkyl group(s), C₁-C₆ alkoxy group(s), nitro group(s), halogen atom(s), or cyano group(s) such as a 4-methylbenzyl group, a 2,4,6-trimethylbenzyl group, a 3,4,5-trimethylbenzyl group, a 4-methoxybenzyl group, a 4-methoxyphenyldiphenylmethyl group, a 2-nitrobenzyl group, a 4-nitrobenzyl group, a 4-chlorobenzyl group, a 4-bromobenzyl group, or a 4-cyanobenzyl group; "alkenyloxycarbonyl groups" such as a vinyloxycarbonyl group and an allyloxycarbonyl group; and "aralkyloxycarbonyl groups" in which the aryl ring may be substituted with 1 or 2 C₁-C₆ alkoxy groups or nitro groups such as a benzyloxycarbonyl group, a 4-methoxybenzyloxycarbonyl group, a 3,4-dimethoxybenzyloxycarbonyl group, a 2-nitrobenzyloxycarbonyl group, and a 4-nitrobenzyloxycarbonyl group. Alkylcarbonyl groups, silyl groups, and aralkyl groups are more preferred.

In the above description, "protecting groups" of the "carboxyl group that may be protected" in the definition of R^{4a} are not particularly limited so long as they are protecting groups of a carboxyl group used in the field of synthetic organic chemistry, and examples thereof include "general protecting groups for an ester of a carboxyl group." Preferred examples thereof include the above-mentioned "C₁-C₆ alkyl groups"; "C₂-C₆ alkenyl groups" such as an ethenyl group, a 1-propenyl group, and a 2-propenyl group; "C₂-C₆ alkynyl groups" such as an ethynyl group, a 1-propynyl group, and a 2-propynyl group; the above-mentioned "C₁-C₆ halogenated alkyl groups"; the above-mentioned "C₁-C₆ hydroxyalkyl groups"; (C₂-C₇ alkylcarbonyl)-(C₁-C₆ alkyl groups) such as an acetylmethyl group; the above-mentioned "aralkyl groups"; and the above-mentioned "silyl groups". C₁-C₆ alkyl groups and aralkyl groups are more preferred.

In the above description, "protecting groups" of the "amino group that may be protected" in the definition of R^{4a} are not particularly limited so long as they are protecting groups of an amino group used in the field of synthetic organic chemistry, and examples thereof include groups similar to "alkylcarbonyl groups"; "arylcarbonyl groups"; "alkoxycarbonyl groups"; "silyl groups"; "aralkyl groups"; "alkenyloxycarbonyl groups"; and "aralkyloxycarbonyl groups" in the above-mentioned "general protecting groups for ester of a hydroxy group" and "substituted methylene groups forming a Schiffsbase" such as N,N-dimethylaminomethylene, benzylidene, 4-methoxybenzylidene, 4-nitrobenzylidene, salicylidene, 5-chlorosalicylidene, diphenylmethylene, and (5-chloro-2-hydroxyphenyl)phenyl methylene. Alkylcarbonyl groups, arylcarbonyl groups, and alkoxycarbonyl groups are preferred, and alkoxycarbonyl groups are more preferred.

The steps requiring protection/deprotection are performed according to known methods (for example, the methods described in Theodora W. Greene, Peter G. M. Wuts, "Protective Groups in Organic Synthesis," 1999, A Wiley-Interscience Publication, etc.).

The thiazepine derivative or pharmacologically acceptable salt thereof of the present invention can be administered in various forms. Examples of the route of administration include oral administration using tablets, capsules, granules, emulsions, pills, powders, syrups (solutions), and the like and parenteral administration using injections (intravenous, intramuscular, subcutaneous, or intraperitoneal administration), drip infusions, suppositories (rectal administration), and the like. These various formulations can be prepared as drug products according to usual methods using aids usually used in the field of drug formulation such as excipients, binders, disintegrating agents, lubricants, flavoring agents, dissolving aids, suspensions, and coating agents in addition to an active ingredient.

In the use as a tablet, examples of carriers that can be used include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrating agents such as dry starch, sodium alginate, agar powder, laminaran powder, sodium hydrogencarbonate, calcium carbonate, polyoxyethylene sorbitan fatty acid esters, sodium lauryl sulfate, stearic monoglyceride, starch, and lactose; disintegration inhibitors such as sucrose, stearin, cocoa butter, and hydrogenated oil; absorption enhancers such as quaternary ammonium salts and sodium lauryl sulfate; humectants such as glycerine and starch; adsorbents such as starch, lactose, kaolin, bentonite, and colloidal silicic acid; lubricants such as purified talc, stearate, fluoboric acid powder, and polyethylene glycol, and so forth. Furthermore, tablets coated in usual ways such as, for example, sugar-coated tablets, gelatin-coated tablets, enteric-coated tablets, film-coated tablets, double-layer tablets, and multilayered tablets can be prepared as required.

In the use as a pill, examples of carriers that can be used include excipients such as glucose, lactose, cocoa butter, starch, hydrogenated vegetable oil, kaolin, and talc; binders such as powdered gum arabic, powdered tragacanth, gelatin, and ethanol; disintegrating agents such as laminaran agar, and so forth.

In the use as a suppository, a wide range of carriers known in this field can be used, and examples thereof include polyethylene glycol, cocoa butter, higher alcohols, higher alcohol esters, gelatin, semisynthetic glycerides, and so forth.

In the use as an injection, the formulations can be prepared as solutions, emulsions, or suspensions. Preferably, these solutions, emulsions, and suspensions are sterilized and are isotonic with blood. Solutions for producing these solutions, emulsions, and suspensions are not particularly limited so long as they can be used as diluents for medical use, and examples thereof include water, ethanol, propylene glycol, ethoxylated isostearyl alcohol, polyoxylated isostearyl alcohol, polyoxy ethylene sorbitan fatty acid esters, and so forth. In this case, a sufficient amount of sodium chloride, glucose, or glycerine may be added to the formulation to prepare an isotonic solution, and usual dissolving aids, buffers, soothing agents, and the like may also be added.

Furthermore, coloring agents, preservatives, perfumes, flavoring agents, sweeteners, and the like can be added to the above-mentioned formulation, if necessary. Furthermore, other drugs can also be added.

The amount of active ingredient compound contained in the above-mentioned formulations is not particularly limited, but is usually 0.5 to 70% by weight of the total composition, preferably 1 to 30% by weight.

The dose varies depending on the symptoms, age, and the like of the patient (a warm-blooded animal, in particular, a human). In the case of oral administration, the recommended adult daily dosage is from 0.1 mg as the lower limit (preferably 1 mg, more preferably 10 mg) to 2000 mg as the upper limit (preferably 100 mg), which is divided into 1 to 6 times depending on the symptoms.

### Examples

Hereafter, the present invention will be more specifically explained with reference to the examples and the test examples. However, the scope of the present invention is not limited to these examples.

Elution for column chromatography in the examples was performed under observation by thin layer chromatography (TLC). In TLC observation, Silica Gel 60F₂₅₄ manufactured by Merck & Co., Inc. was used as the TLC plate, the solvent used as the elution solvent in column chromatography was used as the developing solvent, and a UV detector was used for detection. Silica Gel SK-85 (230 to 400 mesh) manufactured by Merck & Co., Inc. or Chromatorex NH (200 to 350 mesh) manufactured by Fuji Silysia Chemical Ltd. was used as silica gel for the column. In addition to a conventional column chromatography device, an automated chromatography device (SP-1) manufactured by Biotage AB was used as required. Abbreviations used in the examples have the following meanings mg: milligram, g: gram, mL: milliliter, MHz: megahertz.

In the following examples, nuclear magnetic resonance (hereinafter, ¹H NMR) spectra were expressed by using δ values (ppm) as chemical shift values and tetramethylsilane as a reference substance. Fragmentation patterns were represented by using s for a single line, d for a double line, t for a triple line, and q for quadruple line.

Mass spectrometry (MS) was performed by the fast atom bombardment (FAB) method, the electron ionization (EI) method, or the electron spray ionization (ESI) method.

### (Example 1) [3-(3-Chloro-4'-fluorobiphenyl-4-yl)-8-methyl-5,6,8,9-tetrahydro[1,2,4]triazolo[4,3-d][1,4]thiazepin-8-yl]methanol (Example Compound No. 1-69)

### (1a) Ethyl (2EZ)-4-{[t-butyl(dimethyl)silyl]oxy}-3-methyl-2-butenoate

An n-butyllithium/hexane solution (1.52 M, 12 mL, 18 mmol) was added dropwise to a solution of ethyl diethylphosphonoacetate (4.03 g, 18 mmol) in tetrahydrofuran (60 mL) at -70°C under a nitrogen atmosphere, the mixture was stirred for 30 min, followed by the addition of a solution of 1-{[t-butyl(dimethyl)silyl]oxy}-2-propanone (3.22 g, 17 mmol) in tetrahydrofuran (20 mL), and stirred for 2 h with heating to room temperature. Dilute hydrochloric acid was added to the reaction mixture, and the mixture was extracted with ethyl acetate, then washed with water, saturated aqueous sodium hydrogencarbonate, and saturated brine, and dried with anhydrous sodium sulfate. The filtrate was concentrated under reduced pressure, and then the residue was purified by silica gel column chromatography (elution solvent: hexane/ethyl acetate = 5/1) to obtain the title compound as a colorless oily substance (4.02 g, 91 %).
¹H NMR spectrum (400 MHz, CDCl₃) δppm: 0.08 and 0.09 (6H in total, s each), 0.91 and 0.93 (9H in total, s each), 1.27 (3H, t, J=7.5 Hz), 1.97 and 2.05 (3H in total, s each), 4.11 and 4.79 (2H in total, s each), 4.12-4.19(2H, m), 5.67 and 5.97 (1H in total, each s).

### (1b) 7-({[t-Butyl(dimethyl)silyl]oxy}methyl)-7-methyl-1,4-thiazepan-5-one

To ethyl (2EZ)-4-{[t-butyl(dimethyl)silyl]oxy}-3-methyl-2-butenoate (1.18 g, 4.6 mmol) obtained in Example (1a) was added a solution of 2-anfinoethanethiol (690 mg, 9.0 mmol) in piperidine (10 mL), and the mixture was stirred at 120°C for 4 h. Dilute hydrochloric acid was added to the reaction mixture, and the mixture was extracted with ethyl acetate, washed with water, saturated aqueous sodium hydrogencarbonate, and saturated brine, and dried with anhydrous sodium sulfate. The filtrate was concentrated under reduced pressure, and then the residue was purified by silica gel column chromatography (elution solvent: ethyl acetate) to obtain the title compound as a white solid (370 mg, 34%).
¹H NMR spectrum (400 MHz, CDCl₃) δppm: 0.07 (3H, s), 0.08 (3H, s), 0.92 (9H, s), 1.36 (3H, s), 2.76-2.87 (3H, m), 3.03 (1H, d, J=13.6 Hz), 3.48-3.67 (4H, m), 5.97 (1H, br, NH).

### (1c) 7-({[t-Butyl(dimethyl)silyl]oxy}methyl)-7-methyl-1,4-thiazepane-5-thione

A solution of 7-({[t-butyl(dimethyl)silyl]oxy}methyl)-7-methyl-1,4-thiazepan-5-one (368 mg, 1.4 mmol) obtained in Example (1b) and Lawesson's reagent (290 mg, 0.70 mmol) in toluene (10 mL) were stirred at 120°C for 1 h. The mixture was concentrated under reduced pressure, and then the residue purified by silica gel column chromatography (elution solvent: hexane/ethyl acetate = 2/1) to obtain the title compound as a white solid (420 mg, 100%).
¹H NMR spectrum (400 MHz, CDCl₃) δppm: 0.08 (3H, s), 0.08 (3H, s), 0.92 (9H, s), 1.41 (3H, s), 2.81-2.88 (2H, m), 3.35 (1H, d, J=13.7 Hz), 3.49 (1H, d, J=13.7 Hz), 3.60 (1H, d, J=16.5 Hz), 3.62 (1H, d, J=16.5 Hz), 3.70-3.75 (2H, m), 8.15 (1H, br, NH)

### (1d) 7-({[t-Butyl(dimethyl)silyl]oxy}methyl)-7-methyl-5-methylthio-2,3,6,7-tetrahydro-1,4-thiazepine

To a solution of 7-({[t-butyl(dimethyl)silyl]oxy}methyl)-7-methyl-1,4-thiazepane-5-thione (124 mg, 0.40 mmol) obtained in Example (1c) in tetrahydrofuran (3 mL) and water (2 mL) were added potassium hydroxide (110 mg, 2.0 mmol) and methyl iodide (340 mg, 2.4 mmol), and the mixture was stirred at room temperature for 1.5 h. The reaction mixture was extracted with ethyl acetate, washed with water and saturated brine, and dried with anhydrous sodium sulfate. The filtrate was concentrated under reduced pressure, and then the title compound was obtained as a colorless oily substance (106 mg, 82%).
¹H NMR spectrum (400 MHz, CDCl₃) δppm: 0.06 (3H, s), 0.07 (3H, s), 0.92 (9H, s), 1.30 (3H, s), 2.26 (3H, s), 2.68-2.76 (2H, m), 2.87 (1H, d, J=14.7 Hz), 3.06 (1H, d, J=14.7 Hz), 3.46 (1H, d, J=10.0 Hz), 3.59 (1H, d, J=10.0 Hz), 3.96-4.02 (1H, m), 4.10-4.16 (1H, m).

### (1e) t-Butyl 2-(4-bromo-2-chlorobenzoyl)hydrazinecarboxylate

Oxalyl chloride (1.2 mL, 14 mmol) and N,N-dimethylformamide (catalytic amount) were added to a solution of 4-bromo-2-chlorobenzoic acid (2.07 g, 8.8 mmol) in methylene chloride (20 mL), and the mixture was stirred at room temperature for 2 h. The mixture was concentrated under reduced pressure, then dissolved in methylene chloride (10 mL), and added to a solution of t-butyl carbazate (1.58 g, 12 mmol) and triethylamine (3.0 mL, 22 mmol) in methylene chloride (20 mL) with ice cooling, and the mixture was stirred for 30 min. Dilute hydrochloric acid was added, and the mixture was extracted with ethyl acetate, washed with water, saturated aqueous sodium hydrogencarbonate, and saturated brine, and then dried with anhydrous sodium sulfate. The filtrate was concentrated under reduced pressure, then the residue was purified by silica gel column chromatography (elution solvent: hexane/ethyl acetate = 1/3), and the resulting solid was collected by filtration and washed with diisopropyl ether to obtain the title compound as a white solid (2.10 g, 68%).
¹H NMR spectrum (400 MHz, CDCl₃) δppm: 1.52 (9H, s), 6.83 (1H, br, NH), 7.50 (1H, dd, J=2.0, 8.2 Hz), 7.61-7.64 (2H, m), 7.90 (1H, br, NH).

### (1f) t-Butyl 2-[(3-chloro-4'-fluorobiphenyl-4-yl)carbonyl]hydrazinecarboxylate

To a solution of t-butyl 2-(4-bromo-2-chlorobenzoyl)hydrazinecarboxylate (490 mg, 1.4 mmol) obtained in Example (1e) in 1,2-dimethoxyethane (10 mL) and water (2.5 mL) were added (4-fluorophenyl)boronic acid (350 mg, 2.5 mmol), potassium carbonate (390 mg, 2.8 mmol), and tetrakis(triphenylphosphine)palladium(0) (277 mg, 0.24 mmol), and the mixture was stirred at 100°C for 3 h. The mixture was extracted with ethyl acetate, then washed with water, saturated aqueous sodium hydrogencarbonate, and saturated brine, and then dried with anhydrous sodium sulfate. The filtrate was concentrated under reduced pressure, then the residue was purified by silica gel column chromatography (elution solvent: hexane/ethyl acetate = 1/2), and the resulting solid was washed with diisopropyl ether to obtain the title compound as a white solid (247 mg, 48%). ¹H NMR spectrum (400 MHz, CDCl₃) δppm: 1.52 (9H, s), 6.76 (1H, br, NH), 7.16 (2H, t, J=8.6 Hz), 7.39-7.61 (4H, m), 7.82-7.84 (1H, m), 8.03 (1H, br, NH).

### (1g) 3-Chloro-4'-fluorobiphenyl-4-carboxylic acid hydrazide

To t-butyl 2-[(3-chloro-4'-fluorobiphenyl-4-yl)carbonyl]hydrazinecarboxylate (246 mg, 0.67 mmol) obtained in Example (1f) was added 4 N hydrogen chloride/ethyl acetate (8 mL), and the mixture was stirred at room temperature for 1 h. Saturated aqueous sodium hydrogencarbonate was added, and the mixture was extracted with ethyl acetate, washed with water and saturated brine, and then dried with anhydrous sodium sulfate. The filtrate was concentrated under reduced pressure, and then the resulting solid was collected by filtration and washed with hexane and diisopropyl ether to obtain the title compound as a faint yellow solid (161 mg, 90%).
¹H NMR spectrum (400 MHz, CDCl₃) δppm: 4.12-4.20 (2H, br, NH₂), 7.16 (2H, t, J=8.6 Hz), 7.50-7.61 (5H, m), 7.77 (1H, d, J=8.2 Hz).

### (1h) 8-({[t-Butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine

A solution of 7-({[t-butyl(dimethyl)silyl]oxy}methyl)-7-methyl-5-methylthio-2,3,6,7-tetrahydro-1,4-thiazepine (190 mg, 0.60 mmol) obtained in Example (1d) and 3-chloro-4'-fluorobiphenyl-4-carboxylic acid hydrazide (154 mg, 0.58 mmol) obtained in Example (1g) in n-butanol (10 mL) was stirred at 140°C for 3 h. The mixture was concentrated under reduced pressure, and then the residue was purified by silica gel column chromatography (elution solvent: methylene chloride/methanol = 10/1) to obtain the title compound as a faint yellow oily substance (236 mg, 78%). ¹H NMR spectrum (400 MHz, CDCl₃) δppm: 0.08 (3H, s), 0.10 (3H, s), 0.93 (9H, s), 1.29 (3H, s), 2.92-2.98 (2H, m), 3.53-3.59 (2H, m), 3.61 (1H, d, J=17.3 Hz), 3.63 (1H, d, J=17.3 Hz), 4.16-4.20 (2H, m), 7.16-7.20 (2H, m), 7.56-7.59 (4H, m), 7.70 (1H, d, J=2.0 Hz).

### (li) [3-(3-Chloro-4'-fluorobiphenyl-4-yl)-8-methyl-5,6,8,9-tetrahydro [1,2,4]triazolo[4,3-d][1,4]thiazepin-8-yl]methanol

To a solution of 8-({[t-butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine (236 mg, 1.4 mmol) obtained in Example (1h) in methanol (0.5 mL) was added 4 N hydrogen chloride/dioxane (5 mL), and the mixture was stirred at 70°C for 1h. Saturated aqueous sodium hydrogencarbonate was added, and the mixture was extracted with ethyl acetate, washed with saturated brine, and then dried with anhydrous sodium sulfate. The filtrate was concentrated under reduced pressure, then the residue was purified by silica gel column chromatography (elution solvent: methylene chloride/methanol = 8/1), and the resulting solid was collected by filtration and washed with diethyl ether to obtain the title compound as a white solid (140 mg, 76%).
¹H NMR spectrum (400 MHz, CDCl₃) δppm: 1.41 (3H, s), 2.20-2.30 (1H, m, OH), 2.65-2.75 (1H, m), 2.98-3.08 (1H, m), 3.43 (1H, d, J=16.4 Hz), 3.44-3.52 (2H, m), 3.74 (1H, d, J=16.4 Hz), 4.21-4.25 (2H, m), 7.18 (2H, t, J=8.8 Hz), 7.54-7.60 (4H, m), 7.69 (1H, s).
Mass spectrum (FAB): m/z 404 (M+H)⁺
Melting point: 190-193°C.

### (Example 2) [3-(2-Chlorophenyl)-8-methyl-5,6,8,9-tetrahydro[1,2,4]triazolo[4,3-d][1,4]thiazepin-8-yl]methanol (Example Compound No. 1-7)

### (2a) 8-({[t-Butyl(dimethyl)silyl]oxy}methyl)-3-(2-chlorophenyl)-8-methyl-5,6,8,9-tetrahydro[1,2,4]triazolo[4,3-d][1,4]thiazepine

The title compound was obtained as a colorless oily substance (145 mg, 82%) in the same manner as in Example (1h) using 7-({[t-butyl(dimethyl)silyl]oxy}methyl)-7-methyl-5-methylthio-2,3,6,7-tetrahydro-1,4-thiazepine (133 mg, 0.42 mmol) obtained in Example (1d) and 2-chlorobenzoic acid hydrazide (72.0 mg, 0.42 mmol).
¹H NMR spectrum (400 MHz, CDCl₃) δppm: 0.07 (3H, s), 0.09 (3H, s), 0.93 (9H, s), 1.28 (3H, s), 2.83-2.93 (2H, m), 3.51-3.65 (4H, m), 4.11-4.13 (2H, m), 7.41-7.65 (4H, m).

### (2b) [3-(2-Chlorophenyl)-8-methyl-5,6,8,9-tetrahydro[1,2,4]triazolo[4,3-d] [1,4]thiazepin-8-yl]methanol

The title compound was obtained as a colorless foamy substance (80.2 mg, 77%) in the same manner as in Example (li) using 8-({[t-butyl(dimethyl)silyl]oxy}methyl)-3-(2-chlorophenyl)-8-methyl-5,6,8,9-tetrahydro[1,2,4]triazolo[4,3-d][1,4]thiazepine (143 mg, 0.34 mmol) obtained in Example (2a) and 4 N hydrogen chloride/dioxane (5 mL).
¹H NMR spectrum (400 MHz, CDCl₃) δppm: 1.41 (3H, s), 2.28 (1H, br, OH), 2.64-2.71 (1H, m), 2.97-3.07 (1H, m), 3.46 (1H, d, J=15.9 Hz), 3.50 (2H, s), 3.74 (1H, d, J=15.9 Hz), 4.10-4.21 (2H, m), 7.41-7.45 (1H, m), 7.48-7.55 (3H, m).
Mass spectrum (FAB): m/z 310 (M+H)⁺.

### (Example 3) {8-Methyl-3-[2-(trifluoromethyl)phenyl]-5,6,8,9-tetrahydro[1,2,4]triazolo[4,3-d][1,4]thiazepin-8-yl}methanol (Example Compound No. 1-22)

### (3a) 8-({[t-Butyl(dimethyl)silyl]oxy}methyl)-8-methyl-3-[2-(trifluoromethyl)phenyl]-5,6,8,9-tetrahydro[1,2,4]triazolo[4,3-d][1,4]thiazepine

The title compound was obtained as a colorless oily substance (150 mg, 88%) in the same manner as in Example (1h) using 7-({[t-butyl(dimethyl)silyl]oxy}methyl)-7-methyl-5-methylthio-2,3,6,7-tetrahydro-1,4-thiazepine (134 mg, 0.42 mmol) obtained in Example (1d) and 2-(trifluoromethyl)benzoic acid hydrazide (81.6 mg, 0.40 mmol). This product also contained compounds before dehydration but was used as it was for the subsequent reaction.
¹H NMR spectrum (400 MHz, CDCl₃) δppm: 0.06 (3H, s), 0.08 (3H, s), 0.93 (9H, s), 1.33 (3H, s), 2.68-2.93 (2H, m), 3.13 (1H, d, J=9.8 Hz), 3.17 (1H, d, J=9.8 Hz), 3.52-3.60 (3H, m), 3.78-3.85 (1H, m), 7.41-7.44 (1H, m), 7.50-7.54 (1H, m), 7.65-7.72 (2H, m).

### (3b) 8-Methyl-3-[2-(trifluoromethyl)phenyl]-5,6,8,9-tetrahydro[1,2,4]triazolo[4,3-d] [1,4]thiazepin-8-yl]methanol

8-({[t-Butyl(dimethyl)silyl]oxy}methyl)-8-methyl-3-[2-(trifluoromethyl)phenyl]-5,6,8,9-tetrahydro[1,2,4]triazolo[4,3-d][1,4]thiazepine (157 mg, 0.35 mmol) obtained in Example (3a) and 4 N hydrogen chloride/dioxane (5 mL) were used in the same manner as in Example (li), the resulting product was further dissolved in toluene (5 mL) and acetic acid (0.5 mL), and the mixture was stirred at 120°C for 3 h. Saturated aqueous sodium hydrogencarbonate was added to the reaction mixture, and the mixture was extracted with ethyl acetate, then washed with saturated brine, and dried with anhydrous sodium sulfate. The filtrate was concentrated under reduced pressure, and then the residue was purified by silica gel column chromatography (elution solvent: methylene chloride/methanol = 8/1) to obtain the title compound as a white foamy substance (25.2 mg, 21%). ¹H NMR spectrum (400 MHz, CDCl₃) δppm: 1.39 (3H, s), 2.35 (1H, br, OH), 2.55-2.64 (1H, m), 2.85-2.93 (1H, m), 3.43 (1H, d, J=13.3 Hz), 3.43-3.51 (2H, m), 3.73 (1H, d, J=13.3 Hz), 4.06-4.14 (2H, m), 7.42-7.46 (1H, m), 7.67-7.72 (2H, m), 7.83-7.87 (1H, m).
Mass spectrum (FAB): m/z 344 (M+H)⁺.

### (Example 4) {8-Methyl-3-[2-(trifluoromethoxy)phenyl]-5,6,8,9-tetrahydro[1,2,4]triazolo[4,3-d][1,4]thiazepin-8-yl}methanol (Example Compound No. 1-25)

### (4a) 8-({[t-Butyl(dimethyl)silyl]oxy}methyl)-8-methyl-3-[2-(trifluoromethoxy)phenyl]-5,6,8,9-tetrahydro[1,2,4]triazolo[4,3-d][1,4]thiazepine

The title compound was obtained as a colorless oily substance (148 mg, 75%) in the same manner as in Example (1h) using 7-({[t-butyl(dimethyl)silyl]oxy}methyl)-7-methyl-5-methylthio-2,3,6,7-tetrahydro-1,4-thiazepine (140 mg, 0.44 mmol) obtained in Example (1d) and 2-(trifluoromethoxy)benzoic acid hydrazide (91.0 mg, 0.41 mmol). ¹H NMR spectrum (400 MHz, CDCl₃) δppm: 0.07 (3H, s), 0.08 (3H, s), 0.93 (9H, s), 1.27 (3H, s), 2.91-2.95 (2H, m), 3.53 (2H, s), 3.58 (1H, d, J=14.0 Hz), 3.62 (1H, d, J=14.0 Hz), 4.12-4.17 (2H, m), 7.39-7.47 (2H, m), 7.57-7.65 (2H, m).

### (4b) {8-Methyl-3-[2-(trifluoromethoxy)phenyl]-5,6,8,9-tetrahydro[1,2,4]triazolo[4,3-d] [1,4]thiazepin-8-yl}methanol

The title compound was obtained as a white foamy substance (85.5 mg, 77%) in the same manner as in Example (li) using 8-({[t-butyl(dimethyl)silyl]oxy}methyl)-8-methyl-3-[2-(trifluoromethoxy)phenyl]-5,6,8,9-tetrahydro[1,2,4]triazolo[4,3-d][1,4]thiazepine (147 mg, 0.31 mmol) obtained in Example (4a) and 4 N hydrogen chloride/dioxane (5 mL).
¹H NMR spectrum (400 MHz, CDCl₃) δppm: 1.39 (3H, s), 2.39 (1H, br, OH), 2.63-2.76 (1H, m), 2.94-3.04 (1H, m), 3.46 (1H, d, J=15.3 Hz), 3.51 (2H, s), 3.72 (1H, d, J=15.2 Hz), 4.13-4.24 (2H, m), 7.35-7.52 (2H, m), 7.56-7.66 (2H, m).
Mass spectrum (EI): m/z 360 (M+H)⁺.

### (Example 5) [8-Methyl-3-(naphthalen-1-yl)-5,6,8,9-tetrahydro[1,2,4]triazolo[4,3-d][1,4]thiazepin-8-yl]methanol (Example Compound No. 1-35)

### (5a) 8-({[t-Butyl(dimethyl)silyl]oxy}methyl)-8-methyl-3-(naphthalen-1-yl)-5,6,8,9-tetrahydro[1,2,4]triazolo[4,3-d][1,4]thiazepine

The title compound was obtained as a colorless oily substance (152 mg. 81%) in the same manner as in Example (1h) using 7-({[t-butyl(dimethyl)silyl]oxy}methyl)-7-methyl-5-methylthio-2,3,6,7-tetrahydro-1,4-thiazepine (142 mg, 0.45 mmol) obtained in Example (1d) and naphthalene-1-carboxylic acid hydrazide (80.2 mg, 0.43 mmol).
¹H NMR spectrum (400 MHz, CDCl₃) δppm: 0.10 (3H, s), 0.12 (3H, s), 0.95 (9H, s), 1.33 (3H, s), 2.74-2.80 (1H, m), 2.82-2.87 (1H, m), 3.54 (1H, d, J=15.0 Hz), 3.57 (1H, d, J=15.0 Hz), 3.60-3.68 (2H, m), 4.05-4.08 (2H, m), 7.51-7.59 (5H, m), 7.94 (1H, d, J=7.8 Hz), 8.02 (1H, dd, J=2.4, 6.8 Hz).

### (5b) [8-Methyl-3-(naphthalen-1-yl)-5,6,8,9-tetrahydro[1,2,4]triazolo[4,3-d][1,4]thiazepin-8-yl]methanol

The title compound was obtained as a white foamy substance (81.7 mg, 77%) in the same manner as in Example (li) using 8-({[t-butyl(dimethyl)silyl]oxy}methyl)-8-methyl-3-(naphthalen-1-yl)-5,6,8,9-tetrahydro[1,2,4]triazolo[4,3-d][1,4]thiazepine (152 mg, 0.35 mmol) obtained in Example (5a) and 4 N hydrogen chloride/dioxane (5 mL).
¹H NMR spectrum (400 MHz, CDCl₃) δppm: 1.43 (3H, s), 2.49-2.58 (1H, m), 2.82-2.92 (1H, m), 3.48 (1H, d, J=15.3 Hz), 3.50-3.58 (2H, m), 3.83 (1H, d, J=15.3 Hz), 4.06-4.15 (2H, m), 7.44-7.64 (5H, m), 7.93 (1H, d, J=7.8 Hz), 8.02 (1H, dd, J=2.4, 7.5 Hz).
Mass spectrum (FAB): m/z 325 (M⁺).

### (Example 6) [8-Methyl-3-(1-methyl-1H-indol-4-yl)-5,6,8,9-tetrahydro[1,2,4]triazolo[4,3-d][1,4]thiazepin-8-yl]methanol (Example Compound No. 1-37)

### (6a) 8-({[t-Butyl(dimethyl)silyl]oxy}methyl)-8-methyl-3-(1-methyl-1H-indol-4-yl)-5,6,8,9-tetrahydro[1,2,4]triazolo[4,3-d][1,4]thiazepine

Hydrazine monohydrate (2 mL, 32 mmol) was added to a solution of methyl 1-methyl-1H-indole-4-carboxylate (700 mg, 3.7 mmol) in ethanol (5 mL), and the mixture was stirred at 100°C for 2 h. Water was added, and the mixture was extracted with ethyl acetate, washed with water and saturated brine, and then dried with anhydrous sodium sulfate. The filtrate was concentrated under reduced pressure, and then the resulting solid was collected by filtration and washed with diethyl ether to obtain 1-Methyl-1H-indole-4-carboxylic acid hydrazide as a white solid (467 mg, 66%).

The title compound was obtained as a colorless oily substance (153 mg, 93%) in the same manner as in Example (1h) using 7-({[t-butyl(dimethyl)silyl]oxy}methyl)-7-methyl-5-methylthio-2,3,6,7-tetrahydro-1,4-thiazepine (122 mg, 0.38 mmol) obtained in Example (1d) and 1-methyl-1H-indole-4-carboxylic acid hydrazide (72.0 mg, 0.38 mmol) obtained above. ¹H NMR spectrum (400 MHz, CDCl₃) δppm: 0.08 (3H, s), 0.10 (3H, s), 0.93 (9H, s), 1.30 (3H, s), 2.82-2.92 (2H, m), 3.51 (1H, d, J=15.2 Hz), 3.57 (1H, d, J=15.2 Hz), 3.62 (1H, d, J=10.2 Hz), 3.66 (1H, d, J=10.2 Hz), 3.86 (3H, s), 4.18-4.24 (1H, m), 4.34-4.40 (1H, m), 6.44 (1H, d, J=3.1 Hz), 7.21-7.34 (3H, m), 7.47 (1H, d, J=8.3 Hz).

### (6b) [8-Methyl-3-(1-methyl-1H-indol-4-yl)-5,6,8,9-tetrahydro[1,2,4]triazolo[4,3-d][1,4]thiazepin-8-yl]methanol

The title compound was obtained as a white foamy substance (72.5 mg, 62%) in the same manner as in Example (li) using 8-({[t-butyl(dimethyl)silyl]oxy}methyl)-8-methyl-3-(1-methyl-1H-indol-4-yl)-5,6,8,9-tetrahydro[1,2,4]triazolo[4,3-d][1,4]thiazepine (153 mg, 0.35 mmol) obtained in Example (6a) and 4 N hydrogen chloride/dioxane (5 mL).
¹H NMR spectrum (400 MHz, CDCl₃) δppm: 1.42 (3H, s), 2.58 (1H, dd, J=5.5, 14.5 Hz), 2.88 (1H, dd, J=9.0, 14.5 Hz), 3.44 (1H, d, J=14.9 Hz), 3.45-3.49 (2H, m), 3.76 (1H, d, J=14.9 Hz), 3.86 (3H, s), 4.23 (1H, dd, J=8.6, 14.0 Hz), 4.45 (1H, dd, J=6.1, 14.0 Hz), 6.41 (1H, d, J=3.1 Hz), 7.15 (1H, d, J=3.1 Hz), 7.21 (1H, d, J=8.0 Hz), 7.32 (1H, t, J=8.0 Hz), 7.48 (1H, d, J=8.0 Hz).
Mass spectrum (EI): m/z 328 (M⁺).

### (Example 7) {3-[1-(Cyclopropylmethyl)-1H-indol-4-yl]-8-methyl-5,6,8,9-tetrahydro[1,2,4]triazolo[4,3-d][1,4]thiazepin-8-yl}methanol (Example Compound No. 1-38)

### (7a) Methyl 1-(cyclopropylmethyl)-1H-indole-4-carboxylate

Sodium hydride (63%, 85.0 mg, 2.2 mmol) was added to a solution of methyl 1H-indole-4-carboxylate (255 mg, 1.5 mmol) in N,N-dimethylformamide (10 mL) with ice cooling, and the mixture was stirred for 10 min, followed by the addition of bromomethylcyclopropane (330 mg, 2.4 mmol), and stirred at room temperature for 3 h. Dilute hydrochloric acid was added to the reaction mixture, and the mixture was extracted with ethyl acetate, washed with water, saturated aqueous sodium hydrogencarbonate, and saturated brine, and then dried with anhydrous sodium sulfate. The filtrate was concentrated under reduced pressure, and then the residue was purified by silica gel column chromatography (elution solvent: hexane/ethyl acetate = 3/1) to obtain the title compound as a colorless oily substance (250 mg, 75%).
¹H NMR spectrum (400 MHz, CDCl₃) δppm: 0.37-0.39(2H, m), 0.63-0.66 (2H, m), 1.21-1.30 (1H, m), 3.98 (3H, s), 4.03 (2H, d, J=6.6 Hz), 7.13 (1H, d, J=3.0 Hz), 7.22-7.30 (1H, m), 7.37 (1H, d, J=3.0 Hz), 7.57 (1H, d, J=7.6 Hz), 7.90 (1H, d, J=7.4 Hz).

### (7b) 1-(Cyclopropylmethyl)-1H-indole-4-carboxylic acid hydrazide

To a solution of methyl 1-(cyclopropylmethyl)-1H-indole-4-carboxylate (250 mg, 1.1 mmol) obtained in Example (7a) in ethanol (10 mL) was added hydrazine monohydrate (2 mL, 32 mmol), and the mixture was stirred at 100°C for 2 h. Water was added, and the mixture was extracted with ethyl acetate, washed with water and saturated brine, and then dried with anhydrous sodium sulfate. The filtrate was concentrated under reduced pressure, and then the resulting solid was collected by filtration and washed with diisopropyl ether to obtain the title compound as a white solid (170 mg, 68%).
¹H NMR spectrum (400 MHz, CDCl₃) δppm: 0.37-0.39(2H, m), 0.63-0.66 (2H, m), 1.21-1.29 (1H, m), 4.03 (2H, d, J=6.6 Hz), 4.18 (2H, br, NH₂), 6.86 (1H, d, J=2.7 Hz), 7.24-7.30 (1H, m), 7.35-7.41 (2H, m), 7.42-7.56(2H, m).

### (7c) 8-({[t-Butyl(dimethyl)silyl]oxy}methyl)-3-[1-(cyclopropylmethyl)-1H-indol-4-yl]-8-methyl-5,6,8,9-tetrahydro[1,2,4]triazolo[4,3-d][1,4]thiazepine

The title compound was obtained as a colorless oily substance (159 mg, 59%) in the same manner as in Example (1h) using 7-({[t-butyl(dimethyl)silyl]oxy}methyl)-7-methyl-5-methylthio-2,3,6,7-tetrahydro-1,4-thiazepine (195 mg, 0.61 mmol) obtained in Example (1d) and 1-(cyclopropylmethyl)-1H-indole-4-carboxylic acid hydrazide (128 mg, 0.56 mmol) obtained in Example (7b).
¹H NMR spectrum (400 MHz, CDCl₃) δppm: 0.08 (3H, s), 0.10 (3H, s), 0.36-0.40(2H, m), 0.65-0.69 (2H, m), 0.94 (9H, s), 1.21-1.29 (1H, m), 1.30 (3H, s), 2.80-2.88 (2H, m), 3.51-3.56 (2H, m),3.60-3.68 (2H, m), 4.04 (2H, d, J=6.6 Hz), 4.18-4.41 (2H, m), 6.46 (1H, d, J=3.5 Hz), 7.19-7.31 (3H, m), 7.49 (1H, d, J=7.0 Hz).

### (7d) {3-[1-(Cyclopropylmethyl)-1H-indol-4-yl]-8-methyl-5,6,8,9-tetrahydro[1,2,4]triazolo[4,3-d][1,4]thiazepin-8-yl}methanol

The title compound was obtained as a white foamy substance (63.2 mg, 51 %) in the same manner as in Example (li) using 8-({[t-butyl(dimethyl)silyl]oxy}methyl)-3-[1-(cyclopropylmethyl)-1H-indol-4-yl]-8-methyl-5,6,8,9-tetrahydro[1,2,4]triazolo[4,3-d][1,4]thiazepine (159 mg, 0.33 mmol) obtained in Example (7c) and 4 N hydrogen chloride/dioxane (5 mL).
¹H NMR spectrum (400 MHz, CDCl₃) δppm: 0.36-0.40 (2H, m), 0.64-0.68 (2H, m), 1.26-1.32 (1H, m), 1.40 (3H, s). 2.59 (1H, dd, J=6.7, 15.0 Hz), 2.79 (1H, br, OH), 2.90 (1H, dd, J=5.1, 15.0 Hz), 3.45 (1H, d, J=15.3 Hz), 3.50 (2H, s), 3.76 (1H, d, J=15.3 Hz), 4.04 (2H, d, J=7.0 Hz), 4.25 (1H, dd, J=9.8, 15.0 Hz), 4.48 (1H, dd, J=5.4, 15.0 Hz), 6.44 (1H, d, J=2.8 Hz), 7.19 (1H, d, J=7.0 Hz), 7.25-7.32 (2H, m), 7.52 (1H, d, J=8.2 Hz).
Mass spectrum (FAB): m/z 369 (M+H)⁺.

### (Example 8) [3-(5-Chloro-3-methyl-1-benzothiophen-2-yl)-8-methyl-5,6,8,9-tetrahydro[1,2,4]triazolo[4,3-d][1,4]thiazepin-8-yl]methanol (Example Compound No. 1-39)

### (8a) t-Butyl 2-[(5-chloro-3-methyl-1-benzothiophen-2-yl)carbonyl]hydrazinecarboxylate

To a solution of 5-chloro-3-methyl-1-benzothiophene-2-carboxylic acid (250 mg, 1.1mmol) in N,N-dimethylacetamide (10 mL) were added t-butyl carbazate (220 mg, 1.7 mmol) and 1-[3-(dimethylamino)propyl]3-ethylcarbodiimide monohydrochloride (308 mg, 1.6 mmol), and the mixture was stirred overnight. Dilute hydrochloric acid was added, and the mixture was extracted with ethyl acetate, washed with water, saturated aqueous sodium hydrogencarbonate, and saturated brine, and then dried with anhydrous sodium sulfate. The filtrate was concentrated under reduced pressure, and then the residue was purified by silica gel column chromatography (elution solvent: hexane/ethyl acetate = 1/1) to obtain the title compound as a white solid (101 mg, 27%).
¹H NMR spectrum (400 MHz, CDCl₃) δppm: 1.52 (9H, s), 2.70 (3H, s), 6.62 (1H, br, NH), 7.44 (1H, dd, J=2.0, 8.6 Hz), 7.58 (1H, br, NH), 7.75 (1H, d, J=8.6 Hz), 7.80(1H, d, J=2.0 Hz).

### (8b) 5-Chloro-3-methyl-1-benzothiophene-2-carboxylic acid hydrazide

The title compound was obtained as a white solid (60.2 mg, 85%) in the same manner as in Example (1g) using t-butyl 2-[(5-chloro-3-methyl-1-benzothiophen-2-yl)carbonyl]hydrazinecarboxylate (100 mg, 0.29 mmol) obtained in Example (8a) and 4 N hydrogen chloride/ethyl acetate (5 mL).
¹H NMR spectrum (400 MHz, CDCl₃) δppm: 2.68 (3H, d, J=1.6 Hz), 4.14 (2H, br, NH₂), 7.16 (1H, br, NH), 7.43 (1H, dd, J=2.0, 8.2 Hz), 7.74 (1H, d, J=8.2 Hz), 7.78(1H, d, J=2.0 Hz).

### (8c) 8-({[t-Butyl(dimethyl)silyl]oxy}methyl)-3-(5-chloro-3-methyl-1-benzothiophen-2-yl)-8-methyl-5,6,8,9-tetrahydro[1,2,4]triazolo[4,3-d][1,4]thiazepine

The title compound was obtained as a colorless oily substance (111 mg, 92%) in the same manner as in Example (1h) using 7-({[t-butyl(dimethyl)silyl]oxy}methyl)-7-methyl-5-methylthio-2,3,6,7-tetrahydro-1,4-thiazepine (86.5 mg, 0.27 mmol) obtained in Example (1d) and 5-chloro-3-methyl-1-benzothiophene-2-carboxylic acid hydrazide (58.9 mg, 0.24 mmol) obtained in Example (8b).
¹H NMR spectrum (400 MHz, CDCl₃) δppm: 0.07 (3H, s), 0.10 (3H, s), 0.93 (9H, s), 1.27 (3H, s), 2.40 (3H, s), 2.82-2.88 (1H, m), 2.96-3.03 (1H, m), 3.52 (2H, s), 3.62 (1H, d, J=13.5 Hz), 3.64 (1H, d, J=13.5 Hz), 4.22-4.28 (1H, m), 4.30-4.3 (1H, m), 7.42 (1H, dd, J=1.6, 8.2 Hz), 7.77-7.80 (2H, m).

### (8d) [3-(5-Chloro-3-methyl-1-benzothiophen-2-yl)-8-methyl-5,6,8,9-tetrahydro[1,2,4]triazolo[4,3-d][1,4]thiazepin-8-yl]methanol

The title compound was obtained as a white foamy substance (76.5 mg, 93%) in the same manner as in Example (li) using 8-({[t-butyl(dimethyl)silyl]oxy}methyl)-3-(5-chloro-3-methyl-1-benzothiophen-2-yl)-8-methyl-5,6,8,9-tetrahydro[1,2,4]triazolo[4,3-d][1,4]thiazepine (107 mg, 0.22 mmol) obtained in Example (8c) and 4 N hydrogen chloride/dioxane (5 mL).
¹H NMR spectrum (400 MHz, CDCl₃) δppm: 1.40 (3H, s), 2.38 (3H, s), 2.69 (1H, dd, J=6.0, 15.0 Hz), 2.98 (1H, dd, J=10.0, 15.0 Hz), 3.44 (1H, d, J=15.2 Hz), 3.51 (2H, s), 3.75 (1H, d, J=15.2 Hz), 4.24-4.30 (1H, m), 4.43-4.47 (1H, m), 7.42 (1H, dd, J=1.5, 8.2 Hz), 7.75-7.78 (2H, m).
Mass spectrum (FAB): m/z 380 (M+H)⁺.

### (Example 9) [3-(3-Chlorobiphenyl-4-yl)-8-methyl-5,6,8,9-tetrahydro[1,2,4]triazolo[4,3-d][1,4]thiazepin-8-yl]methanol (Example Compound No. 1-40)

### (9a) 3-(4-Bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine

The title compound (2.37 g, yield 76%) was obtained in the same manner as in Example (1h) using 7-({[t-butyl(dimethyl)silyl]oxy}methyl)-7-methyl-5-methylthio-2,3,6,7-tetrahydro-1,4-thiazepine (1.90 g, 6.21 mmol) obtained in Example (1d) and 4-bromo-2-chlorobenzoic acid hydrazide (Tetrahedron. 2005. 22. 5323-5350.) (1.55 g, 6.21 mmol).
¹H NMR spectrum (400 MHz, CDCl₃) δppm: 0.07 (3H, s), 0.09 (3H, s), 0.93 (9H, s), 1.27 (3H, s), 2.93 (2H, brs), 3.50 (1H, d, J=15.0 Hz), 3.55 (1H, d, J=15.0 Hz), 3.59 (1H, d, J=9.8 Hz), 3.63 (1H, d, J=9.8 Hz), 4.12 (2H, t, J=4.7 Hz), 7.38 (1H, d, J=8.2 Hz), 7.56 (1H, dd, J=2.0, 8.2 Hz), 7.70 (1H, d, J=2.0 Hz).

### (9b) 8-({[t-Butyl(dimethyl)silyl]oxy}methyl)-3-(3-chlorobiphenyl-4-yl)-8-methyl-5,6,8,9-tetrahydro[1,2,4]triazolo[4,3-d][1,4]thiazepine

The title compound (156 mg, yield 99%) was obtained in the same manner as in Example (lf) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (157 mg, 0.312 mmol) obtained in Example (9a) and phenylboronic acid (45.7 mg, 0.374 mmol).
¹H NMR spectrum (400 MHz, CDCl₃) δppm: 0.08 (3H, s), 0.10 (3H, s), 0.93 (9H, s), 1.29 (3H, s), 2.96 (2H, brs), 3.52 (1H, d, J = 16.0 Hz), 3.57 (1H, d, J = 16.0 Hz), 3.60 (1H, d, J = 10.0 Hz), 3.65 (1H, d, J = 10.0 Hz), 4.17-4.21 (2H, m), 7.42-7.74 (8H, m).

### (9c) [3-(3-Chlorobiphenyl-4-yl)-8-methyl-5,6,8,9-tetrahydro[1,2,4]triazolo[4,3-d] [1,4]thiazepin-8-yl]methanol

To a solution of 8-({[t-butyl(dimethyl)silyl]oxy}methyl)-3-(3-chlorobiphenyl-4-yl)-8-methyl-5,6,8,9-tetrahydro[1,2,4]triazolo[4,3-d][1,4]thiazepine (156 mg, 0.312 mmol) obtained in Example (9b) in tetrahydrofuran (2 mL) was added tetrabutylammonium fluoride (1 M tetrahydrofuran solution, 241 µL, 0.263 mmol), and the mixture was stirred at room temperature for 1 h. Water (10 mL) was added to the reaction mixture, and the mixture was extracted with ethyl acetate (10 mL), then washed with water (10 mL, twice), and dried with anhydrous sodium sulfate. The filtrate was concentrated under reduced pressure, and then the residue was crystallized from ether to obtain the title compound as a white powder (85.1 mg, 71%).
¹H NMR spectrum (400 MHz, CDCl₃) δppm: 1.41 (3H, s), 2.23 (1H, t, J=6.6 Hz), 2.66-2.72 (1H, m), 3.01 (1H, brs), 3.46 (1H, d, J=115.5 Hz), 3.50 (2H, d, J=6.6 Hz), 3.74 (1H, d, J=15.5 Hz), 4.22-4.25 (2H, m), 7.43-7.64 (7H, m), 7.74 (1H, s).
Mass spectrum (ESI): 386.1088 (M+H)⁺
Melting point: 166-169°C.

### (Example 10) [3-(4'-Fluoro-3-methoxybiphenyl-4-yl)-8-methyl-5,6,8,9-tetrahydro[1,2,4]triazolo[4,3-d][1,4]thiazepin-8-yl]methanol (Example Compound No. 1-105)

### (10a) Methyl 4'-fluoro-3-methoxybiphenyl-4-carboxylate

Trifluoromethanesulfonic anhydride (1.02 g, 3.6 mmol) was added to a solution of methyl 4-hydroxy-2-methoxybenzoate (452 mg, 2.5 mmol) in methylene chloride (10 mL) and triethylamine (0.8 mL, 5.8 mmol) with ice cooling, and the mixture was stirred for 1 h. Water was added, and the mixture was stirred, followed by the addition of dilute hydrochloric acid, extracted with ethyl acetate, washed with water, saturated aqueous sodium hydrogencarbonate, and saturated brine, and dried with anhydrous sodium sulfate. The filtrate was concentrated under reduced pressure, and then the mixture was dissolved in toluene (15 mL), followed by the addition of ethanol (7 mL), water (3 mL), (4-fluorophenyl)boronic acid (420 mg, 3.0 mmol), sodium carbonate (430 mg, 4.0 mmol), and tetrakis(triphenylphosphine)palladium(0) (320 mg, 0.28 mmol), and stirred at 100°C for 1 h. The mixture was extracted with ethyl acetate, then washed with water and saturated brine, and dried with anhydrous sodium sulfate. The filtrate was concentrated under reduced pressure, and then the residue was purified by silica gel column chromatography (elution solvent: hexane/ethyl acetate = 2/1) to obtain the title compound as a colorless oily substance (635 mg, 98%).
¹H NMR spectrum (400 MHz, CDCl₃) δppm: 3.91(3H, s), 3.98 (3H, s), 7.11-7.17 (4H, m), 7.57 (2H, dd, J=5.5, 8.6 Hz), 7.88 (1H, d, J=7.8 Hz).

### (10b) 4'-Fluoro-3-methoxybiphenyl-4-carboxylic acid

To a solution of methyl 4'-fluoro-3-methoxybiphenyl-4-carboxylate (633 mg, 2.4 mmol) obtained in Example (10a) in ethanol (10 mL) were added water (2 mL) and sodium hydroxide (400 mg, 10 mmol), and the mixture was stirred at 100°C for 1 h. Dilute hydrochloric acid was added, and the mixture was concentrated under reduced pressure, then extracted with ethyl acetate, washed with water and saturated brine, and dried with anhydrous sodium sulfate. The filtrate was concentrated under reduced pressure, and then the resulting solid was collected by filtration and washed with hexane to obtain the title compound as a white solid (550 mg, 92%).
¹H NMR spectrum (400 MHz, CDCl₃) δppm: 4.15 (3H, s), 7.14-7.22 (3H, m), 7.31 (1H, d, J=8.5 Hz), 7.56-7.60 (2H, m), 8.24 (1H, d, J=8.2 Hz).

### (10c) t-Butyl 2-[(4'-fluoro-3-methoxybiphenyl-4-yl)carbonyl]hydrazinecarboxylate

The title compound was obtained as a white solid (720 mg, 90%) in the same manner as in Example (1e) using 4'-fluoro-3-methoxybiphenyl-4-carboxylic acid (548 mg, 2.2 mmol) obtained in Example (10b), oxalyl chloride (0.4 mL, 4.6 mmol), N,N-dimethylformamide (catalytic amount), t-butyl carbazate (490 mg, 3.7 mmol), and triethylamine (0.8 mL, 5.8 mmol).
¹H NMR spectrum (400 MHz, CDCl₃) δppm: 1.52 (9H, s), 4.07 (3H, s), 6.93(1H, br, NH), 7.11-7.16 (3H, m), 7.25-7.28 (1H, m), 7.55-7.59 (2H, m), 8.26 (1H, d, J=8.2 Hz), 9.55 (1H, br, NH).

### (10d) 4'-Fluoro-3-methoxybiphenyl-4-carboxylic acid hydrazide

The title compound was obtained as a white solid (440 mg, 85%) in the same manner as in Example (1g) using t-butyl 2-[(4'-fluoro-3-methoxybiphenyl-4-yl)carbonyl]hydrazinecarboxylate (720 mg, 2.0 mmol) obtained in Example (10c) and 4 N hydrogen chloride/ethyl acetate (10 mL).
¹H NMR spectrum (400 MHz, CDCl₃) 8ppm: 4.04 (3H, s), 4.19 (2H, br, NH₂), 7.11 (1H, d, J=1.5 Hz), 7.13-7.21 (2H, m), 7.22-7.28 (1H, m), 7.52-7.61 (2H, m), 8.26 (1H, d, J=9.2 Hz), 8.95 (1H, br, NH).

### (10e) 8-({[t-Butyl(dimethyl)silyl]oxy}methyl)-3-(4'-fluoro-3-methoxybiphenyl-4-yl)-8-methyl-5,6,8,9-tetrahydro[1,2,4]triazolo[4,3-d][1,4]thiazepine

The title compound was obtained as a colorless oily substance (272 mg, 86%) in the same manner as in Example (1h) using 7-({[t-butyl(dimethyl)silyl]oxy}methyl)-7-methyl-5-methylthio-2,3,6,7-tetrahydro-1,4-thiazepine (198 mg, 0.62 mmol) obtained in Example (1d) and 4'-fluoro-3-methoxybiphenyl-4-carboxylic acid hydrazide (160 mg, 0.61 mmol) obtained in Example (10d).
¹H NMR spectrum (400 MHz, CDCl₃) δppm: 0.08 (3H, s), 0.10 (3H, s), 0.93 (9H, s), 1.28 (3H, s), 2.89-2.93 (2H, m), 3.48 (1H, d, J=15.0 Hz), 3.51 (1H, d, J=15.0 Hz), 3.56-3.65 (2H, m), 3.89 (3H, s), 4.10-4.25 (2H, m), 7.12 (1H, d, J=1.5 Hz), 7.14-7.18 (2H, m), 7.20-7.29 (1H, m), 7.52-7.61 (3H, m).

### (10f) [3-(4'-Fluoro-3-methoxybiphenyl-4-yl)-8-methyl-5,6,8,9-tetrahydro[1,2,4]triazolo[4,3-d][1,4]thiazepin-8-yl]methanol

The title compound was obtained as a white solid (134 mg, 63%) in the same manner as in Example (li) using 8-({[t-butyl(dimethyl)silyl]oxy}methyl)-3-(4'-fluoro-3-methoxybiphenyl-4-yl)-8-methyl-5,6,8,9-tetrahydro[1,2,4]triazolo[4,3-d][1,4]thiazepine (272 mg, 0.53 mmol)obtained in Example (10e) and 4 N hydrogen chloride/dioxane (5 mL).
¹H NMR spectrum (400 MHz, CDCl₃) δppm: 1.41 (3H, s), 2.35-2.42 (1H, m, OH), 2.62-2.72 (1H, m), 2.92-3.03 (1H, m), 3.43 (1H, d, J=15.0 Hz), 3.51-3.58 (2H, m), 3.73 (1H, d, J=15.0 Hz), 3.89 (3H, s), 4.18-4.26 (2H, m), 7.13-7.19 (3H, m), 7.22-7.28 (1H, m), 7.56-7.60 (3H, m)
Mass spectrum (EI): m/z 399 (M⁺)
Melting point: 212-215°C.

### (Example 11) [3-(3'-Chloro-3-fluorobiphenyl-4-yl)-8-methyl-5,6,8,9-tetrahydro[1,2,4]triazolo[4,3-d][1,4]thiazepin-8-yl]methanol (Example Compound No. 1-45)

### (11a) 4-Bromo-2-fluorobenzoic acid hydrazide

A solution of 4-bromo-2-fluorobenzoic acid (2.86 g, 13.1 mmol), 1-hydroxybenzotriazole (1.77 g, 13.1 mmol), and 1-(3-dimethylaminopropyl)-3-ethylcarbodiimide monohydrochloride (3.26 g, 17.0 mmol) in N,N-dimethylformamide (50 mL) was added dropwise to a solution of hydrazine monohydrate (6.6 mL,131 mmol) in N,N-dimethylformamide (10 mL) at 0°C. The mixture was stirred at room temperature for 4 h, then water (100 mL) was added to the reaction mixture, and the mixture was extracted 3 times with methylene chloride (100 mL) and dried with anhydrous sodium sulfate. The filtrate was concentrated under reduced pressure, and then the residue was crystallized from ether to obtain the title compound as a white powder (2.26 g, 74%).
¹H NMR spectrum (400 MHz, CDCl₃) δppm: 4.17 (2H, brs), 7.35 (1H, dd, J = 2.0, 11.0 Hz), 7.44 (1H, dd, J=2.0, 8.5 Hz), 7.85 (1H, brs), 7.99 (1H, t, J=8.5 Hz).

### (11b) 3-[4-Bromo-2-fluorophenyl]-8-({[t-butyl(dimethyl)silyl]oxy}methyl)-8-methyl-5,6,8,9-tetrahydro[1,2,4]triazolo[4,3-d][1,4]thiazepine

The title compound was obtained as a white powder (244 mg, 62%) in the same manner as in Example (1h) using 7-({[t-butyl(dimethyl)silyl]oxy}methyl)-7-methyl-5-methylthio-2,3,6,7-tetrahydro-1,4-thiazepine (262 mg, 0.857 mmol) obtained in Example (1d) and 4-bromo-2-fluorobenzoic acid hydrazide (190 mg, 0.815 mmol) obtained in Example (11a).
¹H NMR spectrum (400 MHz, CDCl₃) δppm: 0.06 (3H, s), 0.08 (3H, s), 0.92 (9H, s), 1.26 (3H, s), 2.87-2.93 (1H, m), 2.98-3.04 (1H, m), 3.51 (2H, d, J=1.6 Hz), 3.60 (1H, d, J=10.2 Hz), 3.63 (1H, d, J=10.2 Hz), 4.19-4.23 (2H, m), 7.42 (1H, dd, J=1.6, 9.4 Hz), 7.47 (1H, dd, J=1.6, 8.2 Hz), 7.54 (1H, t, J=8.2 Hz).

### (11c) 8-({[t-Butyl(dimethyl)silyl]oxy}methyl)-3-[3'-chloro-3-fluorobiphenyl-4-yl]-8-methyl-5,6,8,9-tetrahydro[1,2,4]triazolo[4,3-d][1,4]thiazepine

The title compound (127 mg, yield 99%) was obtained in the same manner as in Example (1f) using 3-[4-bromo-2-fluorophenyl]-8-({[t-butyl(dimethyl)silyl]oxy}methyl)-8-methyl-5,6,8,9-tetrahydro[1,2,4]triazolo[4,3-d][1,4]thiazepine (119 mg, 0.245 mmol) obtained in Example (11b) and (3-chlorophenyl)boronic acid (46.0 mg, 0.294 mmol).
¹H NMR spectrum (400 MHz, CDCl₃) δppm: 0.07 (3H, s), 0.09 (3H, s), 0.93 (9H, s), 1.28 (3H, s), 2.92-3.06 (2H, m), 3.53 (2H, d, J=2.3 Hz), 3.61 (1H, d, J=10.0 Hz), 3.65 (1H, d, J=10.0 Hz), 4.23-4.30 (2H, m), 7.39-7.75 (7H, m).

### (11d) [3-(3'-Chloro-3-fluorobiphenyl-4-yl)-8-methyl-5,6,8,9-tetrahydro[1,2,4]triazolo[4,3-d][1,4]thiazepin-8-yl]methanol

The title compound was obtained as a white powder (74.4 mg, 75%) in the same manner as in Example (9c) using 8-({[t-butyl(dimethyl)silyl]oxy}methyl)-3-[3'-chloro-3-fluorobiphenyl-4-yl]-8-methyl-5,6,8,9-tetrahydro[1,2,4]triazolo[4,3-d][1,4]thiazepine (127 mg, 0.245 mmol) obtained in Example (11c).
¹H NMR spectrum (400 MHz, CDCl₃) δppm: 1.41 (3H, s), 2.37 (1H, t, J=7.0 Hz), 2.76 (1H, dd, J=7.6, 15.5 Hz), 3.05 (1H, dd, J=9.0, 15.5 Hz), 3.46 (1H, d, J=15.3 Hz), 3.47-3.53 (2H, m), 3.74 (1H, d, J=15.3 Hz), 4.24-4.39 (2H, m), 7.40-7.54 (5H, m), 7.60 (1H, s), 7.72 (1H, t, J=7.8 Hz).
Mass spectrum (ESI): 404.0998 (M+H)⁺
Melting point: 165-168°C.

### (Example 12) [3-(3-Methoxybiphenyl-4-yl)-8-methyl-5,6,8,9-tetrahydro[1,2,4]triazolo[4,3-d][1,4]thiazepin-8-yl]methanol (Example Compound No. 1-41)

### (12a) Methyl 3-methoxybiphenyl-4-carboxylate

The title compound was obtained as a colorless oily substance (632 mg, 82%) in the same manner as in Example (10a) using methyl 4-hydroxy-2-methoxybenzoate (696 mg, 3.82 mmol), triethylamine (0.786 mL, 5.73 mmol), trifluoromethanesulfonic anhydride (0.940 mL, 5.73 mmol), phenylboronic acid (559 mg, 4.58 mmol), sodium carbonate (1.62 g, 15.3 mmol), and tetrakis(triphenylphosphine)palladium(0) (439 mg, 0.382 mmol).
¹H NMR spectrum (400 MHz, CDCl₃) δppm: 3.91 (3H, s), 3.98 (3H, s), 7.16-7.62 (7H, m), 7.89 (1H, d, J=7.8 Hz).

### (12b) 3-Methoxybiphenyl-4-carboxylic acid hydrazide

The title compound was obtained as a white solid (364 mg, 48%) in the same manner as in Example (7b) using methyl 3-methoxybiphenyl-4-carboxylate (759 mg, 3.13 mmol) obtained in Example (12a) and hydrazine monohydrate (1.60 mL, 31.3 mmol).
¹H NMR spectrum (400 MHz, CDCl₃) δppm: 4.04 (3H, s), 7.16 (1H, d, J=1.6 Hz), 7.32 (1H, dd, J=1.6, 8.2 Hz), 7.38-7.49 (3H, m), 7.60-7.62 (2H, m), 8.27 (1H, d, J=8.2 Hz), 8.95 (1H, brs).

### (12c) 8-({[t-Butyl(dimethyl)silyl]oxy}methyl)-3-(3-methoxybiphenyl-4-yl)-8-methyl-5,6,8,9-tetrahydro[1,2,4]triazolo[4,3-d][1,4]thiazepine

The title compound was obtained as a yellow foamy substance (221 mg, 82%) in the same manner as in Example (1h) using 7-({[t-butyl(dimethyl)silyl]oxy}methyl)-7-methyl-5-methylthio-2,3,6,7-tetrahydro-1,4-thiazepine (232 mg, 1.09 mmol) obtained in Example (1d) and 3-methoxybiphenyl-4-carboxylic acid hydrazide (132 mg, 0.545 mmol) obtained in Example (12b).
¹H NMR spectrum (400 MHz, CDCl₃) δppm: 0.08 (3H, s), 0.10 (3H, s), 0.93 (9H, s), 1.28 (3H, s), 2.93 (2H, t, J=4.7 Hz), 3.48 (1H, d, J=15.0 Hz), 3.54 (1H, d, J=15.0 Hz), 3.60 (1H, d, J=10.0 Hz), 3.64 (1H, d, J=10.0 Hz), 3.89 (3H, s), 4.04-4.24 (2H, m), 7.17 (1H, s), 7.30 (1H, dd, J=1.2, 7.8 Hz), 7.38-7.42 (1H, m), 7.48 (2H, t, J=7.8 Hz), 7.58 (1H, d, J=7.8 Hz), 7.62 (2H, d, J=7.4 Hz).

### (12d) [3-(3-Methoxybiphenyl-4-yl)-8-methyl-5,6,8,9-tetrahydro[1,2,4]triazolo[4,3-d] [1,4]thiazepin-8-yl]methanol

The title compound was obtained as a white foamy substance (149 mg, 89%) in the same manner as in Example (9c) using 8-({[t-butyl(dimethyl)silyl]oxy}methyl)-3-(3-methoxybiphenyl-4-yl)-8-methyl-5,6,8,9-tetrahydro[1,2,4]triazolo[4,3-d][1,4]thiazepine (218 mg, 0.440 mmol) obtained in Example (12c).
¹H NMR spectrum (400 MHz, CDCl₃) δppm: 1.41 (3H, s), 2.70 (1H, dd, J=6.3, 15.6 Hz), 2.89 (1H, brs), 3.03 (1H, dd, J=9.4, 15.3 Hz), 3.44 (1H, d, J=15.3 Hz), 3.50 (1H, d, J=11.7 Hz), 3.55 (1H, d, J=11.7 Hz), 3.72 (1H, d, J=15.3 Hz), 3.89 (3H, s), 4.12-4.29 (2H, m), 7.18 (1H, s), 7.30 (1H, d, J=6.7 Hz), 7.39-7.43 (1H, m), 7.48 (2H, d, J=7.8 Hz), 7.57 (1H, d, J=7.8 Hz), 7.62 (2H, d, J=8.2 Hz).
Mass spectrum (ESI): 382.1596 (M+H)⁺.

### (Example 13) [3-(3-Chloro-2'-fluorobiphenyl-4-yl)-8-methyl-5,6,8,9-tetrahydro[1,2,4]triazolo[4,3-d][1,4]thiazepin-8-yl]methanol (Example Compound No. 1-60)

### (13a) 8-({[t-Butyl(dimethyl)silyl]oxy}methyl)-3-(3-chloro-2'-fluorobiphenyl-4-yl)-8-methyl-5,6,8,9-tetrahydro[1,2,4]triazolo[4,3-d][1,4]thiazepine

The title compound (160 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (156 mg, 0.310 mmol) obtained in Example (9a) and (2-fluorophenyl)boronic acid (52.1 mg, 0.372 mmol).
¹H NMR spectrum (400 MHz, CDCl₃) δppm: 0.09 (3H, s), 0.10 (3H, s), 0.94 (9H, s), 1.30 (3H, s), 2.97 (2H, brs), 3.52 (1H, d, J=14.0 Hz), 3.58 (1H, d, J=14.0 Hz), 3.61 (1H, d, J=11.0 Hz), 3.65 (1H, d, J=11.0 Hz), 4.18-4.22 (2H, m), 7.45-7.72 (7H, m).

### (13b) [3-(3-Chloro-2'-fluorobiphenyl-4-yl)-8-methyl-5,6,8,9-tetrahydro[1,2,4]triazolo[4,3-d][1,4]thiazepin-8-yl]methanol

The title compound was obtained as a white powder (69.8 mg, yield 56%) in the same manner as in Example (9c) using 8-({[t-butyl(dimethyl)silyl]oxy}methyl)-3-(3-chloro-2'-fluorobiphenyl-4-yl)-8-methyl-5,6,8,9-tetrahydro[1,2,4]triazolo[4,3-d][1,4]thiazepine (160 mg, 0.310 mmol) obtained in Example (13a).
¹H NMR spectrum (400 MHz, CDCl₃) δppm: 1.41 (3H, s), 2.32 (1H, t, J=6.7 Hz), 2.68-2.72 (1H, m), 3.03 (1H, brs), 3.46 (1H, d, J=15.0 Hz), 3.51 (2H, d, J=7.0 Hz), 3.74 (1H, d, J=15.0 Hz), 4.24-4.26 (2H, m), 7.18-7.28 (2H, m), 7.38-7.48 (2H, m), 7.56-7.61 (2H, m), 7.72 (1H, s).
Mass spectrum (ESI): 404.1002 (M+H)⁺
Melting point: 147-149°C.

### (Example 14) [3-(3,2'-Dichlorobiphenyl-4-yl)-8-methyl-5,6,8,9-tetrahydro[1,2,4]triazolo[4,3-d][1,4]thiazepin-8-yl]methanol (Example Compound No. 1-61)

### (14a) 8-({[t-Butyl(dimethyl)silyl]oxy}methyl)-3-(3,2'-dichlorobiphenyl-4-yl)-8-methyl-5,6,8,9-tetrahydro[1,2,4]triazolo[4,3-d][1,4]thiazepine

The title compound (211 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (198 mg, 0.394 mmol) obtained in Example (9a) and (2-chlorophenyl)boronic acid (73.9 mg, 0.473 mmol).
¹H NMR spectrum (400 MHz, CDCl₃) δppm: 0.08 (3H, s), 0.10 (3H, s), 0.94 (9H, s), 1.29 (3H, s), 2.98 (2H, brs), 3.52 (1H, d, J=15.3 Hz), 3.57 (1H, d, J=10.6 Hz), 3.61 (1H, d, J=10.0 Hz), 3.65 (1H, d, J=10.0 Hz), 4.19-4.22 (2H, m), 7.34-7.70 (7H, m).

### (14b) [3-(3,2'-Dichlorobiphenyl-4-yl)-8-methyl-5,6,8,9-tetrahydro[1,2,4]triazolo[4,3-d] [1,4]thiazepin-8-yl]methanol

The title compound was obtained as a light yellow powder (81.0 mg, yield 49%) in the same manner as in Example (9c) using 8-({[t-butyl(dimethyl)silyl]oxy}methyl)-3-(3,2'-dichlorobiphenyl-4-yl)-8-methyl-5,6,8,9-tetrahydro[1,2,4]triazolo[4,3-d][1,4]thiazepine (211 mg, 0.394 mmol) obtained in Example (14a).
¹H NMR spectrum (400 MHz, CDCl₃) δppm: 1.42 (3H, s), 2.37 (1H, t, J=6.7 Hz), 2.70-2.76 (1H, m), 3.06 (1H, brs), 3.47 (1H, d, J=15.3 Hz), 3.51 (2H, d, J=6.7 Hz), 3.75 (1H, d, J=15.3 Hz), 4.25-4.28 (2H, m), 7.36-7.37 (3H, m), 7.49-7.52 (2H, m), 7.57 (1H, d, J=8.2 Hz), 7.63 (1H, d, J=1.6 Hz).
Mass spectrum (ESI): 420.0692 (M+H)⁺
Melting point: 177-180°C.

### (Example 15) [3-(3-Chloro-3'-fluorobiphenyl-4-yl)-8-methyl-5,6,8,9-tetrahydro[1,2,4]triazolo[4,3-d][1,4]thiazepin-8-yl]methanol (Example Compound No. 1-62)

### (15a) 8-({[t-Butyl(dimethyl)silyl]oxy}methyl)-3-(3-chloro-3'-fluorobiphenyl-4-yl)-8-methyl-5,6,8,9-tetrahydro[1,2,4]triazolo[4,3-d][1,4]thiazepine

The title compound (169 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (164 mg, 0.326 mmol) obtained in Example (9a) and (3-fluorophenyl)boronic acid (54.7 mg, 0.391 mmol).
¹H NMR spectrum (400 MHz, CDCl₃) δppm: 0.08 (3H, s), 0.10 (3H, s), 0.93 (9H, s), 1.29 (3H, s), 2.96 (2H, brs), 3.52 (1H, d, J=15.0 Hz), 3.57 (1H, d, J=15.0 Hz), 3.61 (1H, d, J=10.2 Hz), 3.65 (1H, d, J=10.2 Hz), 4.16-4.20 (2H, m), 7.11-7.56 (5H, m), 7.60 (1H, s), 7.65-7.77 (1H, m).

### (15b) [3-(3-Chloro-3'-fluorobiphenyl-4-yl)-8-methyl-5,6,8,9-tetrahydro[1,2,4]triazolo[4,3-d] [1,4]thiazepin-8-yl]methanol

The title compound was obtained as a white powder (110 mg, yield 83%) in the same manner as in Example (9c) using 8-({[t-butyl(dimethyl)silyl]oxy}methyl)-3-(3-chloro-3'-fluorobiphenyl-4-yl)-8-methyl-5,6,8,9-tetrahydro[1,2,4]triazolo[4,3-d][1,4]thiazepine (121 mg, 0.219 mmol) obtained in Example (15a).
¹H NMR spectrum (400 MHz, CDCl₃) δppm: 1.41 (3H, s), 2.25 (1H, t, J=7.0 Hz), 2.67-2.73 (1H, m), 3.03 (1H, brs), 3.47 (1H, d, J=15.3 Hz), 3.51 (2H, d, J=5.9 Hz), 3.74 (1H, d, J=15.3 Hz), 4.24 (2H, t, J=5.1 Hz), 7.11-7.16 (1H, m), 7.30 (1H, dt, J=1.6, 9.8 Hz), 7.39 (1H, dt, J=1.2, 7.8 Hz), 7.44-7.49 (1H, m), 7.57-7.63 (2H, m), 7.73 (1H, d, J=1.2 Hz).
Mass spectrum (ESI): 404.1410 (M+H)⁺
Melting point: 206-209°C.

### (Example 16) [3-(3,3'-Dichlorobiphenyl-4-yl)-8-methyl-5,6,8,9-tetrahydro[1,2,4]triazolo[4,3-d][1,4]thiazepin-8-yl]methanol (Example Compound No. 1-63)

### (16a) 8-({[t-Butyl(dimethyl)silyl]oxy}methyl)-3-(3,3'-dichlorobiphenyl-4-yl)-8-methyl-5,6,8,9-tetrahydro[1,2,4]triazolo[4,3-d][11,4]thiazepine

The title compound (166 mg, yield 99%) was obtained in the same manner as in Example (If) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (156 mg, 0.310 mmol) obtained in Example (9a) and (3-chlorophenyl)boronic acid (58.2 mg, 0.372 mmol).
¹H NMR spectrum (400 MHz, CDCl₃) δppm: 0.09 (3H, s), 0.10 (3H, s), 0.94 (9H, s), 1.29 (3H, s), 2.96 (2H, brs), 3.52 (1H, d, J=15.3 Hz), 3.56 (1H, d, J=15.3 Hz), 3.61 (1H, d, J=10.0 Hz), 3.65 (1H, d, J=10.0 Hz), 4.16-4.19 (2H, m), 7.41-7.71 (7H, m).

### (16b) [3-(3,3'-Dichlorobiphenyl-4-yl)-8-methyl-5,6,8,9-tetrahydro[1,2,4]triazolo[4,3-d] [1,4]thiazepin-8-yl]methanol

The title compound was obtained as a light yellow powder (90.3 mg, yield 69%) in the same manner as in Example (9c) using 8-({[t-butyl(dimethyl)silyl]oxy}methyl)-3-(3,3'-dichlorobiphenyl-4-yl)-8-methyl-5,6,8,9-tetrahydro[1,2,4]triazolo[4,3-d][1,4]thiazepine (166 mg, 0.310 mmol) obtained in Example (16a).
¹H NMR spectrum (400 MHz, CDCl₃) δppm: 1.41 (3H, s), 2.27 (1H, brs), 2.67-2.71 (1H, m), 3.0(1H, brs), 3.46 (1H, d, J=15.0 Hz), 3.50-3.51 (2H, m), 3.74 (1H, d, J=15.0 Hz), 4.22-4.24 (2H, m), 7.24-7.26 (1H, m), 7.39-7.50 (3H, m), 7.50-7.59 (2H, m), 7.72 (1H, s).
Mass spectrum (ESI): 420.0688 (M+H)⁺
Melting point: 173-176°C.

### (Example 17) [3-(3-Chloro-3'-trifluoromethylbiphenyl-4-yl)-8-methyl-5,6,8,9-tetrahydro[1,2,4]triazolo[4,3-d][1,4]thiazepin-8-yl]methanol (Example Compound No. 1-65)

### (17a) 8-({[t-Butyl(dimethyl)silyl]oxy}methyl)-3-(3-chloro-3'-trifluoromethylbiphenyl-4-yl)-8-methyl-5,6,8,9-tetrahydro[1,2,4]triazolo[4,3-d][1,4]thiazepine

The title compound (175 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (3-trifluoromethylphenyl)boronic acid (70.2 mg, 0.397 mmol).
¹H NMR spectrum (400 MHz, CDCl₃) δppm: 0.08 (3H, s), 0.10 (3H, s), 0.93 (9H, s), 1.29 (3H, s), 2.96 (2H, brs), 3.52 (1H, d, J=16.0 Hz), 3.57 (1H, d, J=16.0 Hz), 3.61 (1H, d, J=10.0 Hz), 3.65 (1H, d, J=10.0 Hz), 4.17-4.19 (2H, m), 7.44-7.84 (7H, m).

### (17b) [3-(3-Chloro-3'-trifluoromethylbiphenyl-4-yl)-8-methyl-5,6,8,9-tetrahydro[1,2,4]triazolo[4,3-d][1,4]thiazepin-8-yl]methanol

The title compound was obtained as a white powder (107 mg, yield 76%) in the same manner as in Example (9c) using 8-({[t-butyl(dimethyl)silyl]oxy}methyl)-3-(3-chloro-3'-trifluoromethylbiphenyl-4-yl)-8-methyl-5,6,8,9-tetrahydro[1,2,4]triazolo[4,3-d][1,4]thiazepine (175 mg, 0.308 mmol) obtained in Example (17a).
¹H NMR spectrum (400 MHz, CDCl₃) δppm: 1.41 (3H, s), 2.23 (1H, t, J=7.0 Hz), 2.67-2.73 (1H, m), 3.05 (1H, brs), 3.47 (1H, d, J=15.0 Hz), 3.51 (2H, d, J=7.8 Hz), 3.75 (1H, d, J=15.0 Hz), 4.23-4.25 (2H, m), 7.61-7.66 (3H, m), 7.71 (1H, d, J=8.2 Hz), 7.76 (1H, s), 7.80 (1H, d, J=7.4 Hz), 7.85 (1H, s).
Mass spectrum (ESI): 454.1096 (M+H)⁺
Melting point: 195-198°C.

### (Example 18) [3-(3-Chloro-3'-methoxybiphenyl-4-yl)-8-methyl-5,6,8,9-tetrahydro[1,2,4]triazolo[4,3-d][1,4]thiazepin-8-yl]methanol (Example Compound No. 1-66)

### (18a) 8-({[t-Butyl(dimethyl)silyl]oxy}methyl)-3-(3-chloro-3'-methoxybiphenyl-4-yl)-8-methyl-5,6,8,9-tetrahydro[1,2,4]triazolo[4,3-d][1,4]thiazcpine

The title compound (186 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (176 mg, 0.350 mmol) obtained in Example (9a) and (3-methoxyphenyl)boronic acid (63.8 mg, 0.420 mmol).
¹H NMR spectrum (400 MHz, CDCl₃) δppm: 0.08 (3H, s), 0.10 (3H, s), 0.93 (9H, s), 1.29 (3H, s), 2.96 (2H, brs), 3.52 (1H, d, J=14.0 Hz), 3.56 (1H, d, J=14.0 Hz), 3.60 (1H, d, J=10.0 Hz), 3.65 (1H, d, J=10.0 Hz), 3.89 (3H, s), 4.17-4.19 (2H, m), 6.97-7.70 (6H, m), 7.73 (1H, d, J=1.6 Hz).

### (18b) [3-(3-Chloro-3'-methoxybiphenyl-4-yl)-8-methyl-5,6,8,9-tetrahydro[1,2,4]triazolo[4,3-d][1,4]thiazepin-8-yl]methanol

The title compound was obtained as a light yellow powder (51.7 mg, yield 35%) in the same manner as in Example (9c) using 8-({[t-butyl(dimethyl)silyl]oxy}methyl)-3 -(3 -chloro-3'-methoxybiphenyl-4-yl)-8-methyl-5,6,8,9-tetrahydro[1,2,4]triazolo[4,3-d][1,4]thiazepine (176 mg, 0.350 mmol) obtained in Example (18a).
¹H NMR spectrum (400 MHz, CDCl₃) δppm: 1.42 (3H, s), 2.31 (1H, t, J=7.4 Hz), 2.67-2.72 (1H, m), 3.02 (1H, brs), 3.46-3.53 (3H, m), 3.75 (1H, d, J=15.3 Hz), 3.90 (3H, s), 4.24-4.25 (2H, m), 6.97-7.00 (1H, m), 7.13-7.14 (1H, m), 7.20 (1H, d, J=7.4 Hz), 7.42 (1H, t, J=8.2 Hz), 7.56-7.64 (2H, m), 7.75 (1H, d, J=2.0 Hz).
Mass spectrum (FAB): 416 (M+H)⁺
Melting point: 181-184°C.

### (Example 19) [3-(3,4'-Dichlorobiphenyl-4-yl)-8-methyl-5,6,8,9-tetrahydro[1,2,4]triazolo[4,3-d][1,4]thiazepin-8-yl]methanol (Example Compound No. 1-70)

### (19a) 8-({[t-Butyl(dimethyl)silyl]oxy}methyl)-3-(3,4'-dichlorobiphenyl-4-yl)-8-methyl-5,6,8,9-tetrahydro[1,2,4]triazolo[4,3-d][1,4]thiazepine

The title compound (170 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (157 mg, 0.312 mmol) obtained in Example (9a) and (4-chlorophenyl)boronic acid (58.5 mg, 0.374 mmol).
¹H NMR spectrum (400 MHz, CDCl₃) δppm: 0.08 (3H, s), 0.10 (3H, s), 0.94 (9H, s), 1.29 (3H, s), 2.96 (2H, brs), 3.52 (1H, d, J=15.3 Hz), 3.57 (1H, d, J=15.3 Hz), 3.60 (1H, d, J=10.0 Hz), 3.65 (1H, d, J=10.0 Hz), 4.16-4.20 (2H, m), 7.44-7.70 (7H, m).

### (19b) [3-(3,4'-Dichlorobiphenyl-4-yl)-8-methyl-5,6,8,9-tetrahydro[1,2,4]triazolo[4,3-d] [1,4]thiazepin-8-yl]methanol

The title compound was obtained as a white powder (99.6 mg, yield 76%) in the same manner as in Example (9c) using 8-({[t-butyl(dimethyl)silyl]oxy}methyl)-3-(3,4'-dichlorobiphenyl-4-yl)-8-methyl-5,6,8,9-tetrahydro[1,2,4]triazolo[4,3-d][1,4]thiazepine (170 mg, 0.312 mmol) obtained in Example (19a).
¹H NMR spectrum (400 MHz, CDCl₃) δppm: 1.41 (3H, s), 2.27 (1H, t, J=6.6 Hz), 2.67-2.72 (1H, m), 3.02 (1H, brs), 3.46 (1H, d, J=15.3 Hz), 3.47-3.51 (2H, m), 3.74 (1H, d, J=15.3 Hz), 4.22-4.24 (2H, m), 7.46 (2H, d, J=8.7 Hz), 7.54 (2H, d, J=8.7 Hz), 7.51-7.58 (2H, m), 7.71 (1H, s).
Mass spectrum (ESI): 420.0839 (M+H)⁺
Melting point: 219-223°C.

### (Example 20) [3-(3-Chloro-4'-methylbiphenyl-4-yl)-8-methyl-5,6,8,9-tetrahydro[1,2,4]triazolo[4,3-d][1,4]thiazepin-8-yl]methanol (Example Compound No. 1-71)

### (20a) 8-({[t-Butyl(dimethyl)silyl]oxy}methyl)-3-(3-chloro-4'-methylbiphenyl-4-yl)-8-methyl-5,6,8,9-tetrahydro[1,2,4]triazolo[4,3-d][1,4]thiazepine

The title compound (162 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (159 mg, 0.316 mmol) obtained in Example (9a) and 4-tolylboronic acid (51.6 mg, 0.379 mmol).
¹H NMR spectrum (400 MHz, CDCl₃) δppm: 0.08 (3H, s), 0.09 (3H, s), 0.93 (9H, s), 1.29 (3H, s), 2.42 (3H, s), 2.95 (2H, brs), 3.50 (1H, d, J=15.5 Hz), 3.56 (1H, d, J=15.5 Hz), 3.60 (1H, d, J=10.0 Hz), 3.64 (1H, d, J=10.0 Hz), 4.15-4.20 (2H, m), 7.29 (2H, d, J=8.7 Hz), 7.44-7.71 (5H, m).

### (20b) [3-(3-Chloro-4'-methylbiphenyl-4-yl)-8-methyl-5,6,8,9-tetrahydro[1,2,4]triazolo[4,3-d][1,4]thiazepin-8-yl]methanol

The title compound was obtained as a light yellow powder (87.5 mg, yield 69%) in the same manner as in Example (9c) using 8-({[t-butyl(dimethyl)silyl]oxy}methyl)-3-(3-chloro-4'-methylbiphenyl-4-yl)-8-methyl-5,6,8,9-tetrahydro[1,2,4]triazolo[4,3-d][1,4]thiazepine (162 mg, 0.316 mmol) obtained in Example (20a).
¹H NMR spectrum (400 MHz, CDCl₃) δppm: 1.41 (3H, s), 2.30 (1H, brs), 2.42 (3H, s), 2.66-2.72 (1H, m), 3.03 (1H, brs), 3.46 (1H, d, J=15.3 Hz), 3.50 (2H, brs), 3.74 (1H, d, J=15.3 Hz), 4.21-4.24 (2H, m), 7.28 (2H, d, J=8.0 Hz), 7.50 (2H, d, J=8.0 Hz), 7.54 (1H, d, J=7.8 Hz), 7.61 (1H, dd, J=2.0, 8.0 Hz), 7.72 (1H, d, J=2.0 Hz).
Mass spectrum (ESI): 400.1238 (M+H)⁺
Melting point: 223-225°C.

### (Example 21) [3-(3-Chloro-4'-methoxybiphenyl-4-yl)-8-methyl-5,6,8,9-tetrahydro[1,2,4]triazolo[4,3-d] [1 ,4]thiazepin-8-yl]methanol (Example Compound No. 1-73)

### (21a) 8-({[t-Butyl(dimethyl)silyl]oxy}methyl)-3-(3-chloro-4'-methoxybiphenyl-4-yl)-8-methyl-5,6,8,9-tetrahydro[1,2,4]triazolo[4,3-d][1,4]thiazepine

The title compound (182 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (173 mg, 0.344 mmol) obtained in Example (9a) and (4-methoxyphenyl)boronic acid (62.7 mg, 0.413 mmol).
¹H NMR spectrum (400 MHz, CDCl₃) δppm: 0.08 (3H, s), 0.09 (3H, s), 0.93 (9H, s), 1.29 (3H, s), 2.95 (2H, brs), 3.50 (1H, d, J=16.0 Hz), 3.57 (1H, d, J=16.0 Hz), 3.60 (1H, d, J=9.8 Hz), 3.64 (1H, d, J=9.8 Hz), 3.87 (3H, s), 4.13-4.19 (2H, m), 7.01 (2H, d, J=9.0 Hz), 7.45-7.70 (5H, m).

### (21b) [3-(3-Chloro-4'-methoxybiphenyl-4-yl)-8-methyl-5,6,8,9-tetrahydro[1,2,4]triazolo[4,3-d][1,4]thiazepin-8-yl]methanol

The title compound was obtained as a white powder (86.3 mg, yield 60%) in the same manner as in Example (9c) using 8-({[t-butyl(dimethyl)silyl]oxy}methyl)-3-(3-chloro-4'-methoxybiphenyl-4-yl)-8-methyl-5,6,8,9-tetrahydro[1,2,4]triazolo[4,3-d][1,4]thiazepine (182 mg, 0.344 mmol) obtained in Example (21a).
¹H NMR spectrum (400 MHz, CDCl₃) δppm: 1.41 (3H, s), 2.23 (1H, t, J=7.0 Hz), 2.66-2.72 (1H, m), 3.04 (1H, brs), 3.46 (1H, d, J=15.3 Hz), 3.50 (2H, d, J=8.2 Hz), 3.75 (1H, d, J=15.3 Hz), 3.88 (3H, s), 4.22-4.25 (2H, m), 7.02 (2H, d, J=9.0 Hz), 7.52-7.60 (4H, m), 7.71 (1H, d, J=1.6 Hz).
Mass spectrum (ESI): 416.1675 (M+H)⁺
Melting point: 179-182°C.

### (Example 22) {3-[4'-Fluoro-3-(trifluoromethyl)biphenyl-4-yl)-8-methyl-5,6,8,9-tetrahydro[1,2,4]triazolo[4,3-d][1,4]thiazepin-8-yl]methanol (Example Compound No. 1-87)

### (22a) t-Butyl 2-[4-bromo-2-(trifluoromethyl)benzoyl]hydrazinecarboxylate

A solution of 4-bromo-2-(trifluoromethyl)benzoyl chloride (Yamanouchi Pharmaceutical Co., LTD., EP1445253 A1) (12.42 g, 43.2 mmol) in tetrahydrofuran (20 mL) was added dropwise to a solution of t-butyl carbazate (5.71 g, 43.2 mmol) and pyridine (6.98 mL, 86.4 mmol) in tetrahydrofuran (100 mL) at 0°C under a nitrogen atmosphere, and the mixture was stirred at room temperature for 20 h. Water (100 mL) was added to the reaction mixture, and the mixture was extracted with ethyl acetate (100 mL), then washed with 1 M hydrochloric acid (100 mL) and saturated aqueous sodium hydrogencarbonate (100 mL), and dried with anhydrous sodium sulfate. The filtrate was concentrated under reduced pressure, and then the residue was crystallized from ether to obtain the title compound as a white powder (3.89 g, 24%).
¹H NMR spectrum (400 MHz, CDCl₃) δppm: 1.51 (9H, s), 6.64 (1H, brs), 7.43 (1H, brs), 7.82 (1H, d, J=8.0 Hz), 7.77 (1H, dd, J=1.6, 8.0 Hz), 7.88 (1H, d, J=1.6 Hz).

### (22b) 4-Bromo-2-(trifluoromethyl)benzoic acid hydrazide

To a solution of t-butyl 2-[4-bromo-2-(trifluoromethyl)benzoyl]hydrazinecarboxylate (249 mg, 0.650 mmol) obtained in Example (22a) in methanol (2 mL) was added 4 M hydrochloric acid (dioxane solution, 2 mL), and the mixture was stirred at room temperature for 1 h. Saturated aqueous sodium hydrogencarbonate (10 mL) was added to the reaction mixture, and the mixture was extracted with ethyl acetate (10 mL) and then dried with anhydrous sodium sulfate. The filtrate was concentrated under reduced pressure, and then the title compound was obtained as a white powder (181 mg, 99%).
¹H NMR spectrum (500 MHz, CDCl₃) δppm: 4.11 (2H, brs), 6.97 (1H, brs), 7.42 (1H, d, J=8.0 Hz), 7.76 (1H, d, J=8.0 Hz), 7.87 (1H, s).

### (22c) 3-[4-Bromo-2-(trifluoromethyl)phenyl]-8-({ [t-butyl(dimethyl)silyl]oxy}methyl)-8-methyl-5,6,8,9-tetrahydro[1,2,4]triazolo[4,3-d][1,4]thiazepine

The title compound (106 mg, yield 30%) was obtained in the same manner as in Example (1h) using 7-({[t-butyl(dimethyl)silyl]oxy}methyl)-7-methyl-5-methylthio-2,3,6,7-tetrahydro-1,4-thiazepine (208 mg, 0.651 mmol) obtained in Example (1d) and 4-bromo-2-(trifluoromethyl)benzoic acid hydrazide (181 mg, 0.639 mmol) obtained in Example (22b).
¹H NMR spectrum (400 MHz, CDCl₃) δppm: 0.07 (3H, s), 0.09 (3H, s), 0.93 (9H, s), 1.26 (3H, s), 2.82-2.85 (2H, m), 3.49-3.52 (2H, m), 3.57 (1H, d, J=10.0 Hz), 3.61 (1H, d, J=10.0 Hz), 4.04 (2H, t, J=4.7 Hz), 7.32 (1H, d, J=8.6 Hz), 7.83 (1H, dd, J=1.2, 8.6 Hz), 7.98 (1H, d, J=1.2 Hz).

### (22d) 8-({[t-Butyl(dimethyl)silyl]oxy}methyl)-3-[4'-fluoro-3-(trifluoromethyl)biphenyl-4-yl]-8-methyl-5,6,8,9-tetrahydro[1,2,4]triazolo[4,3-d][1,4]thiazepine

The title compound (88.8 mg, yield 99%) was obtained in the same manner as in Example (1f) using 3-[4-bromo-2-(trifluoromethyl)phenyl]-8-({[t-butyl(dimethyl)silyl]oxy}methyl)-8-methyl-5,6,8,9-tetrahydro[1,2,4]triazolo[4,3-d][1,4]thiazepine (86.3 mg, 0.161 mmol) obtained in Example (22c) and (4-fluorophenyl)boronic acid (27.0 mg, 0.193 mmol).
¹H NMR spectrum (400 MHz, CDCl₃) δppm: 0.08 (3H, s), 0.10 (3H, s), 0.94 (9H, s), 1.28 (3H, s), 2.76-2.85 (2H, m), 3.52-3.55 (2H, m), 3.59 (2H, t, J=7.0 Hz), 4.10-4.13 (2H, m), 7.2 (1H, t, J=8.6 Hz), 7.44-7.70 (4H, m), 7.83 (1H, dd, J=2.0, 8.6 Hz), 7.98 (1H, d, J=2.0 Hz).

### (22e) {3-[4'-Fluoro-3-(trifluoromethyl)biphenyl-4-yl)-8-methyl-5,6,8,9-tetrahydro[1,2,4]triazolo[4,3-d][1,4]thiazepin-8-yl]methanol

To a solution of 8-({[t-butyl(dimethyl)silyljoxy}methyl)-3-[4'-fluoro-3-(trifluoromethyl)biphenyl-4-yl]-8-methyl-5,6,8,9-tetrahydro[1,2,4]triazolo[4,3-d][1,4]thiazepine (121 mg, 0.219 mmol) obtained in Example (22d) in tetrahydrofuran (2 mL) was added tetrabutylammonium fluoride (1 M tetrahydrofuran solution, 241 µL, 0.263 mmol), and the mixture was stirred at room temperature for 1 h. Water (10 mL) was added to the reaction mixture, and the mixture was extracted with ethyl acetate (10 mL), then washed with water (10 mL, twice), and dried with anhydrous sodium sulfate. The filtrate was concentrated under reduced pressure, and then the residue was crystallized with ether to obtain the title compound as a white powder (43.6 mg, 46%).
¹H NMR spectrum (400 MHz, CDCl₃) δppm: 1.40 (3H, s), 2.27 (1H, t, J=7.0 Hz), 2.57-2.64 (1H, m), 2.90-2.96 (1H, m), 3.44-3.50 (3H, m), 3.75 (1H, d, J=15.3 Hz), 4.15-4.17 (2H, m), 7.21 (2H, t, J=8.6 Hz), 7.51 (1H, d, J=8.0 Hz), 7.60-7.63 (2H, m), 7.84 (1H, dd, J=1.6, 8.0 Hz), 7.99 (1H, d, J=1.6 Hz).
Mass spectrum (ESI): 438.1291 (M+H)⁺
Melting point: 202-204°C.

### (Example 23) [3-(3'-Fluoro-3-methoxybiphenyl-4-yl)-8-methyl-5,6,8,9-tetrahydro[1,2,4]triazolo[4,3-d][1,4]thiazepin-8-yl]methanol (Example Compound No. 1-98)

### (23a) Methyl 3'-fluoro-3-methoxybiphenyl-4-carboxylate

The title compound was obtained as a colorless oily substance (632 mg, 73%) in the same manner as in Example (10a) using methyl 4-hydroxy-2-methoxybenzoate (603 mg, 3.31 mmol), triethylamine (0.692 mL, 4.97 mmol), trifluoromethanesulfonic anhydride (0.814 mL, 4.97 mmol), (3-fluorophenyl)boronic acid (556 mg, 3.97 mmol), sodium carbonate (1.40 g, 13.2 mmol), and tetrakis(triphenylphosphine)palladium(0) (382 mg, 0.31 mmol).
¹H NMR spectrum (400 MHz, CDCl₃) δppm: 3.92 (3H, s), 3.99(3H, s), 7.10 (1H, dt, J=1.6, 7.0 Hz), 7.14 (1H, d, J=1.6 Hz), 7.19 (1H, dd, J=1.6, 8.2 Hz), 7.3 0 (1H, dt, J=1.6, 9.4 Hz), 7.37-7.46 (2H, m), 7.89 (1H, d, J=7.8 Hz).

### (23b) 3'-Fluoro-3-methoxybiphenyl-4-carboxylic acid hydrazide

The title compound was obtained as a white solid (317 mg, 50%) in the same manner as in Example (7b) using methyl 3'-fluoro-3-methoxybiphenyl-4-carboxylate (632 mg, 2.43 mmol) obtained in Example (23a) and hydrazine monohydrate (1.20 mL, 24:3 mmol).
¹H NMR spectrum (400 MHz, CDCl₃) δppm: 4.04 (3H, s), 4.19 (2H, brs), 7.10 (1H, t, J=9.8 Hz), 7.13 (1H, s), 7.29-7.31 (2H, m), 7.37-7.46 (2H, m), 8.28 (1H, d, J=8.2 Hz), 8.94 (1H, brs).

### (23c) 8-({[t-Butyl(dimethyl)silyl]oxy}methyl)-3-(3'-fluoro-3-methoxybiphenyl-4-yl)-8-methyl-5,6,8,9-tetrahydro[1,2,4]triazolo[4,3-d] [1,4]thiazepine

The title compound was obtained as a white foamy substance (115 mg, 49%) in the same manner as in Example (1h) using 7-({[t-butyl(dimethyl)silyl]oxy}methyl)-7-methyl-5-methylthio-2,3,6,7-tetrahydro-1,4-thiazepine (156 mg, 0.510 mmol) obtained in Example (1d) and 3'-fluoro-3-methoxybiphenyl-4-carboxylic acid hydrazide (119 mg, 0.459 mmol) obtained in Example (23b).
¹H NMR spectrum (400 MHz, CDCl₃) δppm: 0.08 (3H, s), 0.09 (3H, s), 0.93 (9H, s), 1.28 (3H, s), 2.93 (2H, t, J=5.1 Hz), 3.48 (1H, d, J=15.3 Hz), 3.54 (1H, d, J=15.3 Hz), 3.60 (1H, d, J=10.0 Hz), 3.64 (1H, d, J=10.0 Hz), 3.89 (3H, s), 4.18-4.20 (2H, m), 7.10-7.15 (2H, m), 7.28-7.45 (4H, m), 7.60 (1H, d, J=7.8 Hz).

### (23d) [3-(3'-Fluoro-3-methoxybiphenyl-4-yl)-8-methyl-5,6,8,9-tetrahydro[1,2,4]triazolo[4,3-d][1,4]thiazepin-8-yl]methanol

The title compound was obtained as a white foamy substance (36.0 mg, 47%) in the same manner as in Example (9c) using 8-({[t-butyl(dimethyl)silyl]oxy}methyl)-3-(3'-fluoro-3-methoxybiphenyl-4-yl)-8-methyl-5,6,8,9-tetrahydro[1,2,4]triazolo[4,3-d][1,4]thiazepine (115 mg, 0.224 mmol) obtained in Example (23c).
¹H NMR spectrum (400 MHz, CDCl₃) δppm: 1.41 (3H, s), 2.57 (1H, brs), 2.69 (1H, dd, J=6.3, 15.0 Hz), 3.02 (1H, dd, J=9.0, 15.0 Hz), 3.44 (1H, d, J=15.0 Hz), 3.49-3.51 (2H, m), 3.72 (1H, d, J=15.0 Hz), 3.90 (3H, s), 4.17-4.29 (2H, m), 7.11 (1H, t, J=8.2 Hz), 7.15 (1H, s), 7.29-7.32 (2H, m), 7.39-7.47 (2H, m), 7.58 (1H, d, J=7.8 Hz).
Mass spectrum (ESI): 400.1484 (M+H)⁺.

### (Example 24) [3-(3'-Chloro-3-methoxybiphenyl-4-yl)-8-methyl-5,6,8,9-tetrahydro[1,2,4]triazolo[4,3-d][1,4]thiazepin-8-yl]methanol (Example Compound No. 1-99)

### (24a) Methyl 3'-chloro-3-methoxybiphenyl-4-carboxylate

The title compound was obtained as a colorless oily substance (419 mg, 100%) in the same manner as in Example (10a) using methyl 4-hydroxy-2-methoxybenzoate (268 mg, 1.5 mmol), triethylamine (0.5 mL, 3.6 mmol), trifluoromethanesulfonic anhydride (560 mg, 2.0 mmol), 3-chlorophenylboronic acid (280 mg, 1.8 mmol), sodium carbonate (310 mg, 3.0 mmol), and tetrakis(triphenylphosphine)palladium(0) (170 mg, 0.15 mmol). ¹H NMR spectrum (400 MHz, CDCl₃) δppm: 3.91(3H, s), 3.98 (3H, s), 7.12 (1H, s), 7.16 (1H, d, J=8.2 Hz), 7.36-7.41 (2H, m), 7.46-7.49 (1H, m), 7.57 (1H, s), 7.88 (1H, d, J=8.2 Hz).

### (24b) 3'-Chloro-3-methoxybiphenyl-4-carboxylic acid hydrazide

To a solution of methyl 3'-chloro-3-methoxybiphenyl-4-carboxylate (314 mg, 1.1 mmol) obtained in Example (24a) in ethanol (8 mL) was added hydrazine monohydrate (1 mL, 16 mmol), and the mixture was stirred at 100°C for 2.5 h. Water was added, and the mixture was extracted with ethyl acetate, washed with water and saturated brine, and then dried with anhydrous sodium sulfate. The filtrate was concentrated under reduced pressure, and then the resulting solid was collected by filtration and washed with diisopropyl ether to obtain the title compound as a white solid (273 mg, 87%).
¹H NMR spectrum (400 MHz, CDCl₃) δppm: 4.05 (3H, s), 4.19(2H, br, NH₂), 7.12 (1H, d, J=1.5 Hz), 7.25-7.30 (1H, m), 7.38-7.42 (2H, m), 7.47-7.51 (1H, m), 7.58 (1H, s), 8.28 (1H, d, J=7.8 Hz), 8.94 (1H, br, NH).

### (24c) 8-({[t-Butyl(dimethyl)silyl]oxy}methyl)-3-(3'-chloro-3-methoxybiphenyl-4-yl)-8-methyl-5,6,8,9-tetrahydro[1,2,4]triazolo[4,3-d][1,4]thiazepine

The title compound was obtained as a colorless oily substance (234 mg, 85%) in the same manner as in Example (1h) using 7-({[t-butyl(dimethyl)silyl]oxy}methyl)-7-methyl-5-methylthio-2,3,6,7-tetrahydro-1,4-thiazepine (176 mg, 0.55 mmol) obtained in Example (1d) and 3'-chloro-3-methoxybiphenyl-4-carboxylic acid hydrazide (144 mg, 0.52 mmol) obtained in Example (24b).
¹H NMR spectrum (400 MHz, CDCl₃) δppm: 0.08 (3H, s), 0.10 (3H, s), 0.93 (9H, s), 1.28 (3H, s), 2.90-2.96 (2H, m), 3.44-3.52 (2H, m), 3.60 (1H, d, J=10.0 Hz), 3.64 (1H, d, J=10.0 Hz), 3.90 (3H, s), 4.09-4.21 (2H, m), 7.13 (1H, s), 7.22-7.30 (1H, m), 7.38-7.43 (2H, m), 7.48-7.51 (1H, m), 7.59-7.61 (2H, m).

### (24d) [3-(3'-Chloro-3-methoxybiphenyl-4-yl)-8-methyl-5,6,8,9-tetrahydro[1,2,4]triazolo[4,3-d][1,4]thiazepin-8-yl]methanol

The title compound was obtained as a white solid (130 mg, 71%) in the same manner as in Example (1i) using 8-({[t-butyl(dimethyl)silyl]oxy}methyl)-3-(3'-chloro-3-methoxybiphenyl-4-yl)-8-methyl-5,6,8,9-tetrahydro[1,2,4]triazolo[4,3-d][1,4]thiazepine (234 mg, 0.44 mmol) obtained in Example (24c) and 4 N hydrogen chloride/dioxane (5 mL).
¹H NMR spectrum (400 MHz, CDCl₃) δppm: 1.41 (3H, s), 2.36 (1H, br, OH), 2.60-2.70 (1H, m), 2.96-3.04 (1H, m), 3.40-3.52 (3H, m), 3.70 (1H, d, J=15.0 Hz), 3.90 (3H, s), 4.15-4.26 (2H, m), 7.14 (1H, s), 7.23-7.30 (1H, m), 7.36-7.43 (2H, m), 7.47-7.50 (1H, m), 7.55-7.62 (2H, m).
Mass spectrum (FAB): m/z 416 (M+H)⁺
Melting point: 173-176°C.

### (Example 25) 4'-[8-(Hydroxymethyl)-8-methyl-5,6,8,9-tetrahydro[1,2,4]triazolo[4,3-d][1,4]thiazepin-3-yl]-3'-methoxybiphenyl-3-carbonitrile (Example Compound No. 1-103)

### (25a) Methyl 3'-cyano-3-methoxybiphenyl-4-carboxylate

1.10 g of unpurified trifluoromethanesulfonate ester was obtained in the same manner as in Example (10a) using methyl 4-hydroxy-2-methoxybenzoate (646 mg, 3.5 mmol), triethylamine (1.2 mL, 8.7 mmol), and trifluoromethanesulfonic anhydride (1.13 g, 4.0 mmol). The title compound was obtained as a white solid (240 mg, 81%) in the same manner as in Example (10a) using 348 mg of this unpurified product, (3-cyanophenyl)boronic acid (192 mg, 1.3 mmol), sodium carbonate (212 mg, 2.0 mmol), and tetrakis(triphenylphosphine)palladium(0) (120 mg, 0.10 mmol).
¹H NMR spectrum (400 MHz, CDCl₃) δppm: 3.93 (3H, s), 4.00 (3H, s), 7.12 (1H, s), 7.17 (1H, d, J=9.0 Hz), 7.59 (1H, t, J=7.8 Hz), 7.69 (1H, d, J=7.8 Hz), 7.83 (1H, d, J=7.8 Hz), 7.90 (1H, d, J=9.0 Hz), 7.93 (1H, s).

### (25b) 3'-Cyano-3-methoxybiphenyl-4-carboxylic acid

The title compound was obtained as a white solid (195 mg, 86%) in the same manner as in Example (10b) using methyl 3'-cyano-3-methoxybiphenyl-4-carboxylate (240 mg, 0.90 mmol) obtained in Example (25a) and sodium hydroxide (200 mg, 5.0 mmol).
¹H NMR spectrum (400 MHz, CDCl₃) δppm: 4.18 (3H, s), 7.20 (1H, s), 7.26-7.30 (1H, m), 7.58-7.80 (3H, m), 7.89 (1H, s), 8.29 (1H, d, J=8.6 Hz).

### (25c) t-Butyl 2-[(3'-cyano-3-methoxybiphenyl-4-yl)carbonyl]hydrazinecarboxylate

The title compound was obtained as a white solid (168 mg, 60%) in the same manner as in Example (1e) using 3'-cyano-3-methoxybiphenyl-4-carboxylic acid (195 mg, 0.77 mmol) obtained in Example (25b), oxalyl chloride (0.2 mL, 2.3 mmol), N,N-dimethylformamide (catalytic amount), t-butyl carbazate (150 mg, 1.1 mmol), and triethylamine (0.5 mL, 3.6 mmol).
¹H NMR spectrum (400 MHz, CDCl₃) δppm: 1.52 (9H, s), 4.10 (3H, s), 6.98 (1H, br, NH), 7.14 (1H, s), 7.20-7.30 (1H, m), 7.59 (1H, t, J=8.0 Hz), 7.70 (1H, d, J=8.0 Hz), 7.84 (1H, d, J=8.0 Hz), 7.89 (1H, s), 8.31 (1H, d, J=7.8 Hz), 9.5 (1H, br, NH).

### (25d) 3'-Cyano-3-methoxybiphenyl-4-carboxylic acid hydrazide

The title compound was obtained as a white solid (106 mg, 88%) in the same manner as in Example (1g) using t-butyl 2-[(3'-cyano-3-methoxybiphenyl-4-yl)carbonyl]hydrazinecarboxylate (166 mg, 0.45 mmol) obtained in Example (25c) and 4 N hydrogen chloride/ethyl acetate (10 mL).
¹H NMR spectrum (400 MHz, CDCl₃) δppm: 4.06 (3H, s), 4.21 (2H, br, NH₂), 7.12 (1H, s), 7.21-7.3 (1H, m), 7.60 (1H, t, J=8.0 Hz), 7.69 (1H, d, J=8.0 Hz), 7.80 (1H, d, J=8.0 Hz), 7.89 (1H, s), 8.31 (1H, d, J=8.2 Hz), 8.95 (1H, br, NH).

### (25e) 4'-[8-({[t-Butyl(dimethyl)silyl]oxy}methyl)-8-methyl-5,6,8,9-tetrahydro[1,2,4]triazolo[4,3-d][1,4]thiazepin-3-yl]-3'-methoxybiphenyl-3-carbonitrile

The title compound was obtained as a yellow foamy substance (141 mg, 68%) in the same manner as in Example (1h) using 7-({[t-butyl(dimethyl)silyl]oxy}methyl)-7-methyl-5-methylthio-2,3,6,7-tetrahydro-1,4-thiazepine (154 mg, 0.48 mmol) obtained in Example (1d) and 3'-cyano-3-methoxybiphenyl-4-carboxylic acid hydrazide (106 mg, 0.40 mmol) obtained in Example (25d).
¹H NMR spectrum (400 MHz, CDCl₃) δppm: 0.08 (3H, s), 0.10 (3H, s), 0.94 (9H, s), 1.28 (3H, s), 2.91-2.97 (2H, m), 3.49 (1H, d, J=15.2 Hz), 3.55 (1H, d, J=15.2 Hz), 3.61 (1H, d, J=10.0 Hz), 3.65 (1H, d, J=10.0 Hz), 3.92 (3H, s), 4.12-4.21 (2H, m), 7.14 (1H, d, J=1.5 Hz), 7.25-7.30 (1H, m), 7.58-7.71 (3H, m), 7.85 (1H, d, J=8.2 Hz), 7.90 (1H, s).

### (25f) 4'-[8-(Hydroxymethyl)-8-methyl-5,6,8,9-tetrahydro[1,2,4]triazolo[4,3-d] [1,4]thiazepin-3-yl]-3'-methoxybiphenyl-3-carbonitrile

The title compound was obtained as a white solid (69.0 mg, 63%) in the same manner as in Example (li) using 4'-[8-({[t-butyl(dimethyl)silyl]oxy}methyl)-8-methyl-5,6,8,9-tetrahydro[1,2,4]triazolo[4,3-d][1,4]thiazepin-3-yl]-3'-methoxybiphenyl-3-carbonitrile (140 mg, 0.27 mmol) obtained in Example (25e) and 4 N hydrogen chloride/dioxane (5 mL).
¹H NMR spectrum (400 MHz, CDCl₃) δppm: 1.41 (3H, s), 2.36 (1H, br, OH), 2.70 (1H, dd, J=6.6, 15.6 Hz), 3.02-3.06 (1H, m), 3.44 (1H, d, J=15.0 Hz), 3.45-3.48 (2H, m), 3.73 (1H, d, J=15.0 Hz), 4.15-4.25 (2H, m), 7.14 (1H, s), 7.23-7.30 (1H, m), 7.58-7.63 (2H, m), 7.71 (1H, d, J=7.4 Hz), 7.85 (1H, d, J=7.8 Hz), 7.90 (1H, s).
Mass spectrum (FAB): m/z 407 (M+H)⁺
Melting point: 232-236°C.

### (Example 26) [3-(3-Chloro-3',4'-difluorobiphenyl-4-yl)-8-methyl-5,6,8,9-tetrahydro[1,2,4]triazolo[4,3-d][1,4]thiazepin-8-yl]methanol (Example Compound No. 1-114)

### (26a) 8-({[t-Butyl(dimethyl)silyl]oxy}methyl)-3-(3-chloro-3',4'-difluorobiphenyl-4-yl)-8-methyl-5,6,8,9-tetrahydro[1,2,4]triazolo[4,3-d][1,4]thiazepine

The title compound (165 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (3, 4-difluorophenyl)boronic acid (58.4 mg, 0.370 mmol).
¹H NMR spectrum (400 MHz, CDCl₃) δppm: 0.08 (3H, s), 0.09 (3H, s), 0.93 (9H, s), 1.29 (3H, s), 2.95 (2H, brs), 3.51 (1H, d, J=15.0 Hz), 3.57 (1H, d, J=15.0 Hz), 3.60 (1H, d, J=11.0 Hz), 3.64 (1H, d, J=11.0 Hz), 4.16-4.18 (2H, m), 7.31-7.69 (6H, m).

### (26b) [3-(3-Chloro-3',4'-difluorobiphenyl-4-yl)-8-methyl-5,6,8,9-tetrahydro[1,2,4]triazolo[4,3-d][1,4]thiazepin-8-yl]methanol

The title compound was obtained as a white powder (88.4 mg, yield 68%) in the same manner as in Example (9c) using 8-({[t-butyl(dimethyl)silyl]oxy}methyl)-3-(3-chloro-3',4'-difluorobiphenyl-4-yl)-8-methyl-5,6,8,9-tetrahydro[1,2,4]triazolo[4,3-d][1,4]thiazepine (165 mg, 0.308 mmol) obtained in Example (26a).
¹H NMR spectrum (400 MHz, DMSO-d₆) δppm: 1.16 (3H, s), 2.86-3.02 (2H, brs), 3.27-3.43 (4H, m), 4.09 (2H, t, J=5.1 Hz), 5.15 (1H, t, J=5.1 Hz), 7.57 (1H, t, J=8.6 Hz), 7.63 (1H, d, J=7.8 Hz), 7.68-7.72 (1H, m), 7.86 (1H, dd, J=1.6, 7.8 Hz), 7.95-8.00 (1H, m), 8.04 (1H, d, J=2.0 Hz).
Mass spectrum (FAB): 422 (M+H)⁺
Melting point: 231-234°C.

### (Example 27) [3-(3-Chloro-3',5'-difluorobiphenyl-4-yl)-8-methyl-5,6,8,9-tetrahydro[1,2,4]triazolo[4,3-d][1,4]thiazepin-8-yl]methanol (Example Compound No.1-115)

### (27a) 8-({[t-Butyl(dimethyl)silyl]oxy}methyl)-3-(3-chloro-3',5'-difluorobiphenyl-4-yl)-8-methyl-5,6,8,9-tetrahydro[1,2,4]triazolo[4,3-d][1,4]thiazepine

The title compound (166 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (156 mg, 0.310 mmol) obtained in Example (9a) and (3,5-difluorophenyl)boronic acid (58.7 mg, 0.372 mmol).
¹H NMR spectrum (400 MHz, CDCl₃) δppm: 0.08 (3H, s), 0.09 (3H, s), 0.93 (9H, s), 1.29 (3H, s), 2.95 (2H, brs), 3.54 (1H, d, J=5.1 Hz), 3.62 (1H, d, J=6.7 Hz), 3.55-3.65 (2H, m), 4.15-4.18 (2H, m), 7.12-7.69 (6H, m).

### (27b) [3-(3-Chloro-3',5'-difluorobiphenyl-4-yl)-8-methyl-5,6,8,9-tetrahydro[1,2,4]triazolo[4,3-d][1,4]thiazepin-8-yl]methanol

The title compound was obtained as a light yellow powder (94.2 mg, yield 72%) in the same manner as in Example (9c) using 8-({[t-butyl(dimethyl)silyl]oxy}methyl)-3-(3-chloro-3',5'-difluorobiphenyl-4-yl)-8-methyl-5,6,8,9-tetrahydro[1,2,4]triazolo[4,3-d][1,4]thiazepine (166 mg, 0.310 mmol) obtained in Example (27a).
¹H NMR spectrum (400 MHz, DMSO-d₆) δppm: 1.16 (3H, s), 2.89-3.01 (2H, brs), 3.27-3.44 (4H, m), 4.08-4.11 (2H, m), 5.16 (1H, t, J=5.5 Hz), 7.34 (1H, tt, J=2.3, 9.8 Hz), 7.63-7.66 (3H, m), 7.92 (1H, dd, J=1.2, 9.0 Hz), 8.11 (1H, d, J=1.6 Hz).
Mass spectrum (ESI): 422.0898 (M+H)⁺
Melting point: 260-265°C.

### (Example 28) [3-(3,3'-Dichloro-2'-fluorobiphenyl-4-yl)-8-methyl-5,6,8,9-tetrahydro[1,2,4]triazolo[4,3-d][1,4]thiazepin-8-yl]methanol (Example Compound No. 1-116)

### (28a) 8-({[t-Butyl(dimethyl)silyl]oxy}methyl)-3-(3,3'-dichloro-2'-fluorobiphenyl-4-yl)-8-methyl-5,6,8,9-tetrahydro[1,2,4]triazolo[4,3-d][1,4]thiazepine

The title compound (170 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (3-chloro-2-fluorophenyl)boronic acid (64.5 mg, 0.370 mmol).
¹H NMR spectrum (400 MHz, CDCl₃) δppm: 0.08 (3H, s), 0.10 (3H, s), 0.93 (9H, s), 1.29 (3H, s), 2.97 (2H, brs), 3.51 (1H, d, J=16.0 Hz), 3.57 (1H, d, J=16.0 Hz), 3.61 1 (1H, d, J=9.8 Hz), 3.65 (1H, d, J=9.8 Hz), 4.18-4.22 (2H, m), 7.19-7.70 (6H, m).

### (28b) [3-(3,3'-Dichloro-2'-fluorobiphenyl-4-yl)-8-methyl-5,6,8,9-tetrahydro[,2,4]triazolo[4,3-d][1,4]thiazepin-8-yl]methanol

The title compound was obtained as a white powder (75.2 mg, yield 56%) in the same manner as in Example (9c) using 8-({[t-butyl(dimethyl)silyl]oxy}methyl)-3-(3,3'-dichloro-2'-fluorobiphenyl-4-yl)-8-methyl-5,6,8,9-tetrahydro[1,2,4]triazolo[4,3-d][1,4]thiazepine (170 mg, 0.308 mmol) obtained in Example (28a).
¹H NMR spectrum (400 MHz, CDCl₃) δppm: 1.41 (3H, s), 2.31 (1H, t, J=6.3 Hz), 2.68-2.74 (1H, m), 3.04 (1H, brs), 3.47 (1H, d, J=15.3 Hz), 3.51 (2H, d, J=6.7 Hz), 3.74 (1H, d, J=15.3 Hz), 4.24-4.26 (2H, m), 7.21 (1H, dt, J=1.2, 7.8 Hz), 7.32-7.36 (1H, m), 7.45-7.49 (1H, m), 7.58-7.59 (2H, m), 7.70 (1H, s).
Mass spectrum (ESI): 438.0981 (M+H)⁺
Melting point: 156-160°C.

### (Example 29) [3-(3,5'-Dichloro-2'-fluorobiphenyl-4-yl)-8-methyl-5,6,8,9-tetrahydro[1,2,4]triazolo[4,3-d][1,4]thiazepin-8-yl]methanol (Example Compound No. 1-117)

### (29a) 8-({[t-Butyl(dimethyl)silyl]oxy}methyl)-3-(3,5'-dichloro-2'-fluorobiphenyl-4-yl)-8-methyl-5,6,8,9-tetrahydro[1,2,4]triazolo[4,3-d] [1,4]thiazepine

The title compound (170 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (5-chloro-2-fluorophenyl)boronic acid (64.5 mg, 0.370 mmol).
¹H NMR spectrum (400 MHz, CDCl₃) δppm: 0.08 (3H, s), 0.10 (3H, s), 0.93 (9H, s), 1.29 (3H, s), 2.95 (2H, brs), 3.51 (1H, d, J=16.5 Hz), 3.57 (1H, d, J=16.5 Hz), 3.60 (1H, d, J=11.0 Hz), 3.64 (1H, d, J=11.0 Hz), 4.17-4.20 (2H, m), 7.13-7.69 (6H, m).

### (29b) [3-(3,5'-Dichloro-2'-fluorobiphenyl-4-yl)-8-methyl-5,6,8,9-tetrahydro[1,2,4]triazolo[,3-d] [1,4]thiazepin-8-yl]methanol

The title compound was obtained as a white powder (89.3 mg, yield 66%) in the same manner as in Example (9c) using 8-({[t-butyl(dimethyl)silyl]oxy}methyl)-3-(3,5'-dichloro-2'-fluorobiphenyl-4-yl)-8-methyl-5,6,8,9-tetrahydro[1,2,4]triazolo[4,3-d][1,4]thiazepine (170 mg, 0.308 mmol) obtained in Example (29a).
¹H NMR spectrum (400 MHz, CDCl₃) δppm: 1.41 (3H, s), 2.30 (1H, t, J=7.4 Hz), 2.68-2.72 (1H, m), 3.04 (1H, brs), 3.46 (1H, d, J=15.3 Hz), 3.51 (2H, d, J=8.2 Hz), 3.74 (1H, d, J=15.3 Hz), 4.23-4.25 (2H, m), 7.15 (1H, t, J=8.6 Hz), 7.34-7.38 (1H, m), 7.45 (1H, dd, J=2.4, 6.7 Hz), 7.58-7.59 (2H, m), 7.70 (1H, s).
Mass spectrum (ESI): 438.0799 (M+H)⁺
Melting point: 186-189°C.

### (Example 30) [3-(3,3'-Dichloro-4'-fluorobiphenyl-4-yl)-8-methyl-5,6,8,9-tetrahydro[1,2,4]triazolo[4,3-d][1,4]thiazepin-8-yl]methanol (Example Compound No. 1-118)

### (30a) 8-({[t-Butyl(dimethyl)silyl]oxy}methyl)-3-(3,3'-dichloro-4'-fluorobiphenyl-4-yl)-8-methyl-5,6,8,9-tetrahydro[1,2,4]triazolo[4,3-d] [1,4]thiazepine

The title compound (167 mg, yield 90%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (168 mg, 0.334 mmol) obtained in Example (9a) and (3-chloro-4-fluorophenyl)boronic acid (69.9 mg, 0.401 mmol).
¹H NMR spectrum (400 MHz, CDCl₃) δppm: 0.08 (3H, s), 0.10 (3H, s), 0.93 (9H, s), 1.29 (3H, s), 2.95 (2H, brs), 3.51 (1H, d, J=15.0 Hz), 3.56 (1H, d, J=15.0 Hz), 3.60 (1H, d, J=10.0 Hz), 3.65 (1H, d, J=10.0 Hz), 4.17-4.19 (2H, m), 7.46-7.70 (6H, m).

### (30b) [3-(3,3'-Dichloro-4'-fluorobiphenyl-4-yl)-8-methyl-5,6,8,9-tetrahydro[11,2,4]triazolo[4,3-d][1,4]thiazepin-8-yl]methanol

The title compound was obtained as a white powder (75.1 mg, yield 57%) in the same manner as in Example (9c) using 8-({[t-butyl(dimethyl)silyl]oxy}methyl)-3-(3,3'-dichloro-4'-fluorobiphenyl-4-yl)-8-methyl-5,6,8,9-tetrahydro[1,2,4]triazolo[4,3-d][1,4]thiazepine (165 mg, 0.299 mmol) obtained in Example (30a).
¹H NMR spectrum (400 MHz, CDCl₃) δppm: 1.41 (3H, s), 2.29 (1H, t, J=6.7 Hz), 2.67-2.73 (1H, m), 3.04 (1H, brs), 3.46 (1H, d, J=15.3 Hz), 3.51 (2H, d, J=7.0 Hz), 3.74 (1H, d, J=15.3 Hz), 4.21-4.24 (2H, m), 7.24-7.29 (1H, m), 7.45-7.49 (1H, m), 7.55-7.60 (2H, m), 7.65 (1H, dd, J=2.3, 7.0 Hz), 7.69 (1H, d, J=1.6 Hz).
Mass spectrum (ESI): 438.0593 (M+H)⁺
Melting point: 235-239°C.

### (Example 31) [3-(3',4'-Difluoro-3-methoxybiphenyl-4-yl)-8-methyl-5,6,8,9-tetrahydro[1,2,4]triazolo[4,3-d][1,4]thiazepin-8-yl]methanol (Example Compound No. 1-119)

### (31a) Methyl 3',4'-difluoro-3-methoxybiphenyl-4-carboxylate

The title compound was obtained as a white solid (399 mg, 83%) in the same manner as in Example (10a) using methyl 4-hydroxy-2-methoxybenzoate (316 mg, 1.7 mmol), triethylamine (0.6 mL, 4.3 mmol), trifluoromethanesulfonic anhydride (620 mg, 2.2 mmol), (3,4-difluorophenyl)boronic acid (320 mg, 2.0 mmol), sodium carbonate (320 mg, 3.0 mmol), and tetrakis(triphenylphosphine)palladium(0) (203 mg, 0.17 mmol).
¹H NMR spectrum (400 MHz, CDCl₃) δppm: 3.92(3H, s), 3.98 (3H, s), 7.08 (1H, s), 7.13 (1H, d, J=7.8 Hz), 7.22-7.43 (3H, m), 7.88 (1H, d, J=7.8 Hz).

### (31b) 3',4'-Difluoro-3-methoxybiphenyl-4-carboxylic acid

The title compound was obtained as a white solid (386 mg, 97%) in the same manner as in Example (10b) using methyl 3',4'-difluoro-3-methoxybiphenyl-4-carboxylate (398 mg, 1.4 mmol) obtained in Example (31a) and sodium hydroxide (280 mg, 7.0 mmol).
¹H NMR spectrum (400 MHz, CDCl₃) δppm: 4.16 (3H, s), 7.16 (1H, s), 7.25-7.45 (4H, m), 8.25 (1H, d, J=8.2 Hz), 10.67 (1H, br, OH).

### (31c) t-Butyl 2-[(3',4'-difluoro-3-methoxybiphenyl-4-yl)carbonyl]hydrazinecarboxylate

The title compound was obtained as a white solid (470 mg, 89%) in the same manner as in Example (1e) using 3',4'-difluoro-3-methoxybiphenyl-4-carboxylic acid (368 mg, 0.14 mmol) obtained in Example (31b), oxalyl chloride (0.3 mL, 3.5 mmol), N,N-dimethylformamide (catalytic amount), t-butyl carbazate (290 mg, 2.2 mmol), and triethylamine (0.8 mL, 5.8 mmol).
¹H NMR spectrum (400 MHz, CDCl₃) δppm: 1.52 (9H, s), 4.08 (3H, s), 6.93(1H, br, NH), 7.09 (1H, s), 7.23-7.35 (3H, m), 7.39-7.44 (1H, m), 8.27 (1H, d, J=8.2 Hz), 9.55 (1H, br, NH).

### (31d) 3',4'-Difluoro-3-methoxybiphenyl-4-carboxylic acid hydrazide

The title compound was obtained as a white solid (329 mg, 95%) in the same manner as in Example (1g) using t-butyl 2-[(3',4'-difluoro-3-methoxybiphenyl-4-yl)carbonyl]hydrazinecarboxylate (470 mg, 1.2 mmol) obtained in Example (31c) and 4 N hydrogen chloride/ethyl acetate (10 mL).
¹H NMR spectrum (400 MHz, CDCl₃) δppm: 4.04 (3H, s), 4.19(2H, br, NH₂), 7.08 (1H, s), 7.22-7.35 (3H, m), 7.39-7.44 (1H, m), 8.27 (1H, d, J=8.3 Hz), 8.94 (1H, br, NH).

### (31e) 8-({[t-Butyl(dimethyl)silyl]oxy}methyl)-3-(3',4'-difluoro-3-methoxybiphenyl-4-yl)-8-methyl-5,6,8,9-tetrahydro[1,2,4]triazolo[4,3-d] [1,4]thiazepine

The title compound was obtained as a colorless oily substance (189 mg, 74%) in the same manner as in Example (1h) using 7-({[t-butyl(dimethyl)silyl]oxy}methyl)-7-methyl-5-methylthio-2,3,6,7-tetrahydro-1,4-thiazepine (165 mg, 0.52 mmol) obtained in Example (1d) and 3',4'-difluoro-3-methoxybiphenyl-4-carboxylic acid hydrazide (133 mg, 0.48 mmol) obtained in Example (31d).
¹H NMR spectrum (400 MHz, CDCl₃) δppm: 0.08 (3H, s), 0.09 (3H, s), 0.93 (9H, s), 1.28 (3H, s), 2.89-2.96 (2H, m), 3.50-3.57 (2H, m), 3.62 (1H, d, J=16.0 Hz), 3.63 (1H, d, J=16.0 Hz), 3.89 (3H, s), 4.10-4.19 (2H, m), 7.09 (1H, s), 7.22-7.43 (4H, m), 7.59 (1H, d, J=7.9 Hz).

### (31f) [3-(3',4'-Difluoro-3-methoxybiphenyl-4-yl)-8-methyl-5,6,8,9-tetrahydro[1,2,4]triazolo[4,3-d][1,4]thiazepin-8-yl]methanol

The title compound was obtained as a white solid (79.8 mg, 54%) in the same manner as in Example (li) using 8-({[t-butyl(dimethyl)silyl]oxy}methyl)-3-(3',4'-difluoro-3-methoxybiphenyl-4-yl)-8-methyl-5,6,8,9-tetrahydro[1,2,4]triazolo[4,3-d][1,4]thiazepine (189 mg, 0.36 mmol) obtained in Example (31e) and 4 N hydrogen chloride/dioxane (5 mL).
¹H NMR spectrum (400 MHz, CDCl₃) δppm: 1.41 (3H, s), 2.40 (1H, br, OH), 2.62-2.70 (1H, m), 2.96-3.04 (1H, m), 3.42 (1H, d, J=15.0 Hz), 3.42-3.54 (2H, m), 3.71 (1H, d, J=15.0 Hz), 3.90 (3H, s), 4.18-4.28 (2H, m), 7.10 (1H, s), 7.23-7.49 (4H, m), 7.57 (1H, d, J=7.4 Hz).
Mass spectrum (FAB): m/z 418 (M+H)⁺
Melting point: 183-185°C.

### (Example 32) [3-(3'-Chloro-4'-fluoro-3-methoxybiphenyl-4-yl)-8-methyl-5,6,8,9-tetrahydro[1,2,4]triazolo[4,3-d][1,4]thiazepin-8-yl]methanol (Example Compound No. 1-123)

### (32a) Methyl 3'-chloro-4'-fluoro-3-methoxybiphenyl-4-carboxylate

The title compound was obtained as a colorless oily substance (419 mg, 42%) in the same manner as in Example (10a) using methyl 4-hydroxy-2-methoxybenzoate (401 mg, 2.2 mmol), triethylamine (0.7 mL, 5.0 mmol), trifluoromethanesulfonic anhydride (840 mg, 3.0 mmol), (3-chloro-4-fluorophenyl)boronic acid (202 mg, 1.2 mmol), sodium carbonate (202 mg, 1.9 mmol), and tetrakis(triphenylphosphine)palladium(0) (160 mg, 0.14 mmol). ¹H NMR spectrum (400 MHz, CDCl₃) δppm: 3.91 (3H, s), 3.98 (3H, s), 7.07 (1H, s), 7.11 (1H, d, J=7.8 Hz), 7.21-7.26 (1H, m), 7.41-7.47 (1H, m), 7.61-7.65 (1H, m), 7.88 (1H, d, J=7.8 Hz).

### (32b) 3'-Chloro-4'-fluoro-3-methoxybiphenyl-4-carboxylic acid hydrazide

The title compound was obtained as a white solid (240 mg, 87%) in the same manner as in Example (7b) using methyl 3'-chloro-4'-fluoro-3-methoxybiphenyl-4-carboxylate (277 mg, 0.94 mmol) obtained in Example (32a) and hydrazine monohydrate (mL, 16 mmol).
¹H NMR spectrum (400 MHz, CDCl₃) δppm: 4.05 (3H, s), 4.21(2H, br, NH₂), 7.08(1H, s), 7.21-7.26 (2H, m), 7.40-7.48 (1H, m), 7.62-7.65 (1H, m), 8.27 (1H, d, J=8.2 Hz), 8.93 (1H, br, NH).

### (32c) 8-({[t-Butyl(dimethyl)silyl]oxy}methyl)-3-(3'-chloro-4'-fluoro-3-methoxybiphenyl-4-yl)-8-methyl-5,6,8,9-tetrahydro[1,2,4]triazolo[4,3-d][1,4]thiazepine

The title compound was obtained as a colorless oily substance (264 mg, 87%) in the same manner as in Example (1h) using 7-({[t-butyl(dimethyl)silyl]oxy}methyl)-7-methyl-5-methylthio-2,3,6,7-tetrahydro₋1,4-thiazepine (184 mg, 0.58 mmol) obtained in Example (1d) and 3'-chloro-4'-fluoro-3-methoxybiphenyl-4-carboxylic acid hydrazide (164 mg, 0.56 mmol) obtained in Example (32b).
¹H NMR spectrum (400 MHz, CDCl₃) δppm: 0.08 (3H, s), 0.10 (3H, s), 0.93 (9H, s), 1.28 (3H, s), 2.87-2.94 (2H, m), 3.46-3.56 (2H, m),3.60 (1H, d, J=10.2 Hz), 3.65 (1H, d, J=10.2 Hz), 3.90 (3H, s), 4.05-4.20 (2H, m), 7.10(1H, s), 7.22-7.30 (1H, m), 7.38-7.42 (1H, m), 7.46-7.49 (1H, m), 7.59-7.65 (2H, m).

### (32d) [3-(3'-Chloro-4'-fluoro-3-methoxybiphenyl-4-yl)-8-methyl-5,6,8,9-tetrahydro[1,2,4]triazolo[4,3-d][1,4]thiazepin-8-yl]methanol

The title compound was obtained as a white solid (130 mg, 62%) in the same manner as in Example (li) using 8-({[t-butyl(dimethyl)silyl]oxy}methyl)-3-(3'-chloro-4'-fluoro-3-methoxybiphenyl-4-yl)-8-methyl-5,6,8,9-tetrahydro[1,2,4]triazolo[4,3-d][1,4]thiazepine (264 mg, 0.48 mmol) obtained in Example (32c) and 4 N hydrogen chloride/dioxane (5 mL).
¹H NMR spectrum (400 MHz, CDCl₃) δppm: 1.41 (3H, s), 2.20-2.30 (1H, m, OH), 2.64-2.73 (1H, m), 2.95-3.05 (1H, m), 3.43 (1H, d, J=15.0 Hz), 3.43-3.52 (2H, m), 3.70 (1H, d, J=15.0 Hz), 3.90 (3H, s), 4.12-4.24 (2H, m), 7.10 (1H, s), 7.23-7.30 (1H, m), 7.40-7.52 (2H, m), 7.58 (1H, d, J=7.5 Hz), 7.59-7.62 (1H, m).
Mass spectrum (FAB): m/z 434 (M+H)⁺
Melting point: 238-241°C.

### (Example 33) [3-(2-Chloro-4'-fluorobiphenyl-3-yl)-8-methyl-5,6,8,9-tetrahydro[1,2,4]triazolo[4,3-d][1,4]thiazepin-8-yl]methanol (Example Compound No. 1-125)

### (33a) t-Butyl 2-[(3-bromo-2-chlorophenyl)carbonyl]hydrazinecarboxylate

The title compound was obtained as a white solid (335 mg, 47%) in the same manner as in Example (1e) using 3-bromo-2-chlorobenzoic acid (J. Org. Chem. 2003, 68, 2030-2033.) (480 mg, 2.0 mmol), oxalyl chloride (0.5 mL, 5.7 mmol), N,N-dimethylformamide (catalytic amount), t-butyl carbazate (430 mg, 3.3 mmol), and triethylamine (0.8 mL, 5.6 mmol).
¹H NMR spectrum (400 MHz, CDCl₃) δppm: 1.51 (9H, s), 6.73 (1H, br, NH), 7.21 (1H, t, J=8.0 Hz), 7.58-7.62 (1H, m), 7.68 (1H, br, NH), 7.75 (1H, dd, J=1.5, 8.0 Hz).

### (33b) 3-Bromo-2-chlorobenzoic acid hydrazide

The title compound was obtained as a white solid (195 mg, 83%) in the same manner as in Example (1g) using t-butyl 2-[(3-bromo-2-chlorophenyl)carbonyl]hydrazinecarboxylate (330 mg, 0.94 mmol) obtained in Example (33a) and 4 N hydrogen chloride/ethyl acetate (10 mL).
¹H NMR spectrum (400 MHz, CDCl₃) δppm: 4.13 (2H, br, NH₂), 7.21 (1H, t, J=8.0 Hz), 7.49 (1H, dd, J=1.6, 8.0 Hz), 7.74 (1H, dd, J=1.6, 8.0 Hz).

### (33c) 3-(3-Bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine

The title compound was obtained as a white foamy substance (270 mg, 91 %) in the same manner as in Example (1h) using 7-({[t-butyl(dimethyl)silyl]oxy}methyl)-7-methyl-5-methylthio-2,3,6,7-tetrahydro-1,4-thiazepine (202 mg, 0.63 mmol) obtained in Example (1d) and 3-bromo-2-chlorobenzoic acid hydrazide (147 mg, 0.59 mmol) obtained in Example (33b).
¹H NMR spectrum (400 MHz, CDCl₃) δppm: 0.07 (3H, s), 0.09 (3H, s), 0.93 (9H, s), 1.28 (3H, s), 2.93-2.97 (2H, m), 3.48-3.58 (2H, m), 3.60 (1H, d, J=16.5 Hz), 3.62 (1H, d, J=16.5 Hz), 4.09-4.14 (2H, m), 7.25-7.32 (1H, m), 7.29 (1H, d, J=8.0 Hz), 7.82 (1H, dd, J=1.6, 8.0 Hz).

### (33d) 8-({[t-Butyl(dimethyl)silyl]oxy}methyl)-3-(2-chloro-4'-fluorobiphenyl-3-yl)-8-methyl-5,6,8,9-tetrahydro[1,2,4]triazolo[4,3-d][1,4]thiazepine

The title compound was obtained as a white foamy substance (180 mg, 100%) in the same manner as in Example (1f) using 3-(3-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (143 mg, 40.28 mmol) obtained in Example (33c), (4-fluorophenyl)boronic acid (47.0 mg, 0.34 mmol), sodium carbonate (76.0 g, 0.72 mmol), and tetrakis(triphenylphosphine)palladium(0) (67.0 mg, 0.058 mmol).
¹H NMR spectrum (400 MHz, CDCl₃) δppm: 0.07 (3H, s), 0.09 (3H, s), 0.93 (9H, s), 1.28 (3H, s), 2.82-3.01 (2H, m), 3.42-3.55 (2H, m), 3.60 (1H, d, J=17.0 Hz), 3.62 (1H, d, J=17.0 Hz), 4.09-4.21 (2H, m), 7.15 (2H, t, J=8.6 Hz), 7.53-7.58 (3H, m), 7.63-7.72 (2H, m).

### (33e) [3-(2-Chloro-4'-fluorobiphenyl-3-yl)-8-methyl-5,6,8,9-tetrahydro[1,2,4]triazolo[4,3-d] [1,4]thiazepin-8-yl]methanol

The title compound was obtained as a white solid (78.6 mg, 69%) in the same manner as in Example (li) using 8-({[t-butyl(dimethyl)silyl]oxy}methyl)-3-(2-chloro-4'-fluorobiphenyl-3-yl)-8-methyl-5,6,8,9-tetrahydro[1,2,4]triazolo[4,3-d][1,4]thiazepine (180 mg, 0.28 mmol) obtained in Example (33d) and 4 N hydrogen chloride/dioxane (5 mL).
¹H NMR spectrum (400 MHz, CDCl₃) δppm: 1.40 (3H, s), 2.28-2.36 (1H, m), 2.65-2.75 (1H, m), 3.45 (1H, d, J=15.0 Hz), 3.47-3.52 (2H, m), 3.73 (1H, d, J=15.0 Hz), 4.18-4.23 (2H, m), 7.15 (2H, t, J=8.6 Hz), 7.37-7.53 (5H, m).
Mass spectrum (EI): m/z 404 (M+H)⁺
Melting point: 192-195°C.

### (Example 34) [3-(4-Chloro-4'-fluorobiphenyl-3-yl)-8-methyl-5,6,8,9-tetrahydro[1,2,4]triazolo[4,3-d][1,4]thiazepin-8-yl]methanol (Example Compound No. 1-127)

### (34a) Ethyl 4-chloro-4'-fluorobiphenyl-3-carboxylate

To a solution of ethyl 5-bromo-2-chlorobenzoate (1.23 g, 4.7 mmol) in toluene (14 mL), ethanol (7 mL), and water (4 mL) were added (4-fluorophenyl)boronic acid (780 mg, 5.6 mmol), sodium carbonate (1.06 g, 10 mmol), and tetrakis(triphenylphosphine)palladium(0) (440 mg, 0.38 mmol), and the mixture was stirred at 100°C for 3 h. The mixture was extracted with ethyl acetate, then washed with water, saturated aqueous sodium hydrogencarbonate, and saturated brine, and then dried with anhydrous sodium sulfate. The filtrate was concentrated under reduced pressure, and then the residue was purified by silica gel column chromatography (elution solvent: hexane/ethyl acetate = 4/1) to obtain the title compound as a colorless oily substance (515 mg, 39%).
¹H NMR spectrum (400 MHz, CDCl₃) δppm: 1.43 (3H, t, J=7.0 Hz), 4.44 (2H, q, J=7.0 Hz) 7.15 (2H, t, J=8.6 Hz), 7.31-7.35 (1H, m), 7.47-7.58 (3H, m), 7.97 (1H, d, J=2.3 Hz).

### (34b) t-Butyl 2-[(4-chloro-4'-fluorobiphenyl-3-yl)carbonyl]hydrazinecarboxylate

To a solution of ethyl 4-chloro-4'-fluorobiphenyl-3-carboxylic acid (510 mg, 1.8 mmol) obtained in Example (34a) in ethanol (10 mL) were added water (2 mL) and sodium hydroxide (500 mg, 13 mmol), and the mixture was stirred at 100°C for 1 h. Dilute hydrochloric acid was added, and the mixture was concentrated under reduced pressure, then extracted with ethyl acetate, washed with water and saturated brine, and dried with anhydrous sodium sulfate. The filtrate was concentrated under reduced pressure, and then the resulting solid was collected by filtration and washed with hexane to obtain 4-chloro-4'-fluorobiphenyl-3-carboxylic acid as a white solid (385 mg, 84%).

The title compound was obtained as a colorless oily substance (483 mg, 87%) in the same manner as in Example (1e) using this 4-chloro-4'-fluorobiphenyl-3-carboxylic acid (380 mg, 1.5 mmol), oxalyl chloride (0.4 mL, 4.6 mmol), N,N-dimethylformamide (catalytic amount), t-butyl carbazate (290 mg, 2.2 mmol), and triethylamine (0.8 mL, 5.6 mmol).
¹H NMR spectrum (400 MHz, CDCl₃) δppm: 1.52 (9H, s), 7.14 (2H, t, J=8.6 Hz), 7.48-7.59 (4H, m), 7.92 (1H, s).

### (34c) 8-({[t-Butyl(dimethyl)silyl]oxy}methyl)-3-(4-chloro-4'-fluorobiphenyl-3-yl)-8-methyl-5,6,8,9-tetrahydro[1,2,4]triazolo[4,3-d][1,4]thiazepine

4-Chloro-4'-fluorobiphenyl-3-carboxylic acid hydrazide was obtained as a white solid (293 mg, 84%) in the same manner as in Example (1g) using t-butyl 2-[(4-chloro-4'-fluorobiphenyl-3-yl)carbonyl]hydrazinecarboxylate (483 mg, 1.3 mmol) obtained in Example (34b) and 4 N hydrogen chloride/ethyl acetate (10 mL).

The title compound was obtained as a white solid (198 mg, 78%) in the same manner as in Example (1h) using 7-({[t-butyl(dimethyl)silyl]oxy}methyl)-7-methyl-5-methylthio-2,3,6,7-tetrahydro-1,4-thiazepine (171 mg, 0.54 mmol) obtained in Example (1d) and 4-chloro-4'-fluorobiphenyl-3-carboxylic acid hydrazide (130 mg, 0.50 mmol) obtained above.
¹H NMR spectrum (400 MHz, CDCl₃) δppm: 0.08 (3H, s), 0.10 (3H, s), 0.93 (9H, s), 1.28 (3H, s), 2.93-2.97 (2H, m), 3.50-3.58 (2H, m), 3.61 (1H, d, J=16.5 Hz), 3.63 (1H, d, J=16.5 Hz), 4.15-4.20 (2H, m), 7.14 (2H, t, J=8.6 Hz), 7.52-7.58 (3H, m), 7.65 (1H, dd, J=2.0, 8.2 Hz), 7.68 (1H, d, J=2.0 Hz).

### (34d) [3-(4-Chloro-4'-fluorobiphenyl-3-yl)-8-methyl-5,6,8,9-tetrahydro[1,2,4]triazolo[4,3-d] [1,4]thiazepin-8-yl]methanol

The title compound was obtained as a white solid (134 mg, 87%) in the same manner as in Example (li) using 8-({[t-butyl(dimethyl)silyl]oxy}methyl)-3-(4-chloro-4'-fluorobiphenyl-3-yl)-8-methyl-5,6,8,9-tetrahydro[1,2,4]triazolo[4,3-d][1,4]thiazepine (197 mg, 0.38 mmol) obtained in Example (34c) and 4 N hydrogen chloride/dioxane (5 mL).
¹H NMR spectrum (400 MHz, CDCl₃) δppm: 1.41 (3H, s), 2.25-2.35 (1H, m), 2.70-2.75 (1H, m), 3.46 (1H, d, J=15.3 Hz), 3.46-3.53 (2H, m), 3.74 (1H, d, J=15.3 Hz), 4.21-4.25 (2H, m), 7.15 (2H, t, J=9.0 Hz), 7.52-7.60 (3H, m), 7.65-7.69 (2H, m).
Mass spectrum (EI): m/z 404 (M+H)⁺
Melting point: 165-168°C.

### (Example 35) {3-[2-Chloro-4-(pyridin-3-yl)phenyl]-8-methyl-5,6,8,9-tetrahydro[1,2,4]triazolo[4,3-d][1,4]thiazepin-8-yl}methanol (Example Compound No. 1-129)

### (35a) 8-({[t-Butyl(dimethyl)silyl]oxy}methyl)-3-[2-chloro-4-(pyridin-3-yl)phenyl]-8-methyl-5,6,8,9-tetrahydro[1,2,4]triazolo[4,3-d][1,4]thiazepine

The title compound (154 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (pyridin-3-yl)boronic acid (45.4 mg, 0.370 mmol).
¹H NMR spectrum (400 MHz, CDCl₃) δppm: 0.08 (3H, s), 0.10 (3H, s), 0.94 (9H, s), 1.29 (3H, s), 2.96 (2H, brs), 3.51 (1H, d, J=15.3 Hz), 3.57 (1H, d, J=15.3 Hz), 3.61 1 (1H, d, J=11.0 Hz), 3.65 (1H, d, J=11.0 Hz), 4.17-4.21 (2H, m), 7.42-7.70 (3H, m), 7.74 (1H, s), 7.90-7.93 (1H, m), 8.69 (1H, dd, J=2.0, 4.7 Hz), 8.88 (1H, dd, J=0.8, 2.0 Hz).

### (35b) {3-[2-Chloro-(4-pyridin-3-yl)phenyl]-8-methyl-5,6,8,9-tetrahydro[1,2,4]triazolo[4,3-d][1,4]thiazepin-8-yl}methanol

The title compound was obtained as a white powder (83.1 mg, yield 70%) in the same manner as in Example (9c) using 8-({[t-butyl(dimethyl)silyl]oxy}methyl)-3-[2-chloro-4-(pyridin-3-yl)phenyl]-8-methyl-5,6,8,9-tetrahydro[1,2,4]triazolo[4,3-d][1,4]thiazepine (154 mg, 0.308 mmol) obtained in Example (35a).
¹H NMR spectrum (400 MHz, CDCl₃) δppm: 1.41 (3H, s), 2.32 (1H, t, J=7.0 Hz), 2.68-2.74 (1H, m), 3.03 (1H, brs), 3.47 (1H, d, J=15.5 Hz), 3.52 (2H, d, J=7.0 Hz), 3.75 (1H, d, J=15.5 Hz), 4.23-4.25 (2H, m), 7.44 (1H, dd, J=6.7, 7.8 Hz), 7.63 (2H, s), 7.75 (1H, s), 7.91 (1H, dt, J=2.0, 7.8 Hz), 8.69 (1H, dd, J=2.0, 5.1 Hz), 8.88 (1H, d, J=2.0 Hz).
Mass spectrum (ESI): 387.1068 (M+H)⁺
Melting point: 206-209°C.

### (Example 36) {3-[2-Chloro-4-(pyridin-4-yl)phenyl]-8-methyl-5,6,8,9-tetrahydro[1,2,4]triazolo[4,3-d][1,4]thiazepin-8-yl}methanol (Example Compound No. 1-130)

### (36a) 8-({[t-Butyl(dimethyl)silyl]oxy}methyl)-3-[2-chloro-4-(pyridin-4-yl)phenyl]-8-methyl-5,6,8,9-tetrahydro[1,2,4]triazolo[4,3-d][1,4]thiazepine

The title compound (154 mg, yield 99%) was obtained in the same manner as in Example (1f) using 3-(4-bromo-2-chlorophenyl)-8-({[t-butyl(dimethyl)silyl]oxy}methyl)-8-methyl-5,6,8,9-tetrahydro[1,2,4]triazolo[4,3-d][1,4]thiazepine (155 mg, 0.308 mmol) obtained in Example (9a) and (pyridin-4-yl)boronic acid (45.4 mg, 0.370 mmol).
¹H NMR spectrum (500 MHz, CDCl₃) δppm: 0.08 (3H, s), 0.10 (3H, s), 0.94 (9H, s), 1.29 (3H, s), 2.97 (2H, brs), 3.53 (1H, d, J=15.6 Hz), 3.58 (1H, d, J=15.2 Hz), 3.61 (1H, d, J=10.0 Hz), 3.65 (1H, d, J=10.0 Hz), 4.18-4.19 (2H, m), 7.45-7.70 (4H, m), 7.79 (1H, s), 8.75 (2H, d, J=6.5 Hz).

### (36b) {3-[2-Chloro-4-(pyridin-4-yl)phenyl]-8-methyl-5,6,8,9-tetrahydro[1,2,4]triazolo[4,3-d][1,4]thiazepin-8-yl}methanol

The title compound was obtained as a white powder (65.0 mg, yield 55%) in the same manner as in Example (9c) using 8-({[t-butyl(dimethyl)silyl]oxy}methyl)-3-[2-chloro-4-(pyridin-4-yl)phenyl]-8-methyl-5,6,8,9-tetrahydro[1,2,4]triazolo[4,3-d][1,4]thiazepine (154 mg, 0.308 mmol) obtained in Example (36a).
¹H NMR spectrum (400 MHz, DMSO-d₆) δppm: 1.16 (3H, s), 2.87-3.01 (2H, m), 3.28-3.44 (4H, m), 4.09-4.11 (2H, m), 5.16 (1H, t, J=5.5 Hz), 7.70 (1H, d, J=8.2 Hz), 7.84-7.85 (2H, m), 7.96 (1H, dd, J=1.6, 8.2 Hz), 8.14 (1H, d, J=1.6 Hz), 8.71 (2H, d, J=6.3 Hz).
Mass spectrum (ESI): 387.1036 (M+H)⁺
Melting point: 229-233°C.

### (Example 37) {3-[2-Chloro-4-(morpholin-4-yl)phenyl]-8-methyl-5,6,8,9-tetrahydro[1,2,4]triazolo[4,3-d][1,4]thiazepin-8-yl}methanol (Example Compound No. 1-138)

### (37a) t-Butyl 2-{[2-chloro-4-(morpholin-4-yl)phenyl]carbonyl}hydrazinecarboxylate

The title compound was obtained as a colorless oily substance (353 mg, 68%) in the same manner as in Example (1e) using 2-chloro-4-(morpholin-4-yl)benzoic acid (J. Med. Chem. 2000, 43, 4388-4397.) (353 mg, 1.5 mmol), oxalyl chloride (0.3 mL, 3.5 mmol), N,N-dimethylformamide (catalytic amount), t-butyl carbazate (560 mg, 4.2 mmol), and triethylamine (0.8 mL, 6.0 mmol).
¹H NMR spectrum (400 MHz, CDCl₃) δppm: 1.50 (9H, s), 3.25 (4H, t, J=5.0 Hz), 3.85 (4H, t, J=5.0 Hz), 6.79-6.83 (3H, m), 7.80 (1H, d, J=7.0 Hz), 8.22 (1H, br, NH).

### (37b) 2-Chloro-4-(morpholin-4-yl)benzoic acid hydrazide

The title compound was obtained as a white solid (165 mg, 67%) in the same manner as in Example (1g) using t-butyl 2-{[2-chloro-4-(morpholin-4-yl)phenyl]carbonyl}hydrazinecarboxylate (350 mg, 1.0 mmol) obtained in Example (37a) and 4 N hydrogen chloride/ethyl acetate (10 mL).
¹H NMR spectrum (400 MHz, CDCl₃) δppm: 3.24 (4H, t, J=5.0 Hz), 3.85 (4H, t, J=5.0 Hz), 6.80-6.83 (2H, m), 7.61 (1H, br, NH), 7.74 (1H, d, J=8.6 Hz).

### (37c) 8-({[t-Butyl(dimethyl)silyl]oxy}methyl)-3-[2-chloro-4-(morpholin-4-yl)phenyl]-8-methyl-5,6,8,9-tetrahydro[1,2,4]triazolo[4,3-d][1,4]thiazepine

The title compound was obtained as a colorless oily substance (215 mg, 79%) in the same manner as in Example (1h) using 7-({[t-butyl(dimethyl)silyl]oxy}methyl)-7-methyl-5-methylthio-2,3,6,7-tetrahydro-1,4-thiazepine (181 mg, 0.57 mmol) obtained in Example (1d) and 2-chloro-4-(morpholin-4-yl)benzoic acid hydrazide (130 mg, 0.53 mmol) obtained in Example (37b).
¹H NMR spectrum (400 MHz, CDCl₃) δppm: 0.07 (3H, s), 0.09 (3H, s), 0.93 (9H, s), 1.26 (3H, s), 2.88-2.94 (2H, m), 3.25 (4H, t, J=5.0 Hz), 3.47 (1H, d, J=16.0 Hz), 3.53 (1H, d, J=16.0 Hz), 3.58 (1H, d, J=10.0 Hz), 3.63 (1H, d, J=10.0 Hz), 3.84-3.88 (4H, m), 4.10-4.15 (2H, m), 6.87 (1H, dd, J=2.3, 8.6 Hz), 6.96 (1H, d, J=2.3 Hz), 7.36 (1H, d, J=8.6 Hz).

### (37d) {3-[2-Chloro-4-(morpholin-4-yl)phenyl]-8-methyl-5,6,8,9-tetrahydro[1,2,4]triazolo[4,3-d][1,4]thiazepin-8-yl}methanol

The title compound was obtained as a white solid (107 mg, 64%) in the same manner as in Example (li) using 8-({[t-butyl(dimethyl)silyl]oxy}methyl)-3-[2-chloro-4-(morpholin-4-yl)phenyl]-8-methyl-5,6,8,9-tetrahydro[1,2,4]triazolo[4,3-d][1,4]thiazepine (215 mg, 0.42 mmol) obtained in Example (37c) and 4 N hydrogen chloride/dioxane (5 mL).
¹H NMR spectrum (400 MHz, CDCl₃) δppm: 1.39 (3H, s), 2.41 (1H, br, OH), 2.62-2.68 (1H, m), 2.96-3.04 (1H, m), 3.25 (4H, t, J=5.0 Hz), 3.42 (1H, d, J=15.2 Hz), 3.43-3.48 (2H, m), 3.71 (1H, d, J=15.2 Hz), 3.87 (4H, t, J=5.0 Hz), 4.11-4.21 (2H, m), 6.87 (1H, dd, J=3.0, 8.6 Hz), 6.97 (1H, d, J=3.0 Hz), 7.3 (1H, d, J=8.6 Hz).
Mass spectrum (FAB): m/z 395 (M+H)⁺
Melting point: 191-193°C.

### (Example 38) {3-[2-Chloro-4-(piperidin-1-yl)phenyl]-8-methyl-5,6,8,9-tetrahydro[1,2,4]triazolo[4,3-d] [1,4]thiazepin-8-yl}methanol (Example Compound No. 1-139)

### (38a) t-Butyl 2-{[2-chloro-4-(piperidin-1-yl)phenyl]carbonyl}hydrazinecarboxylate

The title compound was obtained as a white solid (387 mg, 70%) in the same manner as in Example (1e) using 2-chloro-4-(piperidin-1-yl)benzoic acid (J. Med. Chem. 2000, 43, 4388-4397.) (373 mg, 1.6 mmol), oxalyl chloride (0.3 mL, 3.5 mmol), N,N-dimethylformamide (catalytic amount), t-butyl carbazate (490 mg, 3.7 mmol), and triethylamine (0.8 mL, 6.0 mmol).
¹H NMR spectrum (400 MHz, CDCl₃) δppm: 1.50 (9H, s), 1.62-1.80 (6H, m), 3.27-3.33 (4H, m), 6.70-6.80 (3H, m), 7.79 (1H, d, J=7.0 Hz), 8.26 (1H, br, NH.

### (38b) 2-Chloro-4-(piperidin-1-yl)benzoic acid hydrazide

The title compound was obtained as a white solid (246 mg, 89%) in the same manner as in Example (1g) using t-butyl 2-{[2-chloro-4-(piperidin-1-yl)phenyl]carbonyl}hydrazinecarboxylate (387 mg, 1.1 mmol) obtained in Example (38a) and 4 N hydrogen chloride/ethyl acetate (10 mL).
¹H NMR spectrum (400 MHz, CDCl₃) δppm: 1.63-1.67 (6H, m), 3.26-3.30 (4H, m), 4.14 (2H, br, NH₂), 6.78-6.81 (2H, m), 7.70-7.73 (2H, m).

### (38c) 8-({[t-Butyl(dimethyl)silyl]oxy}methyl)-3-[2-chloro-4-(piperidin-1-yl)phenyl]-8-methyl-5,6,8,9-tetrahydro[1,2,4]triazolo[4,3-d][1,4]thiazepine

The title compound was obtained as a colorless oily substance (175 mg, 70%) in the same manner as in Example (1h) using 7-({[t-butyl(dimethyl)silyl]oxy}methyl)-7-methyl-5-methylthio-2,3,6,7-tetrahydro-1,4-thiazepine (177 mg, 0.55 mmol) obtained in Example (1d) and 2-chloro-4-(piperidin-1-yl)benzoic acid hydrazide (126 mg, 0.50 mmol) obtained in Example (38b). ¹H NMR spectrum (400 MHz, CDCl₃) δppm: 0.07 (3H, s), 0.09 (3H, s), 0.92 (9H, s), 1.26 (3H, s), 1.63-1.67 (6H, m), 2.88-2.92 (2H, m), 3.26-3.30 (4H, m), 3.46 (1H, d, J=15.0 Hz), 3.53 (1H, d, J=15.0 Hz), 3.58 (1H, d, J=10.0 Hz), 3.62 (1H, d, J=10.0 Hz), 4.10-4.19 (2H, m), 6.81 (1H, d, J=2.3 Hz), 6.85 (1H, dd, J=2.3, 8.6 Hz), 7.30 (1H, d, J=8.6 Hz).

### (38d) {3-[2-Chloro-4-(piperidin-1-yl)phenyl]-8-methyl-5,6,8,9-tetrahydro.[1,2,4]triazolo [4,3-d] [1,4]thiazepin-8-yl}methanol

The title compound was obtained as a white solid (73.8 mg, 55%) in the same manner as in Example (1i) using 8-({[t-butyl(dimethyl)silyl]oxy}methyl)-3-[2-chloro-4-(piperidin-1-yl)phenyl]-8-methyl-5,6,8,9-tetrahydro[1,2,4]triazolo[4,3-d][1,4]thiazepine (173 mg, 0.34 mmol) obtained in Example (38c) and 4 N hydrogen chloride/dioxane (5 mL).
¹H NMR spectrum (400 MHz, CDCl₃) δppm: 1.39 (3H, s), 1.63-1.73 (6H, m), 2.40 (1H, br, OH), 2.61-2.68 (1H, m), 2.96-3.03 (1H, m), 3.27-3.31 (4H, m), 3.41 (1H, d, J=15.3 Hz), 3.44-3.48 (2H, m), 3.71 (1H, d, J=15.3 Hz), 4.15-4.21 (2H, m), 6.86 (1H, dd, J=3.0, 8.2 Hz), 6.95 (1H, d, J=3.0 Hz), 7.29 (1H, d, J=8.2 Hz).
Mass spectrum (FAB): m/z 393 (M+H)⁺
Melting point: 195-198°C.

### (Example 39) {3-[2-Chloro-4-(pyrroIidin-1-yl)phenyl]-8-methyl-5,6,8,9-tetrahydro[1,2,4]triazolo[4,3-d][1,4]thiazepin-8-yl}methanol (Example Compound No. 1-140)

### (39a) t-Butyl 2-{[2-chloro-4-(pyrrolidin-1-yl)phenyl]carbonyl}hydrazinecarboxylate

The title compound was obtained as a white solid (498 mg, 68%) in the same manner as in Example (1e) using 2-chloro-4-(pyrrolidin-1-yl)benzoic acid (J. Med. Chem. 2000, 43, 4388-4397.) (490 mg, 2.2 mmol), oxalyl chloride (0.4 mL, 4.6 mmol), N,N-dimethylformamide (catalytic amount), t-butyl carbazate (570 mg, 4.4 mmol), and triethylamine (1.0 mL, 7.2 mmol).
¹H NMR spectrum (400 MHz, CDCl₃) δppm: 1.50 (9H, s), 1.96-2.08 (4H, m), 3.22-3.38 (4H, m), 6.39-6.47 (3H, m), 7.83 (1H, d, J=7.0 Hz), 8.33 (1H, br, NH).

### (39b) 2-Chloro-4-(pyrrolidin-1-yl)benzoic acid hydrazide

The title compound was obtained as a white solid (319 mg, 91 %) in the same manner as in Example (1g) using t-butyl 2-{[2-chloro-4-(pyrrolidin-1-yl)phenyl]carbonyl}hydrazinecarboxylate (498 mg, 1.5 mmol) obtained in Example (39a) and 4 N hydrogen chloride/ethyl acetate (10 mL).
¹H NMR spectrum (400 MHz, CDCl₃) δppm: 2.00-2.06 (4H, m), 3.28-3.34(4H, m), 4.20 (2H, br, NH₂), 6.44-6.47 (2H, m), 7.71-7.80 (2H, m).

### (39c) 8-({[t-Butyl(dimethyl)silyl]oxy}methyl)-3-[2-chloro-4-(pyrrolidin-1-yl)phenyl]-8-methyl-5,6,8,9-tetrahydro[1,2,4]triazolo[4,3-d][1,4]thiazepine

The title compound was obtained as a colorless oily substance (196 mg, 76%) in the same manner as in Example (1h) using 7-({[t-butyl(dimethyl)silyl]oxy}methyl)-7-methyl-5-methylthio-2,3,6,7-tetrahydro-1,4-thiazepine (178 mg, 0.56 mmol) obtained in Example (1d) and 2-chloro-4-(pyrrolidin-1-yl)benzoic acid hydrazide (126 mg, 0.53 mmol) obtained in Example (39b).
¹H NMR spectrum (400 MHz, CDCl₃) δppm: 0.07 (3H, s), 0.08 (3H, s), 0.92 (9H, s), 1.26 (3H, s), 2.02-2.08 (4H, m), 2.85-2.93 (2H, m), 3.28-3.36 (4H, m), 3.46 (1H, d, J=15.5 Hz), 3.53 (1H, d, J=15.5 Hz), 3.58 (1H, d, J=10.2 Hz), 3.63 (1H, d, J=10.2 Hz), 4.10-4.16 (2H, m), 6.50 (1H, dd, J=3.0, 8.6 Hz), 6.66 (1H, d, J=3.0 Hz), 7.28 (1H, d, J=8.6 Hz).

### (39d) {3-[2-Chloro-4-(pyrrolidin-1-yl)phenyl]-8-methyl-5,6,8,9-tetrahydro[1,2,4]triazolo[4,3-d][1,4]thiazepin-8-yl}methanol

The title compound was obtained as a white solid (84.0 mg, 56%) in the same manner as in Example (1i) using 8-({[t-butyl(dimethyl)silyl]oxy}methyl)-3-[2-chloro-4-(pyrrolidin-1-yl)phenyl]-8-methyl-5,6,8,9-tetrahydro[1,2,4]triazolo[4,3-d][1,4]thiazepine (196 mg, 0.40 mmol) obtained in Example (39c) and 4 N hydrogen chloride/dioxane (5 mL).
¹H NMR spectrum (400 MHz, CDCl₃) δppm: 1.39 (3H, s), 2.02-2.08 (4H, m), 2.41 (1H, br, OH), 2.60-2.66 (1H, m), 2.92-3.02 (1H, m), 3.29-3.35 (4H, m), 3.42 (1H, d, J=15.2 Hz), 3.43-3.48 (2H, m), 3.71 (1H, d, J=15.2 Hz), 4.11-4.22 (2H, m), 6.51 (1H, dd, J=2.5, 8.2 Hz), 6.66 (1H, d, J=2.5 Hz), 7.25-7.33 (1H, m).
Mass spectrum (FAB): m/z 379 (M+H)⁺
Melting point: 193-196°C.

### (Example 40) {3-[2-Chloro-4-(1H-pyrrol-1-yl)phenyl]-8-methyl-5,6,8,9-tetrahydro[1,2,4]triazolo[4,3-d][1,4]thiazepin-8-yl}methanol (Example Compound No. 1-141)

### (40a) 2-Chloro-4-(1H-pyrrol-1-yl)benzoic acid hydrazide

To a solution of 2-chloro-4-(1H-pyrrol-1-yl)benzoic acid (US6521666 B1.) (380 mg, 1.7 mmol) and triethylamine (0.4 mL, 2.8 mmol) in N,N-dimethylformamide (10 mL) were added 1-(3-dimethylaminopropyl)-3-ethylcarbodiimide monohydrochloride (495 mg, 2.6 mmol) and 1-hydroxybenzotriazole (270 mg, 2.0 mmol), and the mixture was stirred for 2 h, followed by the addition of hydrazine monohydrate (0.5 mL, 8 mmol), and stirred at room temperature for 1 h. Water was added to the reaction mixture, and the mixture was extracted with ethyl acetate, then washed with water and saturated brine, and dried with anhydrous sodium sulfate. The filtrate was concentrated under reduced pressure, and then the resulting solid was collected by filtration and washed with diisopropyl ether to obtain the title compound as a white solid (190 mg, 47%).
¹H NMR spectrum (400 MHz, CDCl₃) δppm: 4.16 (2H, br, NH₂), 6.39(2H, t, J=3.0 Hz), 7.11 (2H, t, J=3.0 Hz), 7.37 (1H, dd, J=2.3, 8.2 Hz), 7.45 (1H, d, J=2.3 Hz), 7.56 (1H, br, NH), 7.79 (1H, d, J=8.2 Hz).

### (40b) 8-({[t-Butyl(dimethyl)silyl]oxy)methyl)-3-[2-chloro-4-(1H-pyrrol-1-yl)phenyl]-8-methyl-5,6,8,9-tetrahydro[1,2,4]triazolo[4,3-d][1,4]thiazepine

The title compound was obtained as a colorless oily substance (208 mg, 93%) in the same manner as in Example (1h) using 7-({[t-butyl(dimethyl)silyl]oxy}methyl)-7-methyl-5-methylthio-2,3,6,7-tetrahydro-1,4-thiazepine (155 mg, 0.49 mmol) obtained in Example (1d) and 2-chloro-4-(1H-pyrrol-1-yl)benzoic acid hydrazide (108 mg, 0.46 mmol) obtained in Example (40a).
¹H NMR spectrum (400 MHz, CDCl₃) δppm: 0.08 (3H, s), 0.09 (3H, s), 0.93 (9H, s), 1.28 (3H, s), 2.92-2.98 (2H, m), 3.50 (1H, d, J=15.2 Hz), 3.56 (1H, d, J=15.2 Hz), 3.60 (1H, d, J=10.0 Hz), 3.64 (1H, d, J=10.0 Hz), 4.11-4.20 (2H, m), 6.41(2H, t, J=3.0 Hz),7.13 (2H, t, J=3.0 Hz), 7.44 (1H, dd, J=2.3, 7.8 Hz), 7.55-7.58 (2H, m).

### (40c) {3-[2-Chloro-4-(1H-pyrrol-1-yl)phenyl]-8-methyl-5,6,8,9-tetrahydro[1,2,4]triazolo[4,3-d][1,4]thiazepin-8-yl}methanol

The title compound was obtained as a white solid (82.3 mg, 52%) in the same manner as in Example (1i) using 8-({[t-butyl(dimethyl)silyl]oxy}methyl)-3-[2-chloro-4-(1H-pyrrol-1-yl)phenyl]-8-methyl-5,6,8,9-tetrahydro[1,2,4]triazolo[4,3-d][1,4]thiazepine (208 mg, 0.43 mmol) obtained in Example (40b) and 4 N hydrogen chloride/dioxane (5 mL).
¹H NMR spectrum (400 MHz, CDCl₃) δppm: 1.41 (3H, s), 2.33 (1H, br, OH), 2.67-2.74 (1H, m), 2.98-3.05 (1H, m), 3.45 (1H, d, J=15.2 Hz), 3.46-3.49 (2H, m), 3.74 (1H, d, J=15.2 Hz), 4.18-4.26 (2H, m), 6.42(2H, t, J=3.0 Hz),7.14 (2H, t, J=3.0 Hz), 7.45 (1H, dd, J=1.7, 8.2 Hz), 7.52-7.57 (2H, m).
Mass spectrum (FAB): m/z 375 (M+H)⁺
Melting point: 110-112°C.

### (Example 41) 3-{2-[(4-Fluorophenyl)methoxy]phenyl}-8-methyl-5,6,8,9-tetrahydro[1,2,4]triazolo[4,3-d][1,4]thiazepin-8-yl]methanol (Example Compound No. 1-156)

### (41a) 8-({[t-Butyl(dimethyl)silyl]oxy}methyl)-3-{2-[(4-fluorophenyl)methoxy]phenyl}-8-methyl-5,6,8,9-tetrahydro[1,2,4]triazolo[4,3-d][1,4]thiazepine

The title compound was obtained as a colorless oily substance (150 mg, 72%) in the same manner as in Example (1h) using 7-({[t-butyl(dimethyl)silyl]oxy}methyl)-7-methyl-5-methylthio-2,3,6,7-tetrahydro-1,4-thiazepine (144 mg, 0.45 mmol) obtained in Example (1d) and 2-[(4-fluorophenyl)methoxy]benzoic acid hydrazide (Bioorg. Med. Chem. Lett. 2005, 15, 1863-1865.) (113 mg, 0.43 mmol).
¹H NMR spectrum (400 MHz, CDCl₃) δppm: 0.07 (3H, s), 0.07 (3H, s), 0.92 (9H, s), 1.07 (3H, s), 2.62-2.66 (2H, m), 3.38 (1H, d, J=15.0 Hz), 3.52 (1H, d, J=15.0 Hz), 3.52-3.54 (2H, m), 3.96-4.02 (1H, m), 4.10-4.13 (1H, m), 4.98 (1H, d, J=10.0 Hz), 5.02 (1H, d, J=10.0 Hz), 6.99-7.17 (4H, m), 7.21-7.31 (1H, m), 7.39-7.54 (3H, m).

### (41b) 3-{2-[(4-Fluorophenyl)methoxy]phenyl}-8-methyl-5,6,8,9-tetrahydro[1,2,4]triazolo[4,3-d][1,4]thiazepin-8-yl]methanol

The title compound was obtained as a white foamy substance (85.6 mg, 69%) in the same manner as in Example (1i) using 8-({[t-butyl(dimethyl)silyl]oxy}methyl)-3-{2-[(4-fluorophenyl)methoxy]phenyl}-8-methyl-5,6,8,9-tetrahydro[1,2,4]triazolo[4,3-d][1,4]thiazepine (157 mg, 0.31 mmol) obtained in Example (41a) and 4 N hydrogen chloride/dioxane (5 mL).
¹H NMR spectrum (400 MHz, CDCl₃) δppm: 1.29 (3H, s), 2.36 (1H, br, OH), 2.41 (1H, dd, J=5.9, 15.6 Hz), 2.67 (1H, dd, J=9.3, 15.6 Hz), 3.21-3.30 (2H, m), 3.34 (1H, d, J=15.2 Hz), 3.64 (1H, d, J=15.2 Hz), 4.01-4.05 (1H, m), 4.12-4.17 (1H, m), 5.00 (2H, s), 7.03-7.17 (4H, m), 7.21-7.32 (2H, m), 7.47-7.55 (2H, m).
Mass spectrum (EI): m/z 399 (M⁺).

### (Example 42) {3-[2-Chloro-4-(4-fluorophenoxy)phenyl]-8-methyl-5,6,8,9-tetrahydro[1,2,4]triazolo[4,3-d][1,4]thiazepin-8-yl}methanol (Example Compound No. 1-159)

### (42a) Methyl 2-chloro-4-(4-fluorophenoxy)benzoate

To a solution of methyl 2-chloro-4-hydroxybenzoate (500 mg, 2.7 mmol) in methylene chloride (30 mL) were added (4-fluorophenyl)boronic acid (750 mg, 5.4 mmol), copper(II) acetate (540 mg, 3.0 mmol), triethylamine (2 mL, 14 mmol), and molecular sieves 4A (800 mg), and the mixture was stirred overnight. The mixture was filtered and washed with ethyl acetate, the filtrate was concentrated under reduced pressure, and then the residue was purified by silica gel column chromatography (elution solvent: hexane/ethyl acetate = 4/1) to obtain the title compound as a colorless oily substance (405 mg, 54%).
¹H NMR spectrum (400 MHz, CDCl₃) δppm: 3.91 (3H, s), 6.85 (1H, dd, J=2.5, 9.0 Hz), 6.98 (1H, d, J=2.5 Hz), 7.02-7.13 (4H, m), 7.87 (1H, d, J=9.0 Hz).

### (42b) 2-Chloro-4-(4-fluorophenoxy)benzoic acid

The title compound was obtained as a white solid (350 mg, 91%) in the same manner as in Example (10b) using methyl 2-chloro-4-(4-fluorophenoxy)benzoate (405 mg, 1.4 mmol) obtained in Example (42a) and sodium hydroxide (310 mg, 7.8 mmol).
¹HNMR spectrum (400 MHz, CDCl₃) δppm: 6.90 (1H, d, J=8.0 Hz), 7.05-7.15 (5H, m), 8.03 (1H, d, J=8.0 Hz).

### (42c) t-Butyl 2-[2-chloro-4-(4-fluorophenoxy)phenyl]carbonyl hydrazinecarboxylate

The title compound was obtained as a colorless oily substance (471 mg, 94%) in the same manner as in Example (1e) using 2-chloro-4-(4-fluorophenoxy)benzoic acid (350 mg, 1.3 mmol) obtained in Example (42b), oxalyl chloride (0.3 mL, 3.0 mmol), N,N-dimethylformamide (catalytic amount), t-butyl carbazate (290 mg, 2.2 mmol), and triethylamine (0.8 mL, 5.8 mmol).
¹H NMR spectrum (400 MHz, CDCl₃) δppm: 1.51 (9H, s), 6.74 (1H, br, NH), 6.90 (1H, dd, J=2.4, 8.5 Hz), 6.96 (1H, d, J=2.4 Hz), 7.01-7.08 (2H, m), 7.10-7.13 (2H, m), 7.78 (1H, d, J=8.2 Hz), 8.04 (1H, br, NH).

### (42d) 2-Chloro-4-(4-fluorophenoxy)benzoic acid hydrazide

The title compound was obtained as a white solid (286 mg, 85%) in the same manner as in Example (1g) using t-butyl 2-[2-chloro-4-(4-fluorophenoxy)phenyl]carbonyl hydrazinecarboxylate (471 mg, 1.2 mmol) obtained in Example (42c) and 4 N hydrogen chloride/ethyl acetate (10 mL).
¹H NMR spectrum (400 MHz, CDCl₃) δppm: 4.14 (2H, br, NH₂), 6.89 (1H, dd, J=2.0, 9.0 Hz), 6.96 (1H, d, J=2.0 Hz), 7.01-7.12 (4H, m), 7.49 (1H, br, NH), 7.69 (1H, d, J=9.0 Hz).

### (42e) 8-({[t-Butyl(dimethyl)silyl]oxy}methyl)-3-[2-chloro-4-(4-fluorophenoxy)phenyl]-8-methyl-5,6,8,9-tetrahydro[1,2,4]triazolo[4,3-d][1,4]thiazepine

The title compound was obtained as a colorless oily substance (195 mg, 82%) in the same manner as in Example (1h) using 7-({[t-butyl(dimethyl)silyl]oxy}methyl)-7-methyl-5-methylthio-2,3,6,7-tetrahydro-1,4-thiazepine (148 mg, 0.46 mmol) obtained in Example (1d) and 2-chloro-4-(4-fluorophenoxy)benzoic acid hydrazide (120 mg, 0.44 mmol) obtained in Example (42d).
¹H NMR spectrum (400 MHz, CDCl₃) δppm: 0.07 (3H, s), 0.09 (3H, s), 0.93 (9H, s), 1.27 (3H, s), 2.89-2.96 (2H, m), 3.49 (1H, d, J=22.2 Hz), 3.53 (1H, d, J=22.2 Hz),3.60 (1H, d, J=16.5 Hz), 3.62 (1H, d, J=16.4 Hz), 4.12-4.17 (2H, m), 6.96 (1H, dd, J=2.3, 9.0 Hz), 7.02-7.14 (5H, m), 7.44 (1H, d, J=9.0 Hz).

### (42f) {3-[2-Chloro-4-(4-fluorophenoxy)phenyl]-8-methyl-5,6,8,9-tetrahydro[1,2,4]triazolo[4,3-d][1,4]thiazepin-8-yl}methanol

The title compound was obtained as a white solid (107 mg, 70%) in the same manner as in Example (1i) using 8-({[t-butyl(dimethyl)silyl]oxy}methyl)-3-[2-chloro-4-(4-fluorophenoxy)phenyl]-8-methyl-5,6,8,9-tetrahydro[1,2,4]triazolo[4,3-d][1,4]thiazepine (195 mg, 0.37 mmol) obtained in Example (42e) and 4 N hydrogen chloride/dioxane (5 mL).
¹H NMR spectrum (400 MHz, CDCl₃) δppm: 1.40 (3H, s), 2.33(1H, br, OH), 2.65-2.75 (1H, m), 2.93-3.02 (1H, m), 3.43 (1H, d, J=15.0 Hz), 3.46-3.51 (2H, m), 3.72 (1H, d, J=15.0 Hz), 4.13-4.21 (2H, m), 6.97 (1H, dd, J=2.3, 8.2 Hz), 7.05-7.15 (5H, m), 7.43 (1H, d, J=8.2 Hz).
Mass spectrum (EI): m/z 419 (M⁺)
Melting point: 138-140°C.

### (Example 43) {3-[1-(4-Fluorophenyl)-1H-indol-4-yl]-8-methyl-5,6,8,9-tetrahydro[1,2,4]triazolo[4,3-d][1,4]thiazepin-8-yl}methanol(Example Compound No. 1-163)

### (43a) 8-({[t-Butyl(dimethyl)silyl]oxy}methyl)-3-[1-(4-fluorophenyl)-1H-indol-4-yl]-8-methyl-5,6,8,9-tetrahydro[1,2,4]triazolo[4,3-d][1,4]thiazepine

To a solution of methyl 1-(4-fluorophenyl)-1H-indole-4-carboxylate (600 mg, 2.2 mmol) in ethanol (10 mL) was added hydrazine monohydrate (3 mL, 48 mmol), and the mixture was stirred at 100°C for 3 h. Water was added, and the mixture was extracted with ethyl acetate, washed with water and saturated brine, and then dried with anhydrous sodium sulfate. The filtrate was concentrated under reduced pressure, and then the resulting solid was collected by filtration and washed with diisopropyl ether to obtain 1-(4-fluorophenyl)-1H-indole-4-carboxylic acid hydrazide as a white solid (546 mg, 91 %).

The title compound was obtained as a colorless oily substance (256 mg, 93%) in the same manner as in Example (1h) using 7-({[t-butyl(dimethyl)silyl]oxy}methyl)-7-methyl-5-methylthio-2,3,6,7-tetrahydro-1,4-thiazepine (172 mg, 0.37 mmol) obtained in Example (1d) and 1-(4-fluorophenyl)-1H-indole-4-carboxylic acid hydrazide (146 mg, 0.54 mmol) obtained above.
¹H NMR spectrum (400 MHz, CDCl₃) δppm: 0.09 (3H, s), 0.11 (3H, s), 0.94 (9H, s), 1.31 (3H, s), 2.84-2.88 (1H, m), 2.93-2.97 (1H, m), 3.53 (1H, d, J=15.0 Hz), 3.58 (1H, d, J=15.0 Hz), 3.63-3.69 (2H, m), 4.25-4.30 (1H, m), 4.40-4.44 (1H, m), 6.67 (1H, d, J=3.5 Hz), 7.22-7.53 (7H, m), 7.57 (1H, d, J=8.3 Hz).

### (43b) {3-[1-(4-Fluorophenyl)-1H-indol-4-yl]-8-methyl-5,6,8,9-tetrahydro[1,2,4]triazolo[4,3-d] [1,4]thiazepin-8-yl}methanol

The title compound was obtained as a white solid (119 mg, 61%) in the same manner as in Example (1i) using 8-({[t-butyl(dimethyl)silyl]oxy}methyl)-3-[1-(4-fluorophenyl)-1H-indol-4-yl]-8-methyl-5,6,8,9-tetrahydro[1,2,4]triazolo[4,3-d][1,4]thiazepine (254 mg, 0.49 mmol) obtained in Example (43a) and 4 N hydrogen chloride/dioxane (5 mL).
¹H NMR spectrum (400 MHz, CDCl₃) δppm: 1.43 (3H, s), 2.50 (1H, br, OH), 2.59-2.67 (1H, m), 2.90-2.95 (1H, m), 3.40-3.50 (3H, m), 3.79 (1H, d, J=15.2 Hz), 4.22-4.29 (1H, m), 4.42-4.52 (1H, m), 6.66 (1H, d, J=3.5 Hz), 7.23-7.38 (5H, m), 7.45-7.53 (2H, m), 7.58 (1H, d, J=8.6 Hz).
Mass spectrum (EI): m/z 408 (M⁺).
Melting point: 117-120°C.

### (Example 44) 3-(2-Chlorophenyl)-8-ethyl-5,6,8,9-tetrahydro[,2,4]triazolo[4,3-d][1,4]thiazepine (Example CompoundNo. 2-14)

### (44a) 7-Ethyl-1,4-thiazepan-5-one

The title compound was obtained as a white solid (1.64 g, 34%) by performing the same reaction as in Example (1b) using methyl (2E)-2-pentenoate (3.43 g, 30.1 mmol) and 2-aminoethanethiol (2.78 g, 36.0 mmol). ¹H NMR spectrum (400 MHz, CDCl₃) δppm: 1.05 (3H, t, J=7.3 Hz), 1.61-1.74 (2H, m), 2.69-2.86 (3H, m), 2.94-3.00 (2H, m), 3.59-3.63 (2H, m), 6.00 (1H, br, NH).

### (44b) 7-Ethyl-1,4-thiazepane-5-thione

The title compound was obtained as a mixture with reagent residues (2.39 g) by performing the same reaction as in Example (1c) using 7-ethyl-1,4-thiazepan-5-one (1.64 g, 10.3 mmol) obtained in Example (44a) and Lawesson's reagent (2.3 g, 5.69 mmol). The resulting mixture was used as it was in the subsequent reaction.

### (44c) 7-Ethyl-5-methylthio-2,3,6,7-tetrahydro-1,4-thiazepine.

The title compound was obtained as a mixture with reagent residues (363 mg) by performing the same reaction as in Example (1d) using the 7-ethyl-1,4-thiazepane-5-thione mixture (600 mg) obtained in Example (44b), potassium hydroxide (685 mg, 10.4 mmol), and methyl iodide (1.1 ml, 17.7 mmol). The resulting mixture was used as it was in the subsequent reaction.

### (44d) 3-(2-Chlorophenyl)-8-ethyl-5,6,8,9-tetrahydro[1,2,4]triazolo[4,3-d][1,4]thiazepine

The title compound was obtained as a white solid (515 mg, 68%) by performing the same reaction as in Example (1h) using the 7-ethyl-5-methylthio-2,3,6,7-tetrahydro-1,4-thiazepine mixture (363 mg) obtained in Example (44c) and 2-chlorobenzohydrazide (330 mg, 1.93 mmol).
¹H NMR spectrum (400 MHz, CDCl₃) δppm: 1.09 (3H, t, J=7.3 Hz), 1.54-1.75 (2H, m), 2.77-2.95 (3H, m), 3.42 (1H, dd, J=15.1, 8.6 Hz), 3.69-3.73 (1H, m), 4.10-4.22 (2H, m), 7.39-7.54 (4H, m).
Mass spectrum (FAB): m/z 293 (M+H)⁺
Melting point: 92-93°C.

### (Example 45) 3-(2-Chlorophenyl)-8,8-dimethyl-5,6,8,9-tetrahydro[1,2,4]triazolo[4,3-d][1,4]thiazepine (Example Compound No. 2-40)

### (45a) 7,7-Dimethyl-1,4-thiazepane-5-thione

The title compound was obtained as a white solid (169 mg, 100%) in the same manner as in Example (1c) using 7,7-dimethyl-1,4-thiazepan-5-one (98.0 mg, 0.61 mmol) and Lawesson's reagent (140 mg, 0.34 mmol).
¹H NMR spectrum (400 MHz, CDCl₃) δppm: 1.46 (6H, s), 2.77-2.79 (2H, m), 3.48 (2H, s), 3.75-3.79 (2H, m).

### (45b) 7,7-Dimethyl-5-methylthio-2,3,6,7-tetrahydro-1,4-thiazepine

The title compound was obtained as a colorless oily substance (43.6 mg, 45%) in the same manner as in Example (1d) using 7,7-dimethyl-1,4-thiazepane-5-thione (88.8 mg, 0.51 mmol) obtained in Example (45a), potassium hydroxide (90 mg, 1.6 mmol), and methyl iodide (220 mg, 1.5 mmol).
¹H NMR spectrum (400 MHz, CDCl₃) δppm: 1.37 (6H, s), 2.26 (3H, s), 2.63-2.69 (2H, m), 2.94 (2H, s), 4.02-4.09 (2H, m).

### (45c) 3-(2-Chlorophenyl)-8,8-dimethyl-5,6,8,9-tetrahydro[1,2,4]triazolo[4,3-d][1,4]thiazepine

The title compound was obtained as a white solid (46.5 mg, 71 %) in the same manner as in Example (1h) using 7,7-dimethyl-5-methylthio-2,3,6,7-tetrahydro-1,4-thiazepine (42.0 mg, 0.22 mmol) obtained in Example (45b) and 2-chlorobenzoic acid hydrazide (38.0 mg, 0.22 mmol).
¹H NMR spectrum (400 MHz, CDCl₃) δppm: 1.40 (6H, s), 2.80-2.92 (2H, m), 3.50 (2H, s), 4.11-4.18 (2H, m), 7.39-7.44 (1H, m), 7.46-7.54 (3H, m).
Mass spectrum (EI): m/z 293 (M⁺).
Melting point: 183-185°C.

### (Example 46) 8,8-Dimethyl-3-(2-methylphenyl)-5,6,8,9-tetrahydro[1,2,4]triazolo[4,3-d][1,4]thiazepine (Example Compound No. 2-41)

The title compound was obtained as a white solid (99 mg, 34%) in the same manner as in Example (1h) using 7,7-dimethyl-5-methylthio-2,3,6,7-tetrahydro-1,4-thiazepine (0.20 g, 1.06 mmol) obtained in Example (45b) and 2-methylbenzoic acid hydrazide (0.16 g, 1.07 mmol).
¹H NMR spectrum (400 MHz, CDCl₃) δppm: 1.39 (6H, s), 2.23 (3H, s), 2.73-2.78 (2H, m), 3.50 (2H,s), 4.10-4.16 (2H, m), 7.25-7.36 (3H, m), 7.39-7.46 (1H, m). Mass spectrum (EI): m/z 273 (M⁺).
Melting point: 106-107°C.

### (Example 47) 8,8-Dimethyl-3-(2-methoxyphenyl)-5,6,8,9-tetrahydro[1,2,4]triazolo[4,3-d][1,4]thiazepine (Example Compound No. 2-42)

The title compound was obtained as a white solid (0.10 g, 51%) in the same manner as in Example (1h) using 7,7-dimethyl-5-methylthio-2,3,6,7-tetrahydro-1,4-thiazepine (0.13 g, 0.69 mmol) obtained in Example (45b) and 2-methoxybenzoic acid hydrazide (0.11 g, 0.66 mmol).
¹H NMR spectrum (400 MHz, CDCl₃) δppm: 1.39 (6H, s), 2.80-2.87 (2H, m), 3.48 (2H, s), 3.82 (3H, s), 4.11-4.18 (2H, m), 7.00 (1H, d, J=8.0 Hz), 7.09 (1H, t, J=7.0 Hz), 7.47-7.55 (2H, m).
Mass spectrum (FAB): m/z 290 (M+H)⁺.
Melting point: 141-143°C.

### (Example 48) 3-(2-Ethylphenyl)-8,8-dimethyl-5,6,8,9-tetrahydro[1,2,4]triazolo[4,3-d][1,4]thiazepine (Example Compound No. 2-43)

The title compound was obtained as a white solid (0.12 g, 45%) in the same manner as in Example (1h) using 7,7-dimethyl-5-methylthio-2,3,6,7-tetrahydro-1,4-thiazepine (0.18 g, 0.94 mmol) obtained in Example (45b) and 2-ethylbenzoic acid hydrazide (0.15 g, 0.91 mmol).
¹H NMR spectrum (400 MHz, CDCl₃) δppm: 1.13 (3H, t, J=7.5 Hz), 1.39 (6H, s), 2.55 (2H, q, J=7.5 Hz), 2.71-2.77 (2H, m), 3.50 (2H, s), 4.10-4.15 (2H, m), 7.22 (1H, dd, J=1.5, 7.5 Hz), 7.30 (1H, dt, J=1.5, 7.5 Hz), 7.38 (1H, dd, J=1.5, 7.5 Hz), 7.46 (1H, dt, J=1.5, 7.5 Hz).
Mass spectrum (EI): m/z 287 (M⁺).
Melting point: 125°C.

### (Example 49) 3-(2-Ethoxyphenyl)-8,8-dimethyl-5,6,8,9-tetrahydro[1,2,4]triazolo[4,3-d][1,4]thiazepine (Example Compound No. 2-44)

The title compound was obtained as a white solid (0.23 g, 72%) in the same manner as in Example (1h) using 7,7-dimethyl-5-methylthio-2,3,6,7-tetrahydro-1,4-thiazepine (0.20 g, 1.06 mmol) obtained in Example (45b) and 2-ethoxybenzoic acid hydrazide (0.19 g, 1.05 mmol).
¹H NMR spectrum (400 MHz, CDCl₃) δppm: 1.34 (3H, t, J=7.0 Hz), 1.40 (6H, s), 2.84-2.91 (2H, m), 3.54 (2H, s), 4.09 (2H, q, J=7.0 Hz), 4.14-4.20 (2H, m), 7.00 (1H, d, J=9.0 Hz), 7.08 (1H, t, J=7.5 Hz), 7.46-7.53 (2H, m).
Mass spectrum (EI): m/z 303 (N⁺).
Melting point: 136-137°C.

### (Example 50) 3-(2-Chloro-4-fluorophenyl)-8,8-dimethyl-5,6,8,9-tetrahydro[1,2,4]triazolo[4,3-d][1,4]thiazepine (Example Compound No. 2-45)

The title compound was obtained as a white solid (0.17 g, 67%) in the same manner as in Example (1h) using 7,7-dimethyl-5-methylthio-2,3,6,7-tetrahydro-1,4-thiazepine (0.15 g, 0.79 mmol) obtained in Example (45b) and 2-chloro-4-fluorobenzoic acid hydrazide (0.15 g, 0.80 mmol).
¹H NMR spectrum (400 MHz, CDCl₃) δppm: 1.39 (6H, s), 2.80-2.92 (2H, m), 3.50 (2H, s), 4.14 (2H, t, J=4.7 Hz), 7.12-7.17 (1H, m), 7.26-7.30 (1H, m), 7.48-7.53 (1H, m).
Mass spectrum (EI): m/z 311 (M⁺).
Melting point: 177-182°C.

### (Example 51) 3-(2-Chlorophenyl)-8-ethyl-8-methyl-5,6,8,9-tetrahydro[1,2,4]triazolo[4,3-d][1,4]thiazepine (Example Compound No. 2-53)

### (51a) 7-Ethyl-7-methyl-1,4-thiazepan-5-one

The title compound was obtained as a white solid (1.31 g, 35%) by performing the same reaction as in Example (1b) using ethyl (2EZ)-3-methyl-2-pentenoate (3.42 g, 21.9 mmol) and 2-aminoethanethiol (5.10 g, 66.1 mmol).
¹H NMR spectrum (400 MHz, CDCl₃) δppm: 1.02 (3H, t, J=7.2 Hz), 1.35 (3H, s), 1.71 (2H, q, J=7.2 Hz), 2.74-2.77 (2H, m), 2.90 (1H, d, J=14.1 Hz), 2.98 (1H, d J=14.1 Hz), 3.59-3.63 (2H, m), 5.94 (1H, br, NH).

### (51b) 7-Ethyl-7-methyl-1,4-thiazepane-5-thione

The title compound was obtained as a mixture with reagent residues (2.17 g) by performing the same reaction as in Example (1c) using 7-ethyl-7-methyl-1,4-thiazepan-5-one (1.31 g, 7.56 mmol) obtained in Example (51a) and Lawesson's reagent (1.68 g, 4.15 mmol). The resulting mixture was used as it was in the subsequent reaction.

### (51c) 7-Ethyl-7-methyl-5-methylthio-2,3,6,7-tetrahydro-1,4-thiazepine

The title compound was obtained as a mixture with reagent residues (353 mg) by performing the same reaction as in Example (1d) using the 7-ethyl-7-methyl-1,4-thiazepane-5-thione mixture (500 mg) obtained in Example (51b), potassium hydroxide (300 mg, 4.57 mmol), and methyl iodide (0.35 ml, 5.62 mmol). The resulting mixture was used as it was in the subsequent reaction.

### (51d) 3-(2-Chlorophenyl)-8-ethyl-8-methyl-5,6,8,9-tetrahydro[1,2,4]triazolo[4,3-d][1,4]thiazepine

The title compound was obtained as a white solid (310 mg, 58%) by performing the same reaction as in Example (1h) using the 7-ethyl-7-methyl-5-methylthio-2,3,6,7-tetrahydro-1,4-thiazepine mixture (353 mg) obtained in Example (51 c) and 2-chlorobenzoic acid hydrazide (280 mg, 1.64 mmol).
¹H NMR spectrum (400 MHz, CDCl₃) δppm: 1.05 (3H, t, J=7.4 Hz), 1.30 (3H, s), 1.55-1.70 (2H, m), 2.80-2.83 (2H, m), 3.51 (2H, s), 4.13-4.15 (2H, m), 7.39-7.43 (1H, m), 7.48-7.53 (3H, m).
Mass spectrum (FAB): m/z 307 (M+H)⁺
Melting point: 161-162°C.

### (Example 52) 3-(2-Chlorophenyl)-8-cyclopropyl-8-methyl-5,6,8,9-tetrahydro[1,2,4]triazolo[4,3-d][1,4]thiazepine (Example Compound No. 2-66)

### (52a) 7-Cyclopropyl-7-methyl-1,4-thiazepan-5-one

The title compound was obtained as a white solid (849 mg, 39%) by performing the same reaction as in Example (1b) using ethyl (2EZ)-3-cyclopropyl-2-butenoate (1.82 g, 11.8 mmol) and 2-aminoethanethiol (1.80 g, 23.3 mmol). ¹H NMR spectrum (400 MHz, CDCl₃) δppm: 0.46-0.56(1H, m), 0.50 (3H, t, J=7.02 Hz), 1.08-1.14 (1H, m), 1.17 (3H, s), 2.70-2.90 (3H, m), 3.10 (1H, d, J=13.3 Hz), 3.57-3.70 (2H, m), 6.22 (1H, br, NH)

### (52b) 7-Cyclopropyl-7-methyl-1,4-thiazepane-5-thione

The title compound was obtained as a mixture with reagent residues (1.14 g) by performing the same reaction as in Example (1c) using 7-cyclopropyl-7-methyl-1,4-thiazepan-5-one (849 mg, 4.58 mmol) obtained in Example (52a) and Lawesson's reagent (1.02 g, 2.52 mmol). The resulting mixture was used as it was in the subsequent reaction.

### (52c) 7-Cyclopropyl-7-methyl-5-methylthio-2,3,6,7-tetrahydro-1,4-thiazepine

The title compound was obtained as a mixture with reagent residues (367 mg) by performing the same reaction as in Example (1d) using the 7-cyclopropyl-7-methyl-1,4-thiazepane-5-thione mixture (380 mg) obtained in Example (52b), potassium hydroxide (380 mg, 5.79 mmol), and methyl iodide (0.47 ml, 7.55 mmol). The resulting mixture was used as it was in the subsequent reaction.

### (52d) 3-(2-Chlorophenyl)-8-cyclopropyl-8-methyl-5,6,8,9-tetrahydro[1,2,4]triazolo[4,3-d][1,4]thiazepine

The title compound was obtained as a white solid (279 mg, 43%) by performing the same reaction as in Example (1h) using the 7-cyclopropyl-7-methyl-5-methylthio-2,3,6,7-tetrahydro-1,4-thiazepine mixture (367 mg) obtained in Example (52c) and 2-chlorobenzoic acid hydrazide (320 mg, 1.65 mmol).
¹H NMR spectrum (400 MHz, CDCl₃) δppm: 0.52-0.60 (4H, m), 1.05 (3H, s), 1.07-1.13 (1H, m), 2.84-2.95 (2H, m), 3.49 (1H, d, J=15.1 Hz), 3.58 (1H, d, J=15.1 Hz), 4.14-4.19 (2H, m), 7.39-7.43 (1H, m), 7.47-7.53 (3H, m).
Mass spectrum (FAB): m/z 343 (M+H)⁺
Melting point: 218-220°C.

### (Example 53) 3-(2-Chlorophenyl)-8-(fluoromethyl)-8-methyl-5,6,8,9-tetrahydro[1,2,4]triazolo[4,3-d][1,4]thiazepine (Example Compound No. 2-79)

### (53a) Benzyl (2EZ)-4-fluoro-3-methyl-2-butenoate

Sodium hydride (930 mg, 21.3 mmol) was suspended in tetrahydrofuran (40 mL), a solution of benzyl(dimethoxyphosphoryl)acetate (5.00 g, 19.4 mmol) in tetrahydrofuran (20 mL) was added with ice cooling, and the mixture was stirred at room temperature for 15 min. 1-Fluoroacetone (1.00 g, 13.1 mmol) was added, and then the mixture was further stirred for 5 h. Saturated brine was added to the reaction mixture, and the mixture was extracted with ethyl acetate, washed with saturated brine, and then dried with anhydrous sodium sulfate. The filtrate was concentrated under reduced pressure, and then the residue was purified by silica gel column chromatography (elution solvent: hexane/ethyl acetate = 30/1) to obtain the title compound as a colorless oily substance which was an EZ mixture in an approx. 2:1 ratio (1.73 g, 63%)
¹H NMR spectrum (400 MHz, CDCl₃) δppm: 2.01 (3x1/3H, s), 2.11 (3x2/3H, s), 4.82 (2x2/3H, d, J=46 Hz), 5.12 (2x1/3H, s), 5.17 (2x2/3H, s), 5.53 (2x1/3H, d, J=49 Hz), 5.79 (1x1/3H, s), 6.01 (1x2/3H, s), 7.31-7.37 (5H, m).

### (53b) 7-(Fluoromethyl)-7-methyl-1,4-thiazepan-5-one

The title compound was obtained as a white solid (1.19 g, 81%) by performing the same reaction as in Example (1b) using benzyl (2EZ)-4-fluoro-3-methyl-2-butenoate (1.73 g, 8.30 mmol) obtained in Example (53a) and 2-aminoethanethiol (1.90 g, 24.6 mmol).
¹H NMR spectrum (400 MHz, CDCl₃) δppm: 1.40 (3H, s), 2.73(1H, ddd, J=15.2, 5.9, 3.5 Hz), 2.92-2.98 (1H, m), 2.99 (2H, s), 3.61-3.66 (2H, m), 4.35 (1H, dd, J=17.3, 9.4 Hz), 4.47 (1H, dd, J=17.3, 9.4 Hz), 6.01 (1H, br, NH)

### (53c) 7-(Fluoromethyl)-7-methyl-1,4-thiazepane-5-thione

The title compound was obtained as a mixture with reagent residues (1.27 g) by performing the same reaction as in Example (1c) using 7-(fluoromethyl)-7-methyl-1,4-thiazepan-5-one (1.18 g, 6.66 mmol) obtained in Example (53b) and Lawesson's reagent (1.50 g, 3.71 mmol). The resulting mixture was used as it was in the subsequent reaction.

### (53d) 7-(Fluoromethyl)-7-methyl-5-methylthio-2,3,6,7-tetrahydro-1,4-thiazepine

The title compound was obtained as a mixture with reagent residues (847 mg) by performing the same reaction as in Example (1d) using the 7-(fluoromethyl)-7-methyl-1,4-thiazepane-5-thione mixture (800 mg) obtained in Example (53c), potassium hydroxide (820 mg, 12.5 mmol), and methyl iodide (0.78 ml, 12.5 mmol). The resulting mixture was used as it was in the subsequent reaction.

### (53e) 3-(2-Chlorophenyl)-8-(fluoromethyl)-8-methyl-5,6,8,9-tetrahydro[1,2,4]triazolo[4,3-d][1,4]thiazepine

The title compound was obtained as a white solid (298 mg, 23%) by performing the same reaction as in Example (1h) using the 7-(fluoromethyl)-7-methyl-5-methylthio-2,3,6,7-tetrahydro-1,4-thiazepine mixture (357 mg) obtained in Example (53d) and 2-chlorobenzoic acid hydrazide (285 mg, 1.67 mmol).
¹H NMR spectrum (400 MHz, CDCl₃) δppm: 1.39 (3H, s), 2.80-3.04 (2H, m), 3.53 (1H, d, J=15.1 Hz), 3.68(1H, d, J=15.1 Hz), 4.16-4.19 (2H, m), 4.32 (1H, dd, J=11.3, 9.4 Hz), 4.44 (1H, dd, J=11.3, 9.4 Hz), 7.40-7.44 (1H, m), 7.48-7.54 (3H, m).
Mass spectrum (FAB): m/z 311 (M+H)⁺
Melting point: 167-168°C.

### (Example 54) {2-[3-(2-Chlorophenyl)-8-methyl-5,6,8,9-tetrahydro[1,2,4]triazolo[4,3-d][1,4]thiazepin-8-yl]}ethanol (Example Compound No. 2-92)

### (54a) Ethyl (2EZ)-5-{[t-butyl(dimethyl)silyl]oxy}-3-methyl-2-pentenoate

A methyllithium/diethyl ether solution (1.14 M, 50 mL, 56 mmol) was added dropwise to a suspension of copper(I) iodide (4.75 g, 25 mmol) in tetrahydrofuran (50 mL) at -20°C under a nitrogen atmosphere, and the mixture was stirred for 15 min. Then a solution of ethyl 5-{[t-butyl(dimethyl)silyl]oxy}-2-pentynoate (5.83 g, 20 mmol) in tetrahydrofuran (20 mL) was added at -70°C, the mixture was stirred for 2 h, methanol (5 mL) was further added, and the mixture was heated to room temperature. Dilute hydrochloric acid was added to the reaction mixture, and the mixture was extracted with ethyl acetate, then washed with water, saturated aqueous sodium hydrogencarbonate, and saturated brine, and dried with anhydrous sodium sulfate. The filtrate was concentrated under reduced pressure, and then the residue was purified by silica gel column chromatography (elution solvent: hexane/ethyl acetate = 5/1) to obtain the title compound as a colorless oily substance (5.70 g, 92%).
¹H NMR spectrum (400 MHz, CDCl₃) δppm: 0.04 (6H, s), 0.88 (9H, s), 1.24-1.29 (3H, m), 1.94 and 2.18 (3H in total, each s), 2.34 and 2.84 (2H in total, each t, J=6.7 Hz), 3.72-3.80 (2H, m), 4.10-4.17(2H, m), 5.67 and 5.70 (1H in total, each s).

### (54b) 7-(2-{[t-Butyl(dimethyl)silyl]oxy}ethyl)-7-methyl-1,4-thiazepan-5-one

The title compound was obtained as a white solid (820 mg, 13%) in the same manner as in Example (1b) using ethyl (2EZ)-5-{[t-butyl(dimethyl)silyl]oxy}-3-methyl-2-pentenoate (5.70 g, 21 mmol) obtained in Example (54a), 2-aminoethanethiol (1.93 g, 25 mmol), and piperidine (12 mL).
¹H NMR spectrum (400 MHz, CDCl₃) δppm: 0.06 (6H, s), 0.89 (9H, s), 1.41 (3H, s), 1.92 (2H, t, J=6.3 Hz), 2.75-2.77 (2H, m), 2.94 (1H, d, J=14.1 Hz), 3.01 (1H, d, J=14.1 Hz), 3.58-3.62 (2H, m), 3.83 (2H, t, J=6.3 Hz), 5.95 (1H, br, NH).

### (54c) 7-(2-{[t-Butyl(dimethyl)silyl]oxy}ethyl)-7-methyl-1,4-thiazepane-5-thione

The title compound was obtained as a white solid (730 mg, 85%) in the same manner as in Example (1c) using 7-(2-{[t-butyl(dimethyl)silyl]oxy}ethyl)-7-methyl-1,4-thiazepan-5-one (820 mg, 2.7 mmol) obtained in Example (54b) and Lawesson's reagent (570 mg, 1.4 mmol).
¹H NMR spectrum (400 MHz, CDCl₃) δppm: 0.07 (3H, s), 0.07 (3H, s), 0.89 (9H, s), 1.45 (3H, s), 1.93-1.99 (2H, m), 2.75-2.79 (2H, m), 3.50-3.52 (1H, m), 3.74-3.92 (5H, m).

### (54d) 7-(2-{[t-Butyl(dimethyl)silyl]oxy}ethyl)-7-methyl-5-methylthio-2,3,6,7-tetrahydro-1,4-thiazepine

The title compound was obtained as a colorless oily substance (111 mg, 68%) in the same manner as in Example (1d) using 7-(2-{[t-butyl(dimethyl)silyl]oxy}ethyl)-7-methyl-1,4-thiazepane-5-thione (156 mg, 0.49 mmol) obtained in Example (54c), potassium hydroxide (190 mg, 3.4 mmol), and methyl iodide (0.2 mL, 3.2 mmol).
¹H NMR spectrum (400 MHz, CDCl₃) δppm: 0.07 (6H, s), 0.90 (9H, s), 1.35 (3H, s), 1.83-1.88 (2H, m), 2.26 (3H, s), 2.62-2.68 (2H, m), 3.00 (1H, d, J=14.0 Hz), 3.04 (1H, d, J=14.0 Hz), 3.81 (2H, t, J=7.1 Hz), 3.99-4.04 (1H, m), 4.06-4.14 (1H, m).

### (54e) 8-(2-{[t-Butyl(dimethyl)silyl]oxy}ethyl)-3-(2-chlorophenyl)-8-methyl-5,6,8,9-tetrahydro[1,2,4]triazolo[4,3-d][1,4]thiazepine

The title compound was obtained as a colorless oily substance (106 mg, 88%) in the same manner as in Example (1h) using 7-(2-{[t-butyl(dimethyl)silyl]oxy}ethyl)-7-methyl-5-methylthio-2,3,6,7-tetrahydro-1,4-thiazepine (92.5 mg, 0.28 mmol) obtained in Example (54d) and 2-chlorobenzoic acid hydrazide (47.0 mg, 0.27 mmol).
¹H NMR spectrum (400 MHz, CDCl₃) δppm: 0.06 (3H, s), 0.07 (3H, s), 0.89 (9H, s), 1.37 (3H, s), 1.81-1.89 (2H, m), 2.83-2.87 (2H, m), 3.53-3.56 (2H, m), 3.86 (2H, t, J=6.8 Hz), 4.11-4.15 (2H, m), 7.38-7.44 (1H, m), 7.46-7.53(3H, m).

### (54f) {2-[3-(2-Chlorophenyl)-8-methyl-5,6,8,9-tetrahydro[1,2,4]triazolo[4,3-d][1,4]thiazepin-8-yl]}ethanol

The title compound was obtained as a white foamy substance (48.5 mg, 62%) in the same manner as in Example (1i) using 8-(2-{[t-butyl(dimethyl)silyl]oxy}ethyl)-3-(2-chlorophenyl)-8-methyl-5,6,8,9-tetrahydro[1,2,4]triazolo[4,3-d][1,4]thiazepine (105 mg, 0.24 mmol) obtained in Example (54e) and 4 N hydrogen chloride/dioxane (5 mL).
¹H NMR spectrum (400 MHz, CDCl₃) δppm: 1.43 (3H, s), 1.87 (2H, t, J=6.5 Hz), 2.27 (1H, br, OH), 2.73-2.83 (1H, m), 2.98-3.06 (1H, m), 3.50 (1H, d, J=15.1 Hz), 3.66 (1H, d, J=15.1 Hz), 3.84-3.95 (2H, m), 4.15-4.17 (2H, m), 7.40-7.48 (1H, m), 7.50-7.54 (3H, m).
Mass spectrum (EI): m/z 324 (M+H)⁺.

### (Example 55) N-{4'-[8-(Hydroxymethyl)-8-methyl-5,6,8,9-tetrahydro[1,2,4]triazolo[4,3-d][1,4]thiazepin-3-yl]-3'-methoxybiphenyl-3-yl}methanesulfonamide (Example Compound No. 1-104)

### (55a) Methyl 3'-amino-3-methoxybiphenyl-4-carboxylate

1.10 g of unpurified trifluoromethanesulfonic acid ester was obtained in the same manner as in Example (10a) using methyl 4-hydroxy-2-methoxybenzoate (646 mg, 3.5 mmol), triethylamine (1.2 mL, 8.7 mmol), and trifluoromethanesulfonic anhydride (1.13 g, 4.0 mmol). The title compound was obtained as a colorless oily substance (195 mg, 32%) in the same manner as in Example (10a) using a part of the above product (748 mg), (3-aminophenyl)boronic acid (204 mg, 2.8 mmol), sodium carbonate (424 mg, 4.0 mmol), and tetrakis(triphenylphosphine)palladium(0) (270 mg, 0.23 mmol).
¹H NMR spectrum (400 MHz, CDCl₃) δppm: 3.87 (2H, br, NH₂),3.91(3H, s), 3.97 (3H, s), 6.73 (1H, d, J=8.2 Hz), 6.91 (1H, s), 7.00 (1H, d, J=8.1 Hz), 7.14 (1H, s), 7.17 (1H, d, J=7.4 Hz), 7.23-7.27 (1H, m), 7.87 (1H, d, J=8.1 Hz).

### (55b) Methyl 3-methoxy-3'-[(methylsulfonyl)amino]biphenyl-4-carboxylate

To a solution of methyl 3'-amino-3-methoxybiphenyl-4-carboxylate (193 mg, 0.75 mmol) obtained in Example (55a) in methylene chloride (10 mL) and triethylamine (0.3 mL, 2.2 mmol) was added methanesulfonyl chloride (290 mg, 2.5 mmol) with ice cooling, and the mixture was stirred for 1 h. Water was added, and the mixture was stirred, followed by the addition of dilute hydrochloric acid, extracted with ethyl acetate, washed with water, saturated aqueous sodium hydrogencarbonate, and saturated brine, and dried with anhydrous sodium sulfate. The filtrate was concentrated under reduced pressure, and then the resulting solid was collected by filtration and washed with diisopropyl ether to obtain the title compound as a white solid (266 mg, 100%).
¹H NMR spectrum (400 MHz, CDCl₃) δppm: 3.45 (3H, s), 3.92(3H, s), 3.99 (3H, s), 7.12 (1H, d, J=1.5 Hz), 7.17 (1H, dd, J=1.5, 8.0 Hz), 7.38-7.44 (1H, m), 7.53-7.60 (2H, m), 7.65-7.75 (1H, m), 7.90 (1H, d, J=8.0 Hz).

### (55c) 3-Methoxy-3'-[(methylsulfonyl)amino]biphenyl-4-carboxylic acid

The title compound was obtained as a white solid (236 mg, 93%) in the same manner as in Example (10b) using methyl 3-methoxy-3'-[(methylsulfonyl)amino]biphenyl-4-carboxylate (266 mg, 0.79 mmol) obtained in Example (55b) and sodium hydroxide (200 mg, 5.0 mmol).
¹H NMR spectrum (400 MHz, CDCl₃) δppm: 3.07 (3H, s), 4.16 (3H, s), 7.21(1H, s), 7.32 (1H, d, J=7.8 Hz), 7.41-7.50 (4H, m), 8.25 (1H, d, J=7.8 Hz).

### (55d) t-Butyl 2-({3-methoxy-3'-[(methylsulfonyl)amino]biphenyl-4-yl} carbonyl)hydrazinecarboxylate

The title compound was obtained as a colorless oily substance (238 mg, 75%) in the same manner as in Example (1e) using 3'-cyano-3-methoxybiphenyl-4-carboxylic acid (236 mg, 0.73 mmol) obtained in Example (55c), oxalyl chloride (0.2 mL, 2.3 mmol), N,N-dimethylformamide (catalytic amount), t-butyl carbazate (150 mg, 1.1 mmol), and triethylamine (0.5 mL, 3.6 mmol).
¹H NMR spectrum (400 MHz, CDCl₃) δppm: 1.50 (9H, s), 3.04 (3H, s), 4.07 (3H, s), 7.15(1H, s), 7.21-7.30 (1H, m), 7.39-7.58 (4H, m), 7.60 (1H, br, NH), 8.25 (1H, d, J=8.2 Hz). 9.64 (1H, br, NH).

### (55e) 3-Methoxy-3'-[(methylsulfonyl)amino]biphenyl-4-carboxylic acid hydrazide

The title compound was obtained as a faint yellow solid (145 mg, 79%) in the same manner as in Example (1g) using t-butyl 2-({3-methoxy-3'-[(methylsulfonyl)amino]biphenyl-4-yl}carbonyl)hydrazinecarboxylate (238 mg, 0.54 mmol) obtained in Example (55d) and 4 N hydrogen chloride/ethyl acetate (10 mL). ¹H NMR spectrum (400 MHz, CDCl₃) δppm: 3.07 (3H, s), 4.05 (3H, s), 4.28 (2H, br, NH₂), 7.17(1H, s), 7.20-7.52 (5H, m), 8.28 (1H, d, J=8.2 Hz). 9.00 (1H, br, NH).

### (55f) N-{4'-[8-({[t-Butyl(dimethyl)silyl]oxy}methyl)-8-methyl-5,6,8,9-tetrahydro[1,2,4]triazolo[4,3-d][1,4]thiazepin-3-yl]-3'-methoxybiphenyl-3-yl}methanesulfonamide

The title compound was obtained as a white solid (142 mg, 56%) in the same manner as in Example (1h) using 7-({[t-butyl(dimethyl)silyl]oxy}methyl)-7-methyl-5-methylthio-2,3,6,7-tetrahydro-1,4-thiazepine (167 mg, 0.52 mmol) obtained in Example (1d) and 3-methoxy-3'-[(methylsulfonyl)amino]biphenyl-4-carboxylic acid hydrazide (145 mg, 0.43 mmol) obtained in Example (55e).
¹H NMR spectrum (400 MHz, CDCl₃) δppm: 0.07 (3H, s), 0.09 (3H, s), 0.92 (9H, s), 1.29 (3H, s), 2.89-3.04 (2H, m), 3.05 (3H, s), 3.63 (2H, s), 3.70 (2H, s), 3.88 (3H, s), 4.14-4.27 (2H, m), 7.22 (1H, s), 7.24-7.30 (1H, m), 7.39-7.47 (2H, m), 7.54-7.59 (3H, m), 8.17(1H, br, NH).

### (55g) N-{4'-[8-(Hydroxymethyl)-8-methyl-5,6,8,9-tetrahydro[1,2,4]triazolo[4,3-d] [1,4]thiazepin-3-yl]-3'-methoxybiphenyl-3-yl} methanesulfonamide

The title compound was obtained as a white solid (69.5 mg, 50%) in the same manner as in Example (1i) using N-{4'-[8-({[t-butyl(dimethyl)silyl]oxy}methyl)-8-methyl-5,6,8,9-tetrahydro[1,2,4]triazolo[4,3-d][1,4]thiazepin-3-yl]-3'-methoxybiphenyl-3-yl}methanesulfonamide (142 mg, 0.24 mmol) obtained in Example (55f) and 4 N hydrogen chloride/dioxane (5 mL).
¹H NMR spectrum (400 MHz, CDCl₃) δppm: 1.43 (3H, s), 2.32-2.36 (1H, m), 2.75-2.82 (1H, m), 3.06 (3H, s), 3.53 (1H, d, J=16.0 Hz),3.54-3.65 (2H, m), 3.87 (1H, d, J=16.0 Hz), 3.89 (3H, s), 4.20-4.38 (2H, m), 7.23 (1H, s), 7.24-7.30 (1H, m), 7.39-7.44 (2H, m), 7.48-7.59 (3H, m), 8.14 (1H, br, NH).
Mass spectrum (FAB): m/z 475 (M+H)⁺
Melting point: 250-252°C.

### (Example 56) (-)-[3-(3-chloro-4'-fluorobiphenyl-4-yl)-8-methyl-5,6,8,9-tetrahydro[1,2,4]triazolo[4,3-d][1,4]thiazepin-8-yl]methanol ((-) isomer of Example Compound No. 1-69)

### (56a) Optical resolution of compound obtained in Example (1h)

4 mL of a solution (4 to 6 mg/mL) of (+/-)-8-({[t-butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine obtained in Example (1h) in ethanol was fractionated 8 times under the following HPLC conditions to obtain 55.3 mg of a solid from a fraction containing isomer A (tR = 9.8 min) and 54.6 mg of a solid from a fraction containing isomer B (tR = 14.5 min).
Apparatus: SHIMADZU CLASS-VP System (LC-8/SCL-10AVP/SPD-10AVP)
Column: CHIRALPAK AD-H (2 cm × 25 cm) semi-preparative column, flow rate: 10.0 mL/min, elution solvent: ethanol (100%, isocratic), detection: UV (254 nm)

Analyses by HPLC using a chiral column were performed under the following conditions. Subsequent analyses were performed under similar conditions. The retention time was based on that by chiral HPLC.
Analyzing apparatus: SHIMADZU CLASS-VP System (LC-10ADVP/SCL-10AVP/SPD-M10AVP/CTO10ACVP/DGU12A)
Column: CHIRALPAK AD-H (0.46 cm × 25 cm), flow rate: 1.0 mL/min, elution solvent: ethanol (100%, isocratic), detection: UV (254 nm)
Optical isomer A: 8-({[t-Butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine ¹H NMR spectrum (400 MHz, CDCl₃) δppm: 0.08 (3H, s), 0.10 (3H, s), 0.93 (9H, s), 1.29 (3H, s), 2.92-2.98 (2H, m), 3.53-3.59 (2H, m), 3.61 (1H, d, J=17.3 Hz), 3.63 (1H, d, J=17.3 Hz), 4.16-4.20 (2H, m), 7.16-7.20 (2H, m), 7.56-7.59 (4H, m), 7.70 (1H, d, J=2.0 Hz).
Retention time: 4.5 min.
Optical isomer B: 8-({[t-Butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine ¹H NMR spectrum (400 MHz, CDCl₃) δppm: 0.08 (3H, s), 0.10 (3H, s), 0.93 (9H, s), 1.29 (3H, s), 2.92-2.98 (2H, m), 3.53-3.59 (2H, m), 3.61 (1H, d, J=17.3 Hz), 3.63 (1H, d, J=17.3 Hz), 4.16-4.20 (2H, m), 7.16-7.20 (2H, m), 7.56-7.59 (4H, m), 7.70 (1H, d, J=2.0 Hz).
Retention time: 10.0 min.

### (56b) (-)-[3-(3-Chloro-4'-fluorobiphenyl-4-yl)-8-methyl-5,6,8,9-tetrahydro[11,2,4]triazolo[4,3-d][1,4]thiazepin-8-yl]methanol

The title compound was obtained as a white solid (40.3 mg, 93%) in the same manner as in Example (1i) using the optical isomer A (tR = 4.5 min) obtained in Example (56a), specifically, an optical isomer (55.3 mg, 0.11 mmol) of 8-({[t-butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine, and 4 N hydrogen chloride/dioxane (5 mL).
[α]D²¹ -6.7° (c 0.52, MeOH).
¹H NMR spectrum (400 MHz, CDCl₃) δppm: 1.41 (3H, s), 2.20-2.30 (1H, m, OH), 2.65-2.75 (1H, m), 2.98-3.08 (1H, m), 3.43 (1H, d, J=16.4 Hz), 3.44-3.52 (2H, m), 3.74 (1H, d, J=16.4 Hz), 4.21-4.25 (2H, m), 7.18 (2H, t, J=8.8 Hz), 7.54-7.60 (4H, m), 7.69 (1H, s).
Mass spectrum (FAB): m/z 404 (M+H)⁺
Melting point: 212-215°C.

### (Example 57) (+)-[3-(3-Chloro-4'-fluorobiphenyl-4-yl)-8-methyl-5,6,8,9-tetrahydro[1,2,4]triazolo[4,3-d][1,4]thiazepin-8-yl]methanol ((+) isomer of Example Compound No. 1-69)

The title compound was obtained as a white solid (40.2 mg, 95%) in the same manner as in Example (1i) using the optical isomer B (tR = 10.0 min) obtained in Example (56a), specifically, an optical isomer (54.3 mg, 0.10 mmol) of 8-({[t-butyl(dimethyl)silyl]oxy}methyl)-3-(3-chloro-4'-fluorobiphenyl-4-yl)-8-methyl-5,6,8,9-tetrahydro[1,2,4]triazolo[4,3-d][1,4]thiazepine, and 4 N hydrogen chloride/dioxane (5 mL).
[α]D²¹ +9.8° (c 0.66, MeOH).
¹H NMR spectrum (400 MHz, CDCl₃) δppm: 1.41 (3H, s), 2.20-2.30 (1H, m, OH), 2.65-2.75 (1H, m), 2.98-3.08 (1H, m), 3.43 (1H, d, J=16.4 Hz), 3.44-3.52 (2H, m), 3.74 (1H, d, J=16.4 Hz), 4.21-4.25 (2H, m), 7.18 (2H, t, J=8.8 Hz), 7.54-7.60 (4H, m), 7.69 (1H, s).
Mass spectrum (FAB): m/z 404 (M+H)⁺
Melting point: 211-214°C.

### (Test Example 1) 11β-HSD1 enzyme inhibition experiment

### (1) Enzyme source

A plasmid obtained by incorporating cDNA coding for a full-length of human 11β-HSD1 in a mammal cell expression vector pCIneo (Promega Corporation) was introduced into HEK293 cells using Lipofectamine Plus Reagent (Invitrogen Corporation) according to the package insert. 48 h later, cells were collected and frozen at -80°C. Cells into which the pCIneo vector was introduced were prepared as a control. Cells were thawed and suspended in 20 mM HEPES, 1 mM ethylenediamine tetraacetic acid, 2 mM magnesium chloride (final concentrations), and a buffer containing a protease inhibitor cocktail (Roche), and 4.1 Mpa pressure was applied by N2 cavitation on the ice for 30 min to disrupt cells. The disrupted cell suspension was centrifuged at 1000 × g at 4°C for 10 min, and a supernatant thereof was recovered. The obtained supernatant was further ultracentrifuged at 105,000 × g at 4°C for 30 min, and the resulting precipitate was suspended in an assay buffer (50 mM tris buffer, 10% glycerol) as a microsome fraction and used as an enzyme source.

### (2) Enzyme inhibition experiment

A reaction was performed on a 384-well plate (Greiner Bio One) using a reaction volume of 25 µL, and all the samples were diluted with an assay buffer (50 mM tris buffer, pH 7.4, 10% glycerol). 0.8 mM NADPH, 6 mM glucose 6-phosphate, 0.35 units/mL glucose 6-phosphate dehydrogenase (Sigma Chemical), and 3 mM magnesium chloride and a microsome fraction having a protein amount of 0.7 µg per well (final concentrations) were added to the plate, and a solution containing a test compound dissolved in dimethyl sulfoxide was added at a final concentration of 0.1 % and preincubated at room temperature for 15 min. After completion of preincubation, cortisone at a final concentration of 160 nM was added to start a reaction, and the reaction was performed at room temperature for 3 h. 25 µL of 100 mM carbenoxolone (Sigma Chemical) was added to terminate the reaction, and the amount of produced cortisol was measured with Discovery (Hewlett-Packard Company) using a cortisol prototype kit (Cisbio International) according to the package insert. The compounds of Examples 1, 8, 32, and 52, respectively, at 39 nM exhibited 71.8%, 67.1%,35.8%, and 89.0% inhibiting effects on cortisol production dependent on the 11β-HSD1 expressing cells, which was not observed in microsome fractions derived from similarly treated control cells.

| (Formulation Example) Capsule | |
|---|---|
| Compound of Example 1 or 32 | 100 mg |
| Lactose | 100 mg |
| Maize starch | 148.8 mg |
| Magnesium stearate | 1.2 mg |
| Total amount | 350 mg |

Powders of the above-mentioned prescription were mixed, the mixture was passed through a 60-mesh sieve, and this powder was encapsulated in a gelatin capsule to produce a capsule.

### Industrial Applicability

The novel thiazepine derivative having general formula (I) or pharmacologically acceptable salt thereof of the present invention has an excellent 11β-HSD1 inhibitory effect and is useful as a therapeutic or prophylactic agent (in particular, therapeutic agent) for diabetes, adiposity, hyperlipemia, hypertension, or metabolic syndrome including these conditions in combination in a warm-blooded animal (in particular, a human).

## Claims

1. A thiazepine derivative having general formula (I): 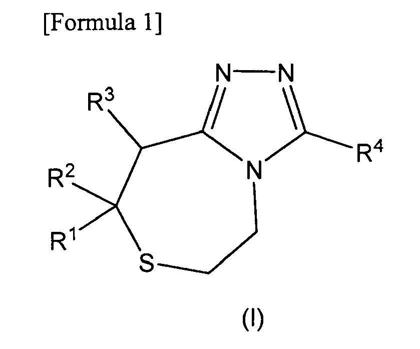 [wherein
R¹ represents a hydrogen atom, a C₁-C₆ alkyl group, a C₁-C₆ halogenated alkyl group, a C₁-C₆ hydroxyalkyl group, a (C₁-C₆ alkoxy)-(C₁-C₆ alkyl) group or a C₃-C₆ cycloalkyl group,
R² represents a C₁-C₆ alkyl group, a C₁-C₆ halogenated alkyl group, a C₁-C₆ hydroxyalkyl group, a (C₁-C₆ alkoxy)-(C₁-C₆ alkyl) group or a C₃-C₆ cycloalkyl group,
R³ represents a hydrogen atom or a C₁-C₆ alkyl group,
R⁴ represents a C₆-C₁₀ aryl group that may be substituted with 1 to 5 group(s) independently selected from Substituent Group a or a heterocyclic group that may be substituted with 1 to 3 group(s) independently selected from Substituent Group a,
Substituent Group a represents the group consisting of a halogen atom, a C₁-C₆ alkyl group, a C₁-C₆ halogenated alkyl group, a C₁-C₆ hydroxyalkyl group, a C₂-C₇ alkylcarbonyl group, a hydroxy group, a C₁-C₆ alkoxy group, a C₁-C₆ halogenated alkoxy group, a (C₁-C₆ alkoxy)-(C₁-C₆ alkyl) group, a C₃-C₆ cycloalkyl group, a C₁-C₆ alkyl group substituted with one C₃-C₆ cycloalkyl group, a carboxyl group, a C₂-C₇ alkoxycarbonyl group, a cyano group, a carbamoyl group, a C₂-C₇ alkylcarbonyloxy group, a C₁-C₆ alkylthio group, a C₁-C₆ alkylsulfonyl group, a nitro group, an amino group, a mono-C₁-C₆ alkylamino group, a di-(C₁-C₆ alkyl)amino group, a mono-(C₁-C₆ alkyl)aminocarbonyl group, a mono-C₂-C₇ alkylcarbonylamino group, a mono-C₁-C₆ alkylsulfonylamino group, a C₆-C₁₀ aryl group that may be substituted with 1 to 5 group(s) independently selected from Substituent Group b, a phenyloxy group that may be substituted with 1 to 5 group(s) independently selected from Substituent Group b, a phenylmethyloxy group that may be substituted with 1 to 5 group(s) independently selected from Substituent Group b, a phenylsulfonyl group that may be substituted with 1 to 5 group(s) independently selected from Substituent Group b and a heterocyclic group that may be substituted with 1 to 3 group(s) independently selected from Substituent Group b, and
Substituent Group b represents the group consisting of a halogen atom, a C₁-C₆ alkyl group, a C₁-C₆ halogenated alkyl group, a C₁-C₆ hydroxyalkyl group, a hydroxy group, a C₁-C₆ alkoxy group, a C₁-C₆ halogenated alkoxy group, a (C₁-C₆ alkoxy)-(C₁-C₆ alkyl) group, a carboxyl group, a C₂-C₇ alkoxycarbonyl group, a cyano group, a carbamoyl group, a C₂-C₇ alkylcarbonyloxy group, an oxo group, a C₁-C₆ alkylthio group, a C₁-C₆ alkylsulfonyl group, a nitro group, an amino group, a mono-C₁-C₆ alkylamino group, a di-(C₁-C₆ alkyl)amino group, a mono-(C₁-C₆ alkyl)aminocarbonyl group, a mono-C₂-C₇ alkylcarbonylamino group and a mono-C₁-C₆ alkylsulfonylamino group]
or a pharmacologically acceptable salt thereof.

2. The thiazepine derivative or pharmacologically acceptable salt thereof according to claim 1, wherein
R¹ represents a C₁-C₆ alkyl group, R² represents a C₁-C₆ hydroxyalkyl group, and R³ represents a hydrogen atom.

3. The thiazepine derivative or pharmacologically acceptable salt thereof according to claim 1, wherein
R¹ represents a methyl group, R² represents a hydroxymethyl group, and R³ represents a hydrogen atom.

4. The thiazepine derivative or pharmacologically acceptable salt thereof according to any one of claims 1 to 3, wherein
R⁴ represents a phenyl group that may be substituted with 1 to 5 group(s) independently selected from Substituent Group a, an indolyl group that may be substituted with 1 to 3 group(s) independently selected from Substituent Group a or a benzothiophenyl group that may be substituted with 1 to 3 group(s) independently selected from Substituent Group a.

5. The thiazepine derivative or pharmacologically acceptable salt thereof according to any one of claims 1 to 3, wherein
R⁴ represents a phenyl group that may be substituted with 1 to 5 group(s) independently selected from [a halogen atom, a C₁-C₆ halogenated alkyl group, a C₁-C₆ alkoxy group, a phenyl group that may be substituted with 1 to 5 group(s) independently selected from (a halogen atom, a C₂-C₇ alkoxycarbonyl group and a cyano group), a pyridin-4-yl group, a pyridin-3-yl group, a morpholin-4-yl group, a piperidin-1-yl group, a pyrrolidin-1-yl group and a 1H-pyrrol-1-yl group], an indolyl group that may be substituted with 1 to 3 group(s) independently selected from (a C₁-C₆ alkyl group and a C₁-C₆ alkyl group substituted with one C₃-C₆ cycloalkyl group) or a benzothiophenyl group that may be substituted with 1 to 3 group(s) independently selected from (a halogen atom and a C₁-C₆ alkyl group).

6. The thiazepine derivative or pharmacologically acceptable salt thereof according to any one of claims 1 to 3, wherein
R⁴ represents a phenyl group substituted with 2 groups independently selected from [a chlorine atom, a methoxy group and a phenyl group that may be substituted with 1 or 2 group(s) independently selected from (a fluorine atom, a chlorine atom and a cyano group)].

7. The thiazepine derivative or pharmacologically acceptable salt thereof according to any one of claims 1 to 3, wherein
R⁴ represents a 3-chlorobiphenyl-4-yl group, a 3-methoxybiphenyl-4-yl group, a 3-chloro-3'-fluorobiphenyl group, a 3,3'-dichlorobiphenyl-4-yl group, a 3-chloro-4'-fluorobiphenyl-4-yl group, a 3,4'-dichlorobiphenyl-4-yl group, a 3'-fluoro-3-methoxybiphenyl-4-yl group, a 3'-chloro-3-methoxybiphenyl-4-yl group, a 3'-cyano-3-methoxybiphenyl-4-yl group, a 4'-fluoro-3-methoxybiphenyl-4-yl group, a 3',4'-difluoro-3-methoxybiphenyl-4-yl group or a 3'-chloro-4'-fluoro-3-methoxybiphenyl-4-yl group.

8. The thiazepine derivative or pharmacologically acceptable salt thereof according to claim 1, wherein the compound having the general formula (I) is
[3-(3-chlorobiphenyl-4-yl)-8-methyl-5,6,8,9-tetrahydro[1,2,4]triazolo[4,3-d][1,4]thiazepin-8-yl]methanol,
[3-(3-methoxybiphenyl-4-yl)-8-methyl-5,6,8,9-tetrahydro[1,2,4]triazolo[4,3-d] [1,4]thiazepin-8-yl]methanol,
[3-(3-chloro-3'-fluorobiphenyl-4-yl)-8-methyl-5,6,8,9-tetrahydro[1,2,4]triazolo[4,3-d][1,4]thiazepin-8-yl]methanol,
[3-(3,3'-dichlorobiphenyl-4-yl)-8-methyl-5,6,8,9-tetrahydro[1,2,4]triazolo[4,3-d] [1,4]thiazepin-8-yl]methanol,
[3-(3-chloro-4'-fluorobiphenyl-4-yl)-8-methyl-5,6,8,9-tetrahydro[1,2,4]triazolo[4,3-d][1,4]thiazepin-8-yl]methanol,
[3-(3,4'-dichlorobiphenyl-4-yl)-8-methyl-5,6,8,9-tetrahydro[1,2,4]triazolo[4,3-d] [1,4]thiazepin-8-yl]methanol,
[3-(3'-fluoro-3-methoxybiphenyl-4-yl)-8-methyl-5,6,8,9-tetrahydro[1,2,4]triazolo[4,3-d][1,4]thiazepin-8-yl]methanol,
[3-(3'-chloro-3-methoxybiphenyl-4-yl)-8-methyl-5,6,8,9-tetrahydro[1,2,4]triazolo[4,3-d][1,4]thiazepin-8-yl]methanol,
4'-[8-(hydroxymethyl)-8-methyl-5,6,8,9-tetrahydro[1,2,4]triazolo[4,3-d][1,4]thiazepin-3-yl]-3'-methoxybiphenyl-3-carbonitrile,
[3-(4'-fluoro-3-methoxybiphenyl-4-yl)-8-methyl-5,6,8,9-tetrahydro[,2,4]triazolo[4,3-d][1,4]thiazepin-8-yl]methanol,
[3-(3',4'-difluoro-3-methoxybiphenyl-4-yl)-8-methyl-5,6,8,9-tetrahydro[1,2,4]triazolo[4,3-d][1,4]thiazepin-8-yl]methanol or
[3-(3'-chloro-4'-fluoro-3-methoxybiphenyl-4-yl)-8-methyl-5,6,8,9-tetrahydro[1,2,4]triazolo[4,3-d][1,4]thiazepin-8-yl]methanol.

9. The thiazepine derivative or pharmacologically acceptable salt thereof according to any one of claims 1 to 7, wherein the general formula (I) is general formula (Ia) or (Ib):

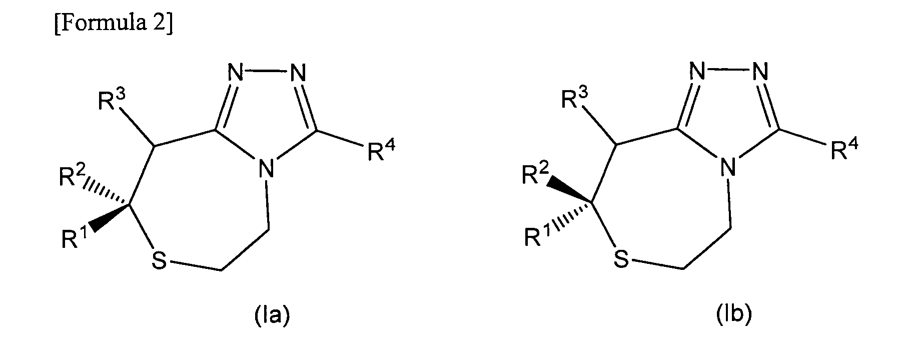

10. The thiazepine derivative or pharmacologically acceptable salt thereof according to claim 9, wherein the compound having the general formula (Ia) or (Ib) is
(-)-[3-(3-chloro-4'-fluorobiphenyl-4-yl)-8-methyl-5,6,8,9-tetrahydro[1,2,4]triazolo[4,3-d][1,4]thiazepin-8-yl]methanol or
(+)-[3-(3-chloro-4'-fluorobiphenyl-4-yl)-8-methyl-5,6,8,9-tetrahydro[1,2,4]triazolo[4,3-d][1,4]thiazepin-8-yl]methanol.

11. An 11β-hydroxysteroid dehydrogenase type 1 inhibitor containing the thiazepine derivative or pharmacologically acceptable salt thereof according to any one of claims 1 to 10 as an active ingredient.

12. A pharmaceutical composition containing the thiazepine derivative or pharmacologically acceptable salt thereof according to any one of claims 1 to 10 as an active ingredient.

13. The pharmaceutical composition according to claim 12, which has an effect of inhibiting 11β-hydroxysteroid dehydrogenase type 1.

14. The pharmaceutical composition according to claim 12, which is used for therapeutic and/or prophylactic treatment of diabetes, adiposity, hyperlipemia, hypertension, or metabolic syndrome.

15. The pharmaceutical composition according to claim 12, which is used for therapeutic and/or prophylactic treatment of diabetes.

16. Use of the thiazepine derivative or pharmacologically acceptable salt thereof according to any one of claims 1 to 10 for the manufacture of a pharmaceutical composition.

17. The use according to claim 16, wherein the pharmaceutical composition is a composition for inhibiting 11 β-hydroxysteroid dehydrogenase type 1.

18. The use according to claim 16, wherein the pharmaceutical composition is a composition for therapeutic and/or prophylactic treatment of diabetes, adiposity, hyperlipemia, hypertension, or metabolic syndrome.

19. The use according to claim 16, wherein the pharmaceutical composition is a composition for therapeutic and/or prophylactic treatment of diabetes.

20. A method for inhibiting 11β-hydroxysteroid dehydrogenase type 1, comprising administering a pharmacologically effective amount of the thiazepine derivative or pharmacologically acceptable salt thereof according to any one of claims 1 to 10 to a warm-blooded animal.

21. A method for therapeutic and/or prophylactic treatment of a disease, comprising administering a pharmacologically effective amount of the thiazepine derivative or pharmacologically acceptable salt thereof according to any one of claims 1 to 10 to a warm-blooded animal.

22. The method according to claim 21, wherein the disease is diabetes, adiposity, hyperlipemia, hypertension, or metabolic syndrome.

23. The method according to claim 21, wherein the disease is diabetes.

24. The method according to any one of claims 20 to 23, wherein the warm-blooded animal is a human.
